Europäisches Patentamt

European Patent Office

Office européen des brevets

(19)

(11) EP 0 198 474 B1

(12) EUROPEAN PATENT SPECIFICATION

(45) Date of publication and mention of the grant of the patent:
**26.06.1996 Bulletin 1996/26**

(51) Int Cl.[6]: **C12N 15/51**, C12N 5/10, C07K 14/02, C12P 21/02, A61K 39/29

(21) Application number: 86105183.7

(22) Date of filing: 15.04.1986

(54) **Hepatitis B surface antigen formed by recombinant DNA techniques, vaccines, diagnostics, cell lines and methods of forming same**

Mittels rekombinanter DNS-Techniken gebildetes Hepatitis-B-Oberflächenantigen, Impfstoffe, Diagnostika, Zellinien und Verfahren zu deren Herstellung

Antigène de surface de l'hépatite B produit par des techniques d'ADN recombinant, vaccins, diagnostics, lignées cellulaires et leur procédé de préparation

(84) Designated Contracting States:
AT BE CH DE FR GB IT LI LU NL SE

(30) Priority: 15.04.1985 EP 85104521

(43) Date of publication of application:
22.10.1986 Bulletin 1986/43

(60) Divisional application: 95119966.0

(73) Proprietor: **SmithKline Beecham Biologics SA**
B-1330 Rixensart (BE)

(72) Inventors:
• **Salstrom, John S.**
**Burnsville Minnesota 55337 (US)**
• **Rohrbaugh, Mark L.**
**St. Paul Minnesota 55117 (US)**
• **Thoma, Hans A.**
**D-8000 Munich 40 (DE)**

(74) Representative: **VOSSIUS & PARTNER**
**Postfach 86 07 67**
**81634 München (DE)**

(56) References cited:
EP-A- 0 013 828      EP-A- 0 020 251
EP-A- 0 155 198      WO-A-83/01783
WO-A-84/02534

• **JOURNAL OF MOLECULAR AND APPLIED GENETICS, vol. 2, no. 5, 1984, pages 497-506, Raven Press, New York, US; N. HSIUNG et al.: " Efficient production of hepatitis B surface antigen using a bovine papilloma virus-metallothionein vector"**
• **GENE, vol. 31, November 1984, pages 49-57, Elsevier Science Publishers, NL; G. CARLONI et al.: " A transformed Vero cell line stably producing hepatitis B surface antigen"**
• **PROCEEDINGS OF THE NATIONAL ACADEMY OF SCIENCES USA, vol. 81, no. 24, December 1984, pages 7708-7712, Washington, US; M. MICHEL et al.: "Synthesis in animal cells of hepatitis B surface antigen particles carrying a receptor for polymerized human serum albumin"**
• **IDEM**
• **PROCEEDINGS OF THE NATIONAL ACADEMY OF SCIENCES OF THE USA, vol. 80, no. 2, January 1983, pages 397-401, Washington, US; G N Parlakis et al.**
• **J Virol 52 (1984) 396-402**
• **Gastroenterology 86 (1984) 910-8**
• **Intern Symp Viral Hepatitis and Liver Disease, Houston, Texas, April 4-8, 1990, Abstracts 278-295**
• **JAMA 247 (1982) 2012-15**
• **J Medical Virology 11 (1983) 1-9**
• **Viral Hepatitis and Liver Disease (1988)) 1091-3**

- **J Imunology 137 (1986) 313-322**
- **Modern Approaches Vaccines: Mol Chem Basis Virus Virulence Immunogenecity; Conference 1983, CSH Lab, 251-256**
- **Virology 130 (1983) 76-90**
- **Am J Med Sci 270 (1975) 401**
- **Postgraduate Medical J 63 (1987) 41-50**
- **Cell 34 (1983) 215-223**

- **EMBO J 4 (1985) 249-255**
- **Biotechnology 3 (april 1985) 317-320**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**Description**

The present invention relates to bacterial plasmids carrying the PreS$_1$-PreS$_2$-S protein coding region, but lacking sequences encoding the hepatitis B core antigen, which are used for transfection of eukaryotic cell lines for production of particles containing polypeptides encoded by the PreS$_1$-PreS$_2$-S protein coding region.

Recent advances in genetic engineering have made it possible for a number of peptides and proteins to be expressed in bacteria, yeast as well as mammalian cells, in increased amounts. Though many proteins and peptides have been expressed in considerable amounts in bacteria, it is a special desire to express large amounts of those proteins and polypeptides which are to be used in human medicine in mammalian cells in order to avoid drawbacks associated with any contaminating products resulting from the bacterial cell, or from expression in bacterial cells.

Of special interest is the expression of hepatitis B surface antigen proteins in increased amounts in mammalian cells. The expression rate of these polypeptides in mammalian cells has so far not been high enough to allow an economic production thereof.

Hepatitis B virus is transmitted among humans and causes a chronic and debilitating infection that results in severe liver damage, primary carcinoma and death. In most cases, complete recovery from hepatitis B infection can be expected. However, in many African and Asian countries, hepatitis B virus infection is endemic. A large number of individuals in the populations of these countries are chronic carriers of hepatitis B virus and thereby have the dangerous potential of further transmitting the disease.

Vaccination is the only known protection against hepatitis B virus. Recently several companies (for example, Merck, Sharp and Dohme, U.S.A., and the Pasteur Institute, France) have introduced a plasma-derived vaccine against hepatitis B virus. The vaccine contains, as the antigen, hepatitis B viral proteins which are normally located on the envelope of the viral particle. Such antigen is commonly referred to as the hepatitis B virus surface antigen (HBsAg), and the viral gene encoding such antigen is called the HBsAg gene. The above-referenced vaccines contain surface antigen isolated from the plasma of hepatitis B virus infected humans. Although the surface antigen itself is not pathogenic, it stimulates the production of antibodies that are directed against hepatitis B virus particles in human beings.

The only commercially available source of hepatitis B surface antigen is the blood of hepatitis B virus infected humans. The isolation and purification of the surface antigen from human serum is a cumbersome and time-consuming process and is, therefore, relatively expensive. In addition, as long as the human serum is the only commercially available source for surface antigen, the amount of surface antigen available for use in vaccines, will be limited.

For large-scale vaccination, especially in areas where hepatitis B virus is endemic, it is of great importance to have a cheap and virtually unlimited source for surface antigen of hepatitis B virus. Because of the potential danger of contamination with pathogenic agents when surface antigen is taken from human serum, it is desirable to avoid the use of human serum as a source for surface antigen. There is also no biological system known besides human beings and chimpanzees in whch hepatitis B virus can grow and be propagated.

Recent advances in molecular biology have made it possible to clone the sequence of the complete genome of hepatitis B virus (Siddiqui, A. et al, Proc. Natl. Aca. Sci., U.S.A., Vol. 76, p. 4664, 1979; Sninsky, J.J. et al. Nature, Vol. 279, p. 346, 1979; and Charnay, P. et al, Nucl. Acids Res., Vol. 7, p. 335, 1979). In addition, the coding regions for the different viral proteins of hepatitis B virus have been identified (see, e.g., European Patent Application Publication Nos. 13828, 20251 and 38765; Galibert et al, Nature, Vol. 281, pp. 646-650, 1979; Pasek et al, Nature, Vol. 282, pp. 575-579, 1979; and Valenzuela et al, Nature, Vol. 280, pp. 815-819, 1979).

Following these advances, several host systems have been tested for the expression of that portion of the viral genome encoding the surface antigen proteins of hepatitis B virus.

It has been demonstrated in many cases that bacteria can provide a cheap production system for certain eukaryotic products. However, many difficulties are observed when eukaryotic products are synthesized in bacteria. For example:

1) the eukaryotic structural gene may not be efficiently transcribed in bacteria because codon usage in bacteria differs from codon usage in eukaryotes;
2) the eukaryotic gene product can be toxic to the host bacteria cell;
3) the structure and function of the eukaryotic gene product may be dependent on certain post-translational processes, such as glycosylation or special linkage of disulfide bonds, neither of which can be accomplished by the bacterial host;
4) only rarely will the gene product be secreted from the bacterial host cell;
5) eukaryotic promoters usually do not work in bacteria and must be substituted by a bacterial promoter, and such substitution can result in modification of the eukaryotic gene product, i.e., the N-terminal part of the product is of bacterial origin and includes the N-formyl methionine as the initial amino acid which does not occur in eukaryotes and which might present a new immunogenic determinant for the mammalian immune system; and
6) during the purification process, bacterial cell wall components may co-purify with the gene product and cause serious allergic reactions or lead to anaphylactic shock in mammalian recipients of the gene product.

There have been problems with hepatitis B surface antigen made in bacteria. First of all, the production rate in bacteria is very low and, in addition to this, the purification has to start from a bacterial lysate because the product is not secreted from the bacterial cell. Another problem arises from the fact that certain post-translational processes do not exist in bacteria, for example glycosylation of the surface antigen which has an influence on the level and specificity of the immune response. For any of these reasons it is desirable to avoid bacteria as production hosts.

Yeast cells transformed with an appropriate recombinant vector containing the hepatitis B surface antigen coding sequence synthesize and accumulate said surface antigen in considerable amounts in yeast culture. But there are some serious drawbacks in this host system, too, i.e., 1) the surface antigen is not secreted from the yeast cells and has to be isolated from a cell lysate of such cells, and 2) the surface antigen monomers made in yeast cells form no covalently-joined dimers, unlike the surface antigen secreted from mammalian cells.

There have been several attempts to establish mammalian cell lines that produce surface antigen. These cell lines have been derived from human hepatocellular carcinomas (Alexander, J.J. et al, Afr. Med. J., Vol. 50, p. 2124, 1976) as well as from cells transfected with cloned hepatitis B virus DNA. In addition, monkey kidney cells have been infected with simian virus 40 (SV40)-based recombinant DNA vectors carrying 40% of the hepatitis B virus genome (Laub, O. et al, J. of Virol., Vol. 48, p. 271, 1983). Also, co-transfection approaches have been used to select cell lines expressing the surface antigen (Standring, D.N. et al, J. of Virol., Vol. 50, p. 563, 1984). Amplification of the dihydrofolote reductase (DHFR) gene has been used to establish mammalian cell lines producing greater quantities of antigen (Michel et al, Proc. Natl. Aca. Sci., Vol. 81, p. 7708, 1984). Also, eukaryotic viral vectors that utilize the transformed phenotype as the selectable marker (Hsiung, N. et al, Molec. and Applied Genetics, Vol. 2, p. 497, 1984) have been chosen for transferring the surface antigen gene into mammalian cells. In these cases, DNA constructions containing oncogenic DNA sequences, or DNA sequences from oncogenic viruses, were used in the selection of cell lines producing surface antigen. The use of an oncogene sequence as a selection marker poses new safety problems when surface antigen made by these cells is used for vaccination.

A number of references discuss the expression of polypeptides synthesized by the hepatitis B virus coding sequence.

European Patent Application Publication No. 013,828-A1 (EPA 013,828), published August 8, 1980, states that nucleotide sequences encoding a 163 amino acid stretch (the PreS coding region) immediately preceding the S protein coding region in the HBV genome, may also be included in the fragments employed to produce useful recombinant DNA molecules for production of polypeptides displaying hepatitis B virus surface antigen antigenicity. In addition, it is described that gene fragments including both the precursor sequence and the structural gene for HBsAg may be excised by the use of one or more of a variety of restriction endonucleases prior to or during construction of a cloning vector. Furthermore, it is described that the 163 codon precursor preceding the structural gene coding for HBsAg should be excised from a cloning vehicle containing the S protein coding sequence prior to using the vector for transformation.

European Patent Application Publication No. 072,318-82 (EPA 072,318), published February 16, 1983, describes a method of making HBsAg by growing yeast cells transformed with a vector including a yeast replicon, a yeast promoter and a DNA segment coding for the S protein and specifically excluding the 163 codon precursor preceding the structural gene coding for HBsAg. A vaccine including an HBsAg antigen and a method of obtaining an HBsAg antibody by purifying the antibody from the serum of animals immunized against HBsAg is also described. A 14 to 18 nm antigen particle capable of forming an immune complex with HBsAg antibody is also described.

In Feitelson et al, Virology, Vol. 130, pp. 75-90, 1983, it was observed that a number of surface antigen-associated polypeptides may be partially encoded within the PreS coding sequence, including 24,000, 28,000, 32,000, 43,000, and 50,000 dalton species.

Laub et al, J. Virol., Vol. 48, No. 1, pp. 271-280, 1983, describe the construction of a simian virus 40 early replacement vector that has a structural gene coding for HBsAg including the 163 codon precursor sequence immediately preceding the structural gene, and expression of the coding sequence by SV40-transformed CV-1 cells (COS cells) transformed by such a vector. Laub et al also report that more HBsAg is expressed by COS cells transformed by a vector containing only the S protein coding sequence as compared to those transformed with a vector containing the 163 codon precursor immediately preceding the structural gene coding for HBsAg.

Takeda Chemical Ind., Japanese Patent Application No. J5-8194-897-A, published November 12, 1983, (Takeda I), describes an HBV DNA (adw subtype) coding for the entire PreS-S protein polypeptide and a vector containing such DNA and a host transformed with the DNA. Takeda Chemical Ind., Japanese Patent Application No. J5-9080-615-A, published May 10, 1984, (Takeda II), describes an HBV DNA (adw subtype) coding for the entire PreS-S protein polypeptide, as well as the polypeptide produced by such DNA and its use in a vaccine. Takeda Chemical Ind., Japanese Patent Application No. J5-9074-985-A, published April 27, 1984 (Takeda III), describes a DNA fragment containing one or more of the entire adw type PreS-S protein coding sequence, the S protein coding sequence or the Hepatitis B Virus Core Antigen (HBcAg) coding sequence, a vector containing such DNA, and hosts transformed with such DNA. Takeda III states that the 43,000 dalton polypeptide coded for by the PreS-S protein coding sequence "may be used as vaccines for prevention of HBV infection." Cloning of the PreS-S protein coding sequence in E. coli but no successful expression of any of the UBV genes and therefore of the polypeptide produced by such sequence is described in Takeda III.

Gerlich, in a presentation at the European Association of Clinical Microbiology in Bologna, October 18, 1983, reported that four potential genes of HBV have been deduced from the DNA sequence of cloned HBV-DNA; that the gene for the surface proteins (S-gene) of HBV consists of one uninterrupted coding sequence which is translated into at least three polypeptides; that the sequence of the major protein, P24, and its glycosylated form, GP27, begins with the third conserved translational start signal of the S-gene; that the minor surface proteins, GP33 or GP36, begin at the second start signal, that only the P41 polypeptide of HBV probably uses the full length coding sequence of the S-gene; that P41 carries a major antigenic determinant of HBV; and that it is present only in the complete viral particles but not in the 20 nm particles of surplus surface antigen from which the current hepatitis B vaccines derive. There is no report in Gerlich specifically regarding the $PreS_1$ region of P41 or that the $PreS_1$ region specifically contains an antigenic determinant which would be useful in an HBV vaccine.

Stibbe et al, <u>Develop. Biol. Standard</u>, Vol. 54, pp. 33-43, 1983, reported at the second WHO/IABS Symposium in Athens on Viral Hepatitis: Standardization in Immunoprophylaxis of Infections by Hepatitis Viruses, Athens, Greece, 1982, that the 20 nm particles of HBsAg isolated from serum of humans infected with HBV contain at least three minor proteins, GP33, GP36 and P41 as ascertained by SDS gel electrophoresis. Stibbe et al, conclude that GP33 and GP36 are probably not essential components of HBV vaccines. There is no report in Stibbe et al specifically regarding the $PreS_1$ region of P41, or that such region specifically contains an antigenic determinant that would be useful in an HBV vaccine.

Stibbe et al, <u>J. Virol.</u>, Vol. 46, No. 2, pp. 626-628, 1983, disclose that minor glycoproteins from the HBsAg coding region, named GP33 and GP36, are coded for by the $PreS_2$-S protein coding sequence. The $PreS_2$ region is a portion of the PreS coding region in the hepatitis B virus genome and codes for the first 55 amino acid immediately preceding the S protein.

Neurath et al, <u>Science</u>, 224, pp. 392-394, 1984, disclose that a polypeptide having a sequence identical to the amino-terminal 26 amino acids of the $PreS_2$ region acts as a highly efficient immunogen, and suggest that the antibodies elicited by the immunogen can be utilized for diagnostic tests.

Heerman et al, <u>J. Virol.</u>, Vol. 52, No. 2, pp. 396-402, 1984, describe the entire 389 amino acid coding sequence comprising <u>both</u> the S protein coding sequence and the PreS coding sequence, which codes for a polypeptide, P39, found in naturally-derived HBV particles and in viral surface antigen filaments, along with its glycosylated form, GP42, as well as the other HBV surface antigen-associated polypeptides P24, GP27, GP33 and GP36. Heerman et al also disclose that the unique portion of the P39/P42 protein, i.e., its $PreS_1$ region, bound monoclonal antibodies which had been induced by immunization with HBV particles. They suggest that such $PreS_1$ region is, probably, highly immunogenic. The $PreS_1$ region is that portion of the PreS coding region, immediately preceding the $PreS_2$ region and comprises the coding sequences for amino acids 1-108 (or 1-122 depending on virus subtype), which is the stretch of amino acids immediately preceding the $PreS_2$ coding sequence. Heerman et al also state that in the search for immunogenic and protective poly- or oligopeptides as an alternate vaccine against HBV, sequences of the PreS region may be of interest.

Michel et al, <u>Proc. Natl. Acad. Sci. U.S.A.</u>, Vol. 81, pp. 7708-7712, 1984, describe the synthesis of HBsAg carrying human serum polyalbumin receptors in Chinese hamster ovary (CHO) cells transfected with a plasmid carrying the $PreS_2$-S protein coding sequence.

Cattaneo et al, <u>Nature</u>, Vol. 305, pp. 335-338, 1985, disclose that the S gene of the HBV genome initiates translation at the beginning of the PreS region (i.e., at 489 nucleotides or 163 codons upstream of the S gene) and the mRNA is processed/polyadenylated at a site within the core gene.

Persing et al, <u>Proc. Natl. Acad. Sci. U.S.A.</u>, Vol. 82, pp. 3440-3444, 1985, disclose that mouse L cells transformed with a $PreS_2$-S protein coding sequence produce three HBsAg related polypeptides of molecular weight 24,000, 27,000 and 35,000 daltons, all of which are organized into complex immunoreactive HBsAg particles of 22 nm diameter which bind to polymerized human serum albumin (HSA); while mouse L cells transformed with the $PreS_2$-S protein coding sequence bearing a frame-shift mutation near the 3' end of the $PreS_2$ region produce only the 24,000 and 27,000 dalton polypeptides organized into 22 nm diameter immunoreactive HBsAg particles which are unable to bind HSA. Persing et al conclude that the $PreS_2$-S protein coding sequence encodes the 35,000 dalton species, that the $PreS_2$ coding portion accounts for the HSA-binding activity of HBsAg, but is not required for assembly and secretion of the HBsAg particles; and that the predominant polypeptide of HBsAg (i.e., the 24,000 dalton species) is not derived primarily by cleavage of larger presursors (i.e., the 27,000 and 35,000 dalton species) encoded by the $PreS_2$-S protein coding sequence.

Milich et al, <u>Science</u>, Vol. 228, pp. 1195-1199, 1985, disclose that vaccines that contain HBsAg particles with amino acids of both $PreS_2$ and HBsAg, wherein such particles were secreted by Chinese hamster ovary (CHO) cells transfected with a plasmid containing the $PreS_2$-S protein coding sequence, can circumvent nonresponsiveness to vaccines which just contain HBsAg since the immunogenic response to HbsAg is independent of the immunogenic response to

PreS$_2$; and that the 26 amino acid residues at the amino-terminus of the 33,000 dalton polypeptide coded for by the PreS$_2$-S protein coding sequence represent a dominant antibody binding site on the PreS$_2$ region.

Neurath et al, Nature, Vol. 315, pp. 154-156, 1985, disclose that the PreS$_2$ region codes for a protein on the HBV envelope with domains specifically recognized by liver cells; the PreS$_2$-S protein coding sequence codes for a protein present in HBV particles; synthethic peptides corresponding to the gene coding PreS$_2$ are highly immunogenic; and conclude that HBV vaccines should contain PreS$_2$.

Valenzuela disclosed, at the May 15, 1985 Bio-Expo-5 Meeting in Boston, expression of the entire PreS$_2$ protein coding sequence in yeast; that such yeast cells do synthesize a particle containing both HBsAg and PreS$_2$ peptide which is very similar in electron microscopy and sedimentation properties to particles containing only HBsAg, and that the PreS$_2$ region does not interfere with the ability to form the 22 nm HBsAg particles. Valenzuela also states that it has been hypothesized that HBV gets into the liver by binding polyalbumin to its polyalbumin receptor which in turn binds the polyalbumin receptor in the liver cell and thus, the virus gets internalized. The polyalbumin receptor of HBV is encoded by the PreS$_2$ region. Valenzuela concludes that a vaccine containing PreS$_2$ will elicit antibodies which, in addition to inactivating HBV through the normal mechanism, might interfere with the way the virus enters liver cells. See, also, Valenzuela et al, Biotechnology, Vol. 3, pp. 317-320, 1985.

Valenzuela et al, Biotechnology, Vol. 3, pp. 323-327, 1985, disclose the use of the HBsAg polyalbumin receptor coded for by the PreS$_2$ coding sequence as a means to prepare polyvalent vaccines. Valenzuela prepared a hybrid HBsAg-Herpes simplex 1 virus glycoprotein D (HSVlgD) particle by expressing the entire HSVlgD-PreS$_2$S protein coding sequence in yeast.

Neurath et al European Patent Application Publication No. 0,154,902-A2, published September 18, 1985, describes a hepatitis B vaccine containing a peptide with an amino acid chain of at least six consecutive amino acids within the PreS gene coding region of the envelope of hepatitis B virus. The vaccine is free of an amino acid sequence corresponding to the naturally occuring envelope proteins of hepatitis B virus and the physiologically acceptable diluant. The peptide is free or can be linked to a carrier. The Neurath European patent application claims priority from two earlier U.S. patent applications, U.S. Serial No. 587,090, filed March 7, 1984, and U.S. Serial No. 698,499, filed May 2, 1985.

Kent et al, Pept. Chem., Vol. 22, pp. 167-70, 1984, disclose that a chemically synthesized peptide comprising the N-terminal 26 amino acids of the PreS$_2$ region is a very good antigen and is a good candidate for a synthetic vaccine.

Lo et al, Biochem. Biophys. Res. Comm., Vol. 129, No. 3, pp. 797-803, 1985, disclose the characterization of the restriction endonuclease map of full length HBV DNA for different subtypes (adw, adr, ayw and adyw) of HBV, including characterization of the restriction endonuclease map of the entire PreS region. Lo et al also disclose cloning and expression of the full length HBV DNA in E. coli using a pUC8 expression vector, and state that they intend to use such HBV gene product, expressed in either prokaryotic or eukaryotic cells, as diagnostic agents and as vaccination sources.

Wong et al, J. Virology, Vol. 55, No. 1, pp. 223-231, 1985, disclose the identification of two HBV polypeptides encoded by the entire PreS open reading frame, i.e., 42 and 46 kd glycosylated polypeptides, containing determinants of both HBsAg and the PreS region. Wong et al also disclose expression of a tribrid fusion protein containing 108 amino acids corresponding to the N-terminal 27 through 133 amino acids of the 174 amino acids of the PreS region (adw$_2$ subtype), as well as β-galactosidase and chloramphenicol acetyltransferase sequences. This peptide contains 15 amino acids from the PreS$_2$ region and 93 from the PreS$_1$ region. Wang et al also disclose that (a) polyclonal antiserum generated to the tribrid fusion protein was capable of detecting 42 and 46 kd polypeptides in partially purified virus particle preparations, and (b) the glycosylated 42 and 46 kd polypeptides appear to correspond to the 39 and 42 kd non-glycosylated polypeptides described in Heerman et al, cited above.

Offensperger et al, Proc. Natl. Acad. Sci. USA, Vol. 82, pp. 7540-7544, 1985, disclose expression of a cloned DNA sequence encoding the PreS$_2$ peptide-β-galactosidase fusion protein by E-coli.

A number of references discuss plasmids containing the promoter of a mouse metallothionein gene.

Hamer et al, U.S. Patent Appln. Serial No. 452,783, filed December 23, 1982 and Pavlakis et al, Proc. Natl. Acad. Sci. USA, Vol. 80, p. 397, 1983, both disclose recombinant bovine papilloma virus plasmids containing the gene for human growth hormone (hGH) and a metallothionein gene (MMT) promoter.

Hofschneider et al, European Patent Appln. No. EP 0,105,141A2, published April 11, 1984, disclose recombinant plasmids containing the S protein coding sequence and either bovine papilloma virus type 1 DNA or Maloney mouse sarcoma virus DNA. The plasmids disclosed by Hofschneider et al preferably employ the natural promoter associated with the S protein coding sequence, but the Hofschneider reference does state (but does not exemplify) that "[a] further example for a preferred natural eukaryotic expression signal is the metallothionein signal from mouse cells."

Fogel et al, European Patent Appln. Publication No. EP 0,096,491A2, disclose the use of plasmids containing an entire metallothionein gene sequence, including the promoter region, as a means for obtaining strong expression of genes downstream of the MMT gene in mammalian hosts transformed with such plasmid.

University Patent, Inc., PCT Patent Appln. Publication No. WO 83/01783, published May 26, 1983, and Palmiter et al, Cell, Vol. 29, p. 701, 1982, disclose a plasmid comprising the mouse MT-1 gene promoter fused to a structural gene (preferably the gene for thymidine kinase from herpes simplex virus), and microinjection of mouse embryos with such plasmids, and state that gene expression of the resulting fused polypeptide product in differentiated cells of adult mice resulting from the embryos is subsequently regulable by administration of heavy metal ions. Palmiter et al also disclose plasmids containing the mouse MT-1 promoter fused to rat growth hormone, microinjection of such plasmids into mouse embryo, and regulation of gene expression of the resulting fused polypeptide product by heavy metal administration.

The present invention relates to a recombinant DNA vector being free of now-HBV viral or oreogenic nucleic and sequences comprising the hepatitis B $Pres_1$-$PresS_2$-S protein coding region and a mouse metallothionein (MNT) promoter. Such vector preferably additionnally comprises a transcription termination site and a selection marker preferably a drug resistance marker. Preferaby, the promoter is incorporated into said vector comprising the DNA sequence in addition to as in place of a hepatitis B natural promoter, preferably immediately upstream of the $PreS_1$-$PreS_2$-S protein coding sequence.

The present invention also relates to a transfected host eukaryotic cell transfected with a recombinant DNA vector comprising the $Pres_1$-$PreS_2$-S protein coding region and an MMT-promoter. Such vector preferably additionally comprises a a transcription termination site and a selection marker. Such host is preferably a mammalian cell.

The invention relates further to eukaryotic cell co-transfected with the recombinant DNA vector of the invention being defined as the first recombinant DNA vector and with a second recombinant DNA vector including a $PreS_2$-S protein coding region or an S protein coding region and the promoter of a metallothionein gene.

Further, the invention relates to eukaryotic cell co-transfected with the recombinant vector of the invention being defined as the first recombinant DNA vector and with a second recombinant DNA vector including a $PreS_2$-S protein coding region and with a third recombinant DNA vector including the promoter of a metallothionein gene and a selection marker, the metallothionein gene promoter in the third vector being located preferably immediately upstream of the selection marker.

In a preferred embodiment of said cell, the metallothionein gene promoter is incorporated into the second vector and is located preferably immediately upstream of the $PreS_2$-S protein coding region.

In a further preferred embodiment of said cell, the second and/or third vector include a transcription termination site, such as the DEF region of the mouse globin gene, and/or wherein the second vector includes a selection marker.

In a further preferred embodiment of said cell the second and/or third DNA vector contain no other viral DNA segments.

In a further preferred embodiment of said cell the DNA vectors include different selection markers.

In a further preferred embodiment of said cell, the first, the second and/or the third vector include a replicon.

In a further preferred embodiment of said cell the eukaryotic cell is a mammalian cell, such as a Chinese hamster ovary cell, a vero cell, an L-cell, a mouse fibroblastic cell or a rat fibroblastic cell.

Additionally, the invention relates to a method of preparing such transfected host cell which comprises transfecting a eukaryotic cell with the recombinant DNA vector of the present invention.

The present invention also relates to a method for preparing particles comprising at least one protein coded for by the entire $Pres_1$-$PreS_2$-S protein coding region which comprises: a) cultivating the transfected host eukaryotic cell of this invention under culture medium conditions which enable such host to express such protein; and b) isolating such particles. Preferably, such transfected host is able to secrete such proteins assembled into such particles into the medium. Preferably, such method additionally comprises the addition of heavy metal ions or steroid hormones to such culture medium to enhance the expression of the proteins. The present invention also relates to the particles prepared by such method.

The present invention also relates to a method of preparing a vaccine comprising an immunoprotective amount of the particles of this invention.

The proteins coded for by the $PreS_1$-$PreS_2$-S protein coding region comprised in the recombinant DNA vector of the present invention can be used in a method for detecting the presence of antibodies to in a sample of mammalian blood sera which comprises:

a) contacting the sample with a solid substrate coated with non-labeled particles of this invention;
b) incubating and washing said contacted sample;
c) contacting said contacted sample with labeled particles of this invention thereby producing a labeled contacted sample;
d) incubating and washing said labeled contacted sample; and
e) determining the extent of labeled particle in the labeled contacted sample.

They can further be used in a method for detecting the presence of proteins coded for by the $PreS_1$-$PreS_2$-S protein coding region in a sample of mammalian blood sera which comprises:

a) producing a composition containing an antibody produced by an immunogen comprised by the particles of this invention;

b) contacting the sample with a first portion of the composition and the immunogen which has been labeled, incubating and washing the first portion;

c) contacting an antigen-free control with a second portion of the composition and the immunogen which has been labeled, incubating and washing the second portion;

d) adding the same amount of staphylococci bearing protein A to the composition of steps b) and c) above, incubating both compositions and separating liquid from solids; and

e) determining the extent of labeled immunogen in each of the resultant compositions from step d) above.

They can further be used for preparing a diagnostic kit for detecting the presence of antibodies to proteins coded for by the $PreS_1$-$PreS_2$-S protein coding region in a sample of mammalian blood sera which comprises:

a) unlabeled proteins comprising the particle of this invention attached to a solid support; and

b) labeled antibodies to human IgG or IgM, for example.

Said proteins may be further used for preparing a diagnostic kit for detecting the presence of proteins coded for by the $PreS_1$-$PreS_2$-S protein coding region in a sample of mammalian blood sera which comprises:

a) antibodies, produced by the particles of this invention, attached to a solid support; and

b) labeled antibodies, produced by the particles of this invention.

The present invention also relates to a co-transfected eukaryotic host cell co-transfected with a recombinant DNA vector of this invention and a second recombinant DNA vector comprising the $PreS_2$-S protein coding region, or only the S protein coding region, and an MMT-promoter. Preferably, the second recombinant vector additionally comprises a transcription termination site and, optionally, a selection marker. Optionally, the co-transfected host is co-transfected with the recombinant DNA vector of this invention, the second recombinant DNA vector and a third recombinant DNA vector comprising an MMT-promoter and a selection marker. Preferably, the third recombinant vector additionally comprises a transcription termination site. Additionally, this invention relates to a method of preparing such co-transfected host cell which comprises co-transfecting a eukaryotic cell with the recombinant DNA vector of this invention, the second recombinant DNA vector described above, and, optionally, the third recombinant DNA vector described above. Preferably, said DNA vectors are co-transfected in pairwise combinations, or are co-transfected together or are transfected in a series of steps.

The present invention also relates to a method for preparing particles comprising at least one protein coded for by the entire $PreS_1$-$PreS_2$-S protein coding region which comprises: a) cultivating the co-transfected host eukaryotic cell of this invention under culture medium conditions which enable such host to express such protein; and b) isolating such particles. Preferably, such co-transfected host is able to secrete such proteins assembled into such particles in the medium. Preferably, such method additionally comprises the addition of heavy metal ions or steroid hormones to such culture medium to enhance the expression of such proteins. This invention also relates to the particles prepared by such method.

This invention relates to a recombinant DNA concatamer comprising: a) a vector comprising the $PreS_1$-$PreS_2$-S protein coding region and at least one vector comprising one of the following coding regions: $PreS_1$-$PreS_2$-S protein coding region or $PreS_2$-S protein coding region or S protein coding region: and b) the MMT promoter. Such concatamer preferably additionally comprises a selection marker and a transcription termination site. Such concatamers can be prepared by techniques known in the art.

This invention also relates to a host eukaryotic cell transfected with the concatamer of this invention. Such host is preferably a mammalian cell. Additionally, the invention relates to a method of preparing such transfected host cell with the concatamer of this invention which comprises transfecting a eukaryotic cell with the concatamer of this invention.

This invention also relates to a method for preparing a particle comprising at least one protein coded for by the entire $PreS_1$-$PreS_2$-S protein coding region which comprises: a) cultivating the transfected host of this invention, which comprises the concatamer of this invention, under culture medium conditions which enable such host to express such protein; and b) isolating such particle. Preferably, such transfected host is able to secrete such proteins assembled into such particles into the medium. Preferably, such method additionally comprises the addition of heavy metal ions or steroid hormones to such culture medium to enhance the expression of the proteins. This invention also relates to the particles prepared by such method.

BRIEF DESCRIPTION OF THE DRAWINGS

Figure 1 includes a partial restriction endonuclease map of plasmid pBPV342-12. Figure 1 also illustrates the location of plasmid pML2-derived DNA, bovine papilloma virus (BPV) DNA, the MMT-promoter and the sv-PAS-t transcription termination region on plasmid pBPV342-12. Figure 1 also illustrates the splitting of plasmid pBPV342-12 with the restriction endonuclease BamHI.

Figure 2 includes a partial restriction endonuclease map of plasmid pAOI which contains the complete hepatitis B virus genome in linear form. Figure 2 also illustrates the splitting of pAOI with the restriction endonuclease EcoRI, ligation of the EcoRI linear HBV product with T4 DNA-ligase to form concatamers and splitting of the concatamer product obtained with the restriction endonuclease BgIII to generate a specific DNA fragment containing the intact DNA coding sequence for the PreS$_1$-PreS$_2$-S protein coding region.

Figure 3 includes a partial restriction endonuclease map of plasmid pDMI and illustrates its construction by ligation of a BamHI DNA fragment comprising plasmid pMMT-neo with a BgIII genomic fragment of hepatitis B virus encoding the PreS$_1$-PreS$_2$-S protein coding sequence.

Figures 4A and 4B include a partial restriction endonuclease map of plasmid pDM2 and illustrates its construction from ligation of a BamHI restriction endonuclease fragment and a BamHI-BgIII restriction endonuclease fragment, both from plasmid pDM1.

Figure 5 includes a partial restriction endonuclease map of plasmid pDM3 and illustrates its construction from pDM1 and pMMT-neo.

Figure 6 is a graphical representation illustrating the elution pattern of the particles of this invention from a BioRad (A5m) column.

Figure 7 is a graphical representation of a typical isopycnic CsCl centrifugation of the particles of this invention from about 30 fractions from the first peak resulting from the BioRad (A5m) column.

Figures 8A, 8B and 8C are graphs that illustrate seropositive conversion induced in mice by different dilutions of purified particles of the present invention.

Figures 9A and 9B include a partial restriction endonuclease map of plasmid pENDO-1 and illustrates its multistep construction.

Figure 10 includes a partial restriction endonuclease map of plasmid pENDO-2 and illustrates its construction from ligation of restriction endonuclease DNA fragments of plasmid pENDO-1 and plasmid pDM2.

Figure 11 includes a partial restriction endonuclease map of plasmid pENDO-0 and illustrates its construction.

By the term "PreS$_1$-PreS$_2$-S protein coding region" is meant the region of the HBV DNA genome that encodes the entire polypeptide known as the PreS$_1$-PreS$_2$-S polypeptide, including the codon for the initial methionine through to the final termination codon (TAA) that specifies insertion of no amino acid and directs that translation terminate, or any functional derivative of such coding region. Proteins coded for by such region include the PreS$_1$-PreS$_2$-S polypeptide (389 amino acid residues in HBV subtype adw, for example). Preferred PreS$_1$-PreS$_2$-S protein coding regions are derived from the following subtypes: adr, ayw, adyw and adw. By the term "functional derivative" is meant any derivative of the PreS$_1$-PreS$_2$-S protein coding region which codes for polypeptides which, when assembled into a particle, function in substantially the same way as the particle resulting from the assembly of the polypeptides coded for the native PreS$_1$-PreS$_2$-S protein coding region in terms of immunogenic capability. Such derivatives include partial sequences of the PreS$_1$-PreS$_2$-S protein coding region, as well as derivatives produced by modification of such coding region. Techniques for modifying such coding region are known in the art and include, for example, treatment with chemical or biological mutagens, irradiation or direct genetic engineering, such as by inserting, deleting or substituting nucleic acids by the use of enzymes or other recombinant and molecular biological techniques.

By the term "PreS$_2$-S protein coding sequence" is meant the region of the HBV DNA genome that encodes the entire polypeptide known as the PreS$_2$-S polypeptide including the codon for the initial methionine through to the final termination codon (TAA) that specifies insertion of no amino acid, as specified above, or any functional derivative of such coding region. Proteins coded for by such region include the PreS$_2$-S polypeptide (281 amino acid residues in HBV subtype adw) and the S polypeptide (226 amino acid residues in HBV subtype adw, for example). Preferred PreS$_2$-S protein coding regions are derived from the following HBV subtypes: adr, ayw, adyw and adw. By the term "functional derivative" is meant any derivative of the PreS$_2$-S protein coding region which codes for polypeptides which, when assembled into a particle, function in substantially the same way as the particle resulting from the assembly of polypeptides coded for by the native PreS$_2$-S protein coding region in terms of immunogenic capability. Such derivatives include partial sequences of the PreS$_2$-S protein coding region, as well as derivatives produced by modification of such coding region. Techniques which may be employed for modifying such coding region are known in the art and some have been outlined above.

By the term "S protein coding sequence" is meant the region of the HBV DNA genome that encodes the entire polypeptide known as the S polypeptide including the codon for the initial methionine through to the final termination codon (TAA) that specifies insertion of no amino acid, as specified above, or any functional derivative of such coding region. Proteins coded for by such region include the S peptide (226 amino acid residues in HBV subtype adw, for example). Preferred S protein coding regions are derived from the following HBV subtypes: adr, ayw, adyw and adw. By the term "functional derivative" is meant any derivative of the S protein coding region which codes for polypeptides which, when assembled into a particle, function in substantially the same way as the particle resulting from the assembly of polypeptides coded for by the native S protein coding region in terms of immunogenic capability. Such derivatives include partial sequences of the S protein coding region, as well as derivatives produced by modification of such coding region. Techniques which may be employed for modifying such coding region are known in the art and some have been outlined above.

By the term "promoter" is meant that DNA sequence within a DNA molecule upstream of a gene that directs the appropriate host cell RNA polymerase complex to attach to the DNA molecule at a specific site on said DNA molecule and to become properly positioned to begin transcription of the gene at a specific point on the DNA to result in the synthesis of an RNA copy of one of the DNA strands.

By the term "transcription" is meant that process carried out by the host cell biosynthetic machinery, including RNA polymerase complexes, that, using one of the two complementary DNA strands as a template, polymerizes ribonucleotides in a sequential fashion, 5' to 3', (3' to 5' with respect to the DNA template strand) to yield an exact copy of one of the DNA strands, but containing ribonucleotides instead of deoxyribonucleotides.

By the term "transcription termination sequence" is meant that region within the DNA molecule, designated $t$, that signals to the RNA polymerase complex engaged in the process of transcription to terminate progress of such transcription to yield an RNA molecule that can be processed properly, including polyadenylation and transport for such RNA molecules that are destined to be used as messenger RNA molecules in the host cell cytoplasm.

By the term "polyadenylation" is meant the process by which the host cell biosynthetic machinery recognizes a specific sequence, usually, 5'-AATAAA-3', designated PAS, for polyadenylation signal, within the RNA molecule and that directs the addition to the 3' end of said molecule some 15-20 nucleotides downstream (in the direction of the 3' end) of a non-template determined polyriboadenylate moiety, specifically added by the enzyme poly-A polymerase.

By the term "selection marker" is meant a gene determinant that, when expressed in the cell, confers a specific set of characteristics upon the cell that allows such a cell to be distinguished, or selected out, from other cells not carrying or expressing said gene determinant.

Preferred selection markers include drug resistance markers. By the term "drug-resistance marker" is meant a special class of selection markers that confer upon a cell expressing such a marker, resistance to the lethal effects of a drug or an antibiotic that ordinarily blocks growth, or kills, cells not carrying or expressing said drug-resistance marker.

Preferred drug resistance markers include the coding sequences for the neomycine-resistance gene, neo; the Eco-gpt gene (Mulligan, R.C. and Berg, P., Science, Vol. 209, p. 1422, 1980), the dihydrofolate reduction (DHFR) gene (Ringold, G. et al, J. of Molec. and Appld. Genet., Vol. 1, p. 165, 1981).

By the term "particles comprising at least one protein coded for by the entire $PreS_1$-$PreS_2$-S protein coding region" is meant a hepatitis B particle, devoid of any nucleic acid, formed by the assembly within or from a culture lysate of a euraryotic cell transfected with the entire $Pres_1$, $PreS_2$ and S region wherein such particle contains subunits composed predominantly of the S polypeptide (dimers), but also composed of smaller amounts of the entire $PreS_1$-$PreS_2$-S, optionally, the $PreS_2$-S polypeptides.

This invention relates to a recombinant DNA vector comprising the $PreS_1$-$PreS_2$-S protein coding region and a MMT-promoter. The MMT-promoter may be incorporated into the DNA vector either in addition to the natural promoter for the DNA sequence (hepatitis B promoter) or in place of the hepatitis B promoter. Preferably, the MMT-promoter is located in the DNA vector immediately upstream of the $PreS_1$-$PreS_2$-S protein coding region. The MMT promotor is operatively linked to the DNA sequence which means that the coding region is under the control of the promotor. Preferably, such vector also comprises a transcription termination sequence and a selection marker which preferably is effective in a eucaryotic cell. Preferably, such selection marker is a drug-resistance marker, such as the neomycine gene. Preferably, such transcription termination sequence is the DEF region (mg-PAS-t) of the mouse globin gene.

The DEF region of the mouse globin gene is described by Falck-Pederson et al, Cell, Vol. 40, p. 897, 1985. Such vector may be prepared by conventional recombinant DNA and other molecular biological techniques. In the most preferable case, using the mg-PAS-t region, the vector does not contain any viral DNA segments and is not oncogenic, i.e., it will not transform (make cancerous) any host cell into which it is introduced. Such vector, upon transfection into a host, if it does not contain an autonomous replication sequence (replicon) capable of functioning in a host transfected therewith, integrates into the host chromosome and replicates passively with the host genome.

The recombinant DNA vector of this invention should comprises the following characteristics:

1) The $PreS_1$-$PreS_2$-S protein coding region.
2) A MMT-promoter located immediately upstream of the $PreS_1$-$PreS_2$-S protein coding region.
3) The vector should be able to replicate in bacteria, or other procaryotic host into which it is transformed, for growth, amplification and preparation of large quantities of the recombinant vector. Thus, such vector should include a bacterial or other procaryotic replicon, i.e., a DNA segment bearing all the functions required for autonomous replication and maintenance of the vector extrachromosomally in a procaryotic host cell, such as a bacterial host cell, transformed therewith. Such replicons are well known in the art.

4) The vector replicon should be small (i.e., smaller than 6-8 kilobase pairs) to enable easy genetic and molecular biological manipulation thereof.

5) The vector should carry a selection marker, preferably a drug-resistance marker such as ampicillin, for use in bacterial host cells transformed therewith.

6) The vector should carry a second selection marker, preferably a drug-resistance marker such as neomycine, for use as such in eukaryotic host cells transfected therewith.

7) The vector should contain convenient endonuclease restriction sites for cloning.

8) The vector should contain a transcription termination and polyadenylation sequence.

Most preferably, the vector of this invention does not comprise an autonomous replicating sequence (replicon) capable of functioning in a eukaryotic host cell transfected therewith. A primary reason for using a non-replicating vector system in eukaryotic host cells is that all vector systems capable of autonomous and extrachromosomal replication in a mammalian host eukaryotic cell transfected therewith comprise replicons which are derived from oncogenic viruses. It is desirable to employ vectors comprising DNA not derived from oncogenic viruses for expressing DNA sequences encoding polypeptides of pharmaceutical imortance, such as the proteins coded for by the $PreS_1$-$PreS_2$-S protein coding region.

The vector of this invention comprises the MMT-promoter. The MMT-promoter is a non-viral, strong transcriptional promoter. The coding sequence of the MMT-promoter is described by Pavlakis and Hamer, Proc. Natl. Acad. Sci., Vol. 11, p. 397, 1983. The MMT-promoter is regulable by heavy metal ions, such as zinc and cadmium, and by steroid hormones, such as dexamethasone. Such regulation is well known (see , e.g., Yagle and Palmiter, Molecular and Cellular Biol, Vol. 5, p. 291, 1985).

This invention also relates to a transfected host eukaryotic cell transformed with a recombinant DNA vector of this invention. Preferably, such host is a mammalian cell, most preferably a Chinese hamster ovary (CHO) cell line, a vero cell line, an L-cell line, or a mouse or rat fibroblastic cell line. Such host may be prepared by transfecting a eukaryotic cell with a recombinant DNA vector of this invention by conventional techniques, such as by the method of Graham and van der Eb, Virology, Vol. 52, p. 456, 1973, or Wigler, M., Cell, Vol. 14, p. 725, 1978.

This invention also relates to a method for preparing a particle comprising proteins coded for by the $PreS_1$-$PreS_2$-S protein coding region wherein at least one of such proteins corresponds to a polypeptide coded for by the entire $PreS_1$-$PreS_2$-S protein coding region which comprises: a) cultivating the transfected host of this invention under culture medium conditions which enable such host to express such proteins, and b) isolating such particle. Preferably, such co-transfected host is able to secrete such proteins assembled into such particles into the medium. Preferably, heavy metal ions or steroid hormones, such as dexamethasone, are added to such culture medium to induce the MMT-promoter and thereby enhance expression of such coding region. Heavy metal ions such as cadmium or zinc are most preferred. The optimal concentration of heavy metal ions or steroid hormone contained in the medium can be determined by conventional techniques.

This invention also relates to the particles prepared by such method. If the transfected host cell of this invention secretes such particles directly into the culture medium, such particles can then be isolated from the culture medium of the transfected host of this invention by conventional protein isolation techniques. If the transfected host cell of this invention does not secrete such particles, they are obtained from a culture lysate of such host by conventional culture lysate techniques. Such particles are obtained in glycosylated form and are composed of proteins coded for by the $PreS_1$-$PreS_2$-S protein coding region, including at least one protein coded for by the entire $PreS_1$-$PreS_2$-S protein coding region.

This invention also relates to a vaccine comprising an immunoprotective amount of the particle of this invention. By "immunoprotective amount" is meant that quantity (preferably 1-20 ug) of the particles of this invention required to induce and maintain a level of antibody in the host sufficient to neutralize the infectious agent and prevent proliferation and subsequent disease by said infectious agent.

The particle of the subject invention contains no viral DNA components and therefore is free from undesirable side effects commonly found in naturally-derived vaccines, such as unintentional infection with the virus, allergic reactions, fevers, and the like. The vaccine of the present invention comprising an immunoprotective amount of the particles of this invention can be used to improve the HBV immune response and to overcome non-responsiveness to hepatitis B virus vaccines which do not include proteins coded for by the entire $PreS_1$-$PreS_2$-S protein coding sequence.

A vaccine of this invention can be prepared by combining an immunoprotective amount of the particles of this invention in an appropriate buffer, such as phosphate buffered saline. The vaccine may additionally comprise an adjuvant, such as aluminum hydroxide and the like.

Alternatively, the polypeptides comprised by the particles of the present invention can be disassociated from said particles and reassociated in a lipid vesicle. A vaccine against hepatitis B virus can be prepared using said lipid vesicles comprising the polypeptides of the present invention. The lipid vesicles in the appropriate concentration can be used as a vaccine with or without adjuvant, such as aluminum hydroxide.

The particles of the vaccine of this invention can be employed with a physiologically acceptable diluant (medium), such as phosphate buffered saline.

The vaccine of this invention, comprising an immunoprotective amount of the particles of this invention, can be prepared and used in the same general manner as disclosed in U.S. Patent 4,118,479, the entire contents of which are hereby incorporated by reference.

The vaccine can be administered by subcutaneous, intradermal or intramuscular injection. While the preferred route would depend on the particular vaccine, it is believed that intramuscular injection is generally more suitable. Frequency of administration will vary depending upon the vaccine.

The proteins coded for by the $PreS_1$-$PreS_2$-S protein coding region comprised in the recombinant DNA vector of the invention can be used in a method for detecting the presence of antibodies to in a sample of mammalian blood sera which comprises:

a) contacting the sample with a solid substrate coated with non-labeled particles of this invention;
b) incubating and washing said contacted sample;
c) contacting said contacted sample with labeled particles of this invention thereby producing a labeled contacted sample;
d) incubating and washing said labeled contacted sample; and
e) determining the extent of labeled particle in the labeled contacted sample.

They can further be used in a method for detecting the presence of proteins coded for by the $PreS_1$-$PreS_2$-S protein coding region in a sample of mammalian blood sera which comprises:

a) producing a composition containing an antibody produced by an immunogen comprised by the particles of this invention;
b) contacting the sample with a first portion of the composition and the immunogen which has been labeled, incubating and washing the first portion;
c) contacting an antigen-free control with a second portion of the composition and the immunogen which has been labeled, incubating and washing the second portion;
d) adding the same amount of staphylococci bearing protein A to the composition of steps b) and c) above, incubating both compositions and separating liquid from solids; and
e) determining the extent of labeled immunogen in each of the resultant compositions from step d) above.

Said proteins can be further used for the preparation of a diagnostic kit for detecting the presence of antibodies to proteins coded for by the $PreS_1$-$PreS_2$-S protein coding region in a sample of mammalian blood sera which comprises:

a) unlabeled proteins comprising the particle of this invention attached to a solid support; and
b) labeled antibodies to human IgG or IgM, for example.

They can further be used for preparing a diagnostic kit for detecting the presence of proteins coded for by the $PreS_1$-$PreS_2$-S protein coding region in a sample of mammalian blood sera which comprises:

a) antibodies, produced by the particles of this invention, attached to a solid support; and
b) labeled antibodies, produced by the particles of this invention.

They can also be used for developing diagnostic tests for direct detection of hepatitis B surface antigens and antibodies, raised to such antigens. Radioimmunoassay (RIA) or enzyme-linked immunosorbant assay (ELISA) are employed to detect HBV antigens containing proteins coded for the $Pres_1$-$PreS_2$-S coding regions in sera of HBV infected animals, such as humans. One diagnostic test includes the following:

1. A solid substrate containing binding sites thereon, such as polystyrene beads, is coated with antibodies to particles containing amino acid sequences corresponding to those of the $PreS_1$-$PreS_2$-S containing polypeptides;
2. The coated beads are then washed with, for example, Tris buffered saline to remove excess antibodies;
3. The beads are then contacted with a protein-containing solution, such as bovine serum albumine (BSA) or gelatin, to saturate protein binding sites on the beads (to reduce non-specific binding). A convenient concentration of such protein-containing solution can be employed, such as 1 mg/ml to 50 mg/ml;

4. The beads are then washed to remove excess BSA or gelatin;

5. The beads are then incubated with serum samples suspected to contain HBV or HBV surface antigens (normal serum is utilized as a control);

6. The beads are then washed with a solution, such as Tris buffered saline solution, and mixed with a radio-labeled antibody, such as $^{125}$I-labeled antibody to HBV surface antigens;

7. The beads are then incubated; and

8. The beads are then washed and counted in a gamma counter.

Particles of the present invention coded for by the $PreS_1$-$PreS_2$-S protein coding region can be employed as a diagnostic tool to detect antibodies to the $PreS_1$-$PreS_2$-S regions of HBV surface antigen proteins in a given sample. The $PreS_1$-$PreS_2$-S containing particles are adsorbed on a solid substrate containing binding sites thereon, for example, polystyrene beads. The substrate is thereafter contacted with a substance, for example, gelatin, BSA or powdered milk, to saturate the non-specific binding sites thereon. Thereafter, the substrate is washed with a buffered solution and thereafter the buffer is removed. A specimen, such as human serum, diluted with animal serum, is added to the substrate. The resulting mass is then incubated and washed. Thereafter, radio-labeled, such as iodinated ($^{125}$I), antibodies to human IgG or IgM is added to the mass. The resulting mass is incubated then washed and counted, such as in the gamma counter. If the count is higher than the count obtained for the normal serum control, the specimen contains antibodies to the $PreS_1$-$PreS_2$-S coding regions of HBV.

The above procedure for detection of antibodies to the proteins coded for by the $PreS_1$-$PreS_2$-S protein coding region of HBV is believed to be applicable as a diagnostic tool in detecting hepatitis B virus infection.

A diagnostic test kit for detecting antigens coded for the $PreS_1$-$PreS_2$-S protein coding regions of the HBV genome in a test sample would include the following:

1. A solid substrate coated with antibodies to a particle having amino acid sequences corresponding to the $PreS_1$-$PreS_2$-S regions of HBV surface antigen particles of the present invention;

2. A protein-containing solution to saturate protein binding sites on the solid substrate; and

3. A given amount of radio-labeled antibody, such as antibody to the HBV surface antigen polypeptides.

Another diagnostic test kit for detecting antibodies to the proteins coded for by the $PreS_1$-$PreS_2$-S protein coding region of the hepatitis B virus genome in the test sample includes the following:

1. A solid substrate havaing adsorbed thereon particles containing amino acid sequences corresponding to the $PreS_1$-$PreS_2$-S coding regions of the HBV surface antigen proteins of the present invention, the substrate being exposed to a protein-containing solution to saturate non-specific protein binding sites on a solid substrate; and

2. A given amount of radio-labeled antibodies to human IgG or IgM.

Radio-labeled antibodies used in the above-described test kits can be packaged in solution form or in lyophilized form suitable for reconstitution. In addition, enzyme-linked or fluorescent-labeled antibodies can be substituted for the described radio-labeled antibodies.

The above-described test kit and process for detecting antibodies to the $PreS_1$-$PreS_2$-S coding region polypeptides of hepatitis B virus can be utilized in applications such as detecting HBV infection in a patient by taking serum from the patient and applying the above-described test or using the above-described test; and predicting recovery from HBV infection by taking serum from an infected patient and applying the described antibody detection procedures.

The test procedures and test kits for antibody detection can be used for making qualitative comparisons between different HBV vaccines by taking serum from vaccinated patients and then utilizing the above-described test procedures or kits for antibody detection. In general, all known immunoassays using this antigen as reagent can be performed using the $PreS_1$, $PreS_2$, or S-containing particles of this invention. Generally, all known immunoassays using antibody-containing serum or reagents can be performed using antibody serum produced through the use of a peptide produced by recombinant DNA techniques of the present invention. These immunoassays include all those disclosed by Langone and Van Vunakis in Methods of Enzymology, Academic Press, Vols. 70, 73 and 74. Those assays disclosed in disclosures of the following U.S. Patents: 4,459,359; 4,343,896; 4,331,761; 4,292,403; 4,228,240; 4,157,280; 4,152,411; 4,169,012; 4,016,043; 3,839,153; 3,654,090; and Re. 31,006 and Vols. 70, 73 and 74 of Methods of Enzymology are incorporated herein by reference.

This invention also relates to a co-transfected host eukaryotic cell co-transfected with the recombinant vector of this invention and a second recombinant DNA vector comprising the $PreS_2$-S protein coding region, or only the S protein coding region, and an MMT-promoter. Preferably, the second recombinant vector additionally comprises a transcription termination site and, optionally, a selection marker. Optionally, the co-transfected host is co-transfected with the recombinant DNA vector of this invention, the second recombinant DNA vector and a third recombinant DNA vector comprising an MMT-promoter and a selection marker. Preferably, the third recombinant vector additionally comprises a transcription termination site. Preferably, the second and optional third recombinant DNA vector also contain a replicon for growth and amplification in a procaryotic host cell, such as a bacterial host cell, when such host is transformed with said second or optional third recombinant DNA vector.

Additionally, this invention relates to a method of preparing such co-transfected host cell which comprises co-transfecting a eukaryotic cell with the recombinant DNA vector of this invention, the second recombinant DNA vector described above, and, optionally, the third recombinant DNA vector described above. The second and optional third recombinant DNA vectors described above can be constructed by conventional techniques. If the optional third vector is not employed, the second recombinant DNA vector preferably additionally comprises a selection marker, such as a drug resistance marker. Preferably, if the second and/or optional third recombinant DNA vector comprise a transcription termination site, such site is the mg-PAS-t termination site. Preferably, in the second recombinant DNA vector described above, the MMT-promoter is located in such vector immediately upstream of the $PreS_2$-S protein coding region. Preferably, in the optional third recombinant DNA vector described above, the MMT-promoter is located in such vector immediately upstream of the selection marker. Preferably, neither the second recombinant vector nor the optional third recombinant vector described above contain any non-HBV viral DNA segments and are non-oncogenic. Thus, upon transfection into a host, such preferred vectors integrate into the host chromosome and are replicated passively with the host genome.

The vector of this invention, the second recombinant DNA vector described above, and, optionally, the third recombinant DNA vector described above are co-transfected into a eukaryotic host in pairwise combinations, or all three vectors together, using conventional methods. Alternatively, the vector of this invention, the second recombinant DNA vector and, optionally, the third recombinant DNA vector are transfected into a eukaryotic host in a series of steps. Initially, the vector of this invention is transfected into a eukaryotic host according to the method of this invention to produce the transfected host of this invention. Subsequently, such transfected host is then transfected with the second recombinant DNA vector described above, and, optionally, the third recombinant DNA vector described above according to conventional methods to produce the co-transfected host of this invention.

Preferably, the vector of this invention, the second recombinant DNA vector and the optional third recombinant DNA vector comprise selection markers different from each other to enable identification of the newly-transfected host at each subsequent transfection step leading to the final transfected host of the invention. Such secondary transfection steps are carried out in order to increase the gene copy number of the $PreS_1$-$PreS_2$-S protein coding region comprised by the multiply transfected host cell. Preferably, neither the second recombinant vector nor the optional third recombinant vector comprise a replicon capable of functioning in a eukaryotic cell.

This invention also relates to a method for preparing a particle, produced by the co-transfected host of this invention, comprising at least one protein coded for by the entire $PreS_1$-$PreS_2$-S protein coding region which comprises: a) cultivating the co-transfected host eukaryotic cell of this invention under culture medium conditions which enable such host to express such proteins; and b) isolating such particle. Preferably, such co-transfected host is able to secrete such proteins assembled into such particles into the medium. Preferably, such method additionally comprises the addition of heavy metal ions or steroid hormones to such culture medium to enhance the expression of the $PreS_1$-$PreS_2$-S protein coding region contained by the co-transformed vectors. Heavy metal ions, particularly zinc or cadmium, are most preferred. The optimal concentration of heavy metal ions or steroid hormones contained in the medium can be determined by conventional techniques. This invention also relates to the particles prepared by such method. If the co-transfected host of this invention secretes such particles directly into the culture medium, such particles can then be isolated from the culture medium of the co-transformed host of this invention by conventional protein isolation techniques. If the co-transfected host does not secrete such particles into the culture medium, then they are obtained from a culture lysate of such co-transfected host by conventional culture lysate techniques. Such particles are isolated in glycosylated form and are composed of proteins coded for by the $PreS_1$-$PreS_2$-S protein coding region, the $PreS_2$-S protein coding region, and the S protein coding region.

This invention also relates to a method for preparing a vaccine comprising an immunoprotective amount (Preferably 1-20 ug) of the particles produced by the co-transfected host of this invention.

The particle produced by the co-transfected host of the subject invention contains no viral DNA components and therefore is free from undesirable side effects commonly found in naturally-derived vaccines, such as unintentional infection with the virus, allergic reactions, fevers, and the like. The vaccine produced by the method of the present invention comprising an immunoprotective amount of the particles produced by the co-transfected host of this invention can be used to improve the HBV immune response and to overcome non-responsiveness to hepatitis B virus vaccines which do not include at least one protein coded for by the entire $pres_1$-$Pres_2$-s protein coding sequence.

Said vaccine can be prepared by combining an immunoprotective amount of the particles produced by the co-transfected host of this invention in an appropriate buffer, such as phosphate buffered saline. The vaccine may additionally comprise an adjuvant, such as aluminum hydroxide and the like.

Alternatively, the polypeptides comprised by the particles produced by the co-transfected host of the present invention can be disassociated from said particles and reassociated in a lipid vesicle. A hepatitis B vaccine can be prepared using said lipid vesicles comprising the polypeptides of said particles of the present invention. The vaccine comprising the lipid vesicles may additionally comprise an adjuvant, such as aluminum hydroxide.

The particles produced by the co-transfected host of ths invention comprising the vaccine of this invention can be employed with a physiologically acceptable diluant (medium), such as phosphate buffered saline.

The vaccine of this invention comprising an immunoprotective amount of the particles of this invention can be prepared and used in the same general manner as disclosed in U.S. Patent 4,118,479.

The vaccine can be administered by subcutaneous, intradermal or intramuscular injection. While the preferred route would depend on the particular vaccine, it is believed that intramuscular injection is generally more suitable. Frequency of administration will vary depending upon the vaccine.

The proteins coded for by the $PreS_1$-$PreS_2$-S protein coding region comprised in the vector of the invention can be used in a method for detecting the presence of antibodies to in a sample of mammalian blood sera which comprises:

a) contacting the sample with a solid substrate coated with non-labeled particles produced by the co-transfected host of this invention;
b) incubating and washing said contacted sample;
c) contacting said contacted sample with labeled particles of this invention thereby producing a labeled contacted sample;
d) incubating and washing said labeled contacted sample; and
e) determining the extent of labeled particle in the labeled contacted sample.

Said proteins can further be used in a method for detecting the presence of proteins coded for by the $PreS_1$-$PreS_2$-S protein coding region in a sample of mammalian blood sera which comprises:

a) producing a composition containing an antibody produced by an immunogen comprised by the particles of this invention;
b) contacting the sample with a first portion of the composition and the immunogen which has been labeled, incubating and washing the first portion;
c) contacting an antigen-free control with a second portion of the composition and the immunogen which has been labeled, incubating and washing the second portion;
d) adding the same amount of staphylococci bearing protein A to the composition of steps b) and c) above, incubating both compositions and separating liquid from solids; and
e) determining the extent of labeled immunogen in each of the resultant compositions from step d) above.

Said proteins can further be used for preparing a diagnostic kit for detecting the presence of antibodies to proteins coded for by the $PreS_1$-$PreS_2$-S protein coding region in a sample of mammalian blood sera which comprises:

a) unlabeled proteins comprising the particle of this invention attached to a solid support; and
b) labeled antibodies to human IgG or IgM, for example.

Furthermore, they can be employed for preparing a diagnostic kit for detecting the presence of proteins coded for by the $PreS_1$-$PreS_2$-S protein coding region in a sample of mammalian blood sera which comprises:

a) antibodies, produced by the particles of this invention, attached to a solid support; and
b) labeled antibodies, produced by the particles of this invention.

This invention relates to a recombinant DNA concatamer comprising: a) a plasmid vector carrying the $PreS_1$-$PreS_2$-S protein coding region and at least one additional plasmid vector comprising one of the following coding regions: $PreS_1$-$PreS_2$-S protein coding region of $PreS_2$-S protein coding region or S protein coding region; and b) the MMT promoter. Such concatamer preferably additionally comprises a selection marker and a transcription termination site.

This invention also relates to a eukaryotic host cell transfected with the concatamer of this invention. Such host is preferably a mammalian cell. Additionally, the invention relates to a method of preparing such transfected host cell with the concatamer of this invention which comprises transfecting a eukaryotic cell with the concatamer of this invention.

This invention also relates to a method for preparing a particle, produced by the transfected host of this invention, comprising at least one protein coded for by the entire $PreS_1$-$PreS_2$-S protein coding region, which comprises: a) cultivating the transfected eukaryotic host cell of this invention, which comprises the concatamer of this invention, under culture medium conditions which enable such host to express such proteins; and b) isolating such particle.

Preferably, such co-transfected host is able to secrete such proteins assembled into such particles into the medium. Preferably, such method additionally comprises the addition of heavy metal ions or steroid hormones to such culture medium to enhance the expression of the $Pres_1$-$Pres_2$-S protein coding region contained by the transfected vectors. Heavy metal ions, particularly zinc or cadmium, are most preferred. The optimal concentration of heavy metal ions or steroid hormones contained in the medium can be determined by conventional techniques. This invention also relates to the particles prepared by such method. If the host transfected with the concatamer of this invention secretes such particles directly into the culture medium, such particles can then be isolated from the culture medium of the transfected host of this invention by conventional protein isolation techniques. If the transfected host does not secrete such particles into the culture medium, then they are obtained from a culture lysate of such transfected host by conventional culture lysate techniques. Such particles are isolated in glycosylated form and are composed of proteins coded for by the $PreS_1$-$PreS_2$-S protein coding region, the $PreS_2$-S protein coding region, and the S protein coding region.

This invention also relates to a method for preparing a vaccine comprising an immunoprotective amount (preferably 1-20 ug) of the particles produced by the host transfected with the concatamer of this invention.

The particle produced by the host transfected with the concatamer host of the subject invention contains no viral DNA components and therefore is free from undesirable side effects commonly found in naturally-derived vaccines, such as unintentional infection with the virus, allergic reactions, fevers, and the like. The vaccine of the present invention comprising an immunoprotective amount of the particles produced by the host transfected with the concatamer of this invention can be used to improve the HBV immune response and to overcome non-responsiveness to hepatitis B virus vaccines which do not include at least one protein coded for by the entire $PreS_1$-$PreS_2$-S protein coding sequence.

Said vaccine can be prepared by combining an immunoprotective amount of the particles produced by the host transfected with the concatamer of this invention in an appropriate buffer, such as phosphate buffered saline. The vaccine may additionally comprise an adjuvant, such as aluminum hydroxide and the like.

Alternatively, the polypeptides comprised by the particles produced by the host transfected with the concatamer of the present invention can be disassociated from said particles and reassociated in a lipid vesicle. A hepatitis B vaccine can be prepared using said lipid vesicles comprising the polypeptides of said particles of the present invention. The vaccine comprising the lipid vesicles may additionally comprise an adjuvant, such as aluminum hydroxide.

The particles produced by the host transfected with the concatamer of this invention comprising the vaccine of this invention can be employed with a physiologically acceptable diluant (medium), such as phosphate buffered saline.

The vaccine of this invention comprising an immunoprotective amount of the particles of this invention can be prepared and used in the same general manner as disclosed in U.S. Patent 4,118,479.

The vaccine can be administered by subcutaneous, intradermal or intramuscular injection. While the preferred route would depend on the particular vaccine, it is believed that intramuscular injection is generally more suitable. Frequency of administration will vary depending upon the vaccine.

The proteins coded for by the $PreS_1$-$PreS_2$-S protein coding region comprised in the vector of the invention can be used in a method for detecting the presence of antibodies to in a sample of mammalian blood sera which comprises:

a) contacting the sample with a solid substrate coated with non-labeled particles produced by the host transfected with the concatamer of this invention;
b) incubating and washing said contacted sample;
c) contacting said contacted sample with labeled particles of this invention thereby producing a labeled contacted sample;
d) incubating and washing said labeled contacted sample; and
e) determining the extent of labeled particle in the labeled contacted sample.

They can further be used in a method for detecting the presence of proteins coded for by the $PreS_1$-$PreS_2$-S protein coding region in a sample of mammalian blood sera which comprises:

a) producing a composition containing an antibody produced by an immunogen comprised by the particles of this invention;
b) contacting the sample with a first portion of the composition and the immunogen which has been labeled, incubating and washing the first portion;
c) contacting an antigen-free control with a second portion of the composition and the immunogen which has been labeled, incubating and washing the second portion;
d) adding the same amount of staphylococci bearing protein A to the composition of steps b) and c) above, incubating both compositions and separating liquid from solids; and
e) determining the extent of labeled immunogen in each of the resultant compositions from step d) above.

Furthermore, they can be employed for preparing a diagnostic kit for detecting the presence of antibodies to proteins coded for by the PreS$_1$-PreS$_2$-S protein coding region in a sample of mammalian blood sera which comprises:

a) unlabeled proteins comprising the particle of this invention attached to a solid support; and
b) labeled antibodies to human IgG or IgM, for example.

They can further be used for preparing a diagnostic kit for detecting the presence of proteins coded for by the PreS$_1$-PreS$_2$-S protein coding region in a sample of mammalian blood sera which comprises:

a) antibodies, induced by the particles of this invention, attached to a solid support; and
b) labeled antibodies, produced by the particles of this invention.

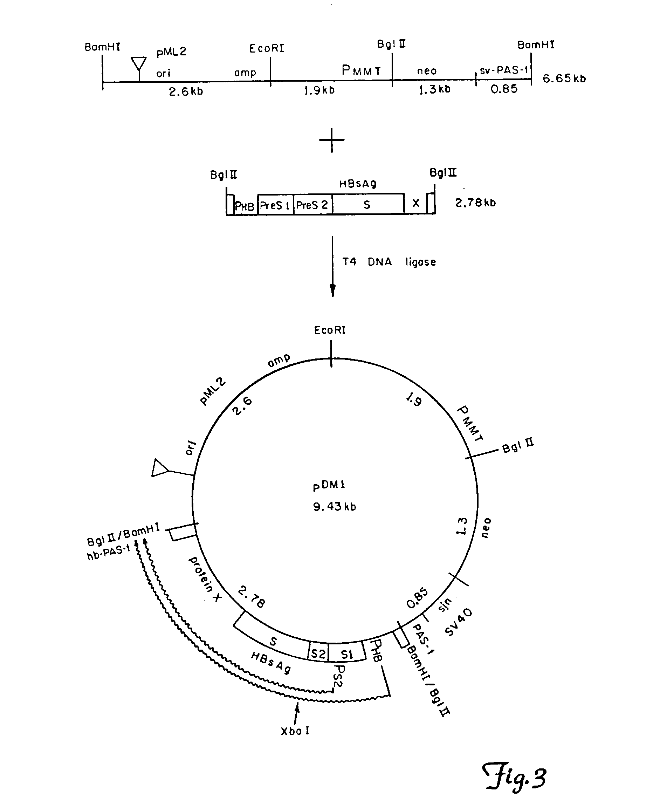

As a starting material for the first preferred embodiment, one vector incorporating gene sequences from recombinant plasmids pBPV342-12 (Law et al, Molecular and Cellular Diol, Vol, 3, p. 2110, 1983) and pAOI (Cummings, I. W. et al, Proc. Natl. Aca. Sci., U.S.A., Vol. 77, p. 1842, 1980) is constructed. Some of the genetic element from these plasmids are combined to create a vector, designated pDM1 (see Figure 3). Plasmid pDM1, which contains the gene segment carrying the PreS$_1$-PreS$_2$-S protein coding region under the control of the natural promoter system for said protein coding region, the neomycine-resistance gene, the MMT promoter and an SV40 PAS-t function, is then introduced by transfection into any one of a number of eukaryotic cells, such as mammalian cells, to produce high-level expression and, preferably, secretion of the particles of this invention.

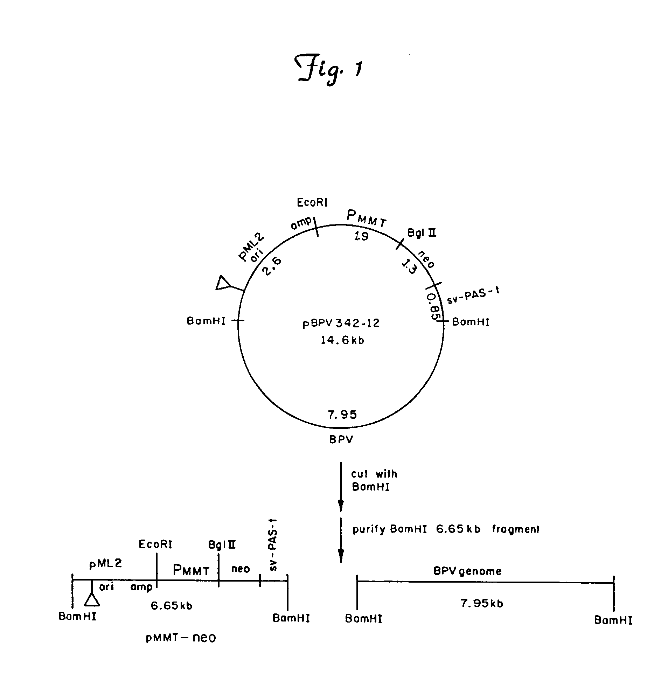

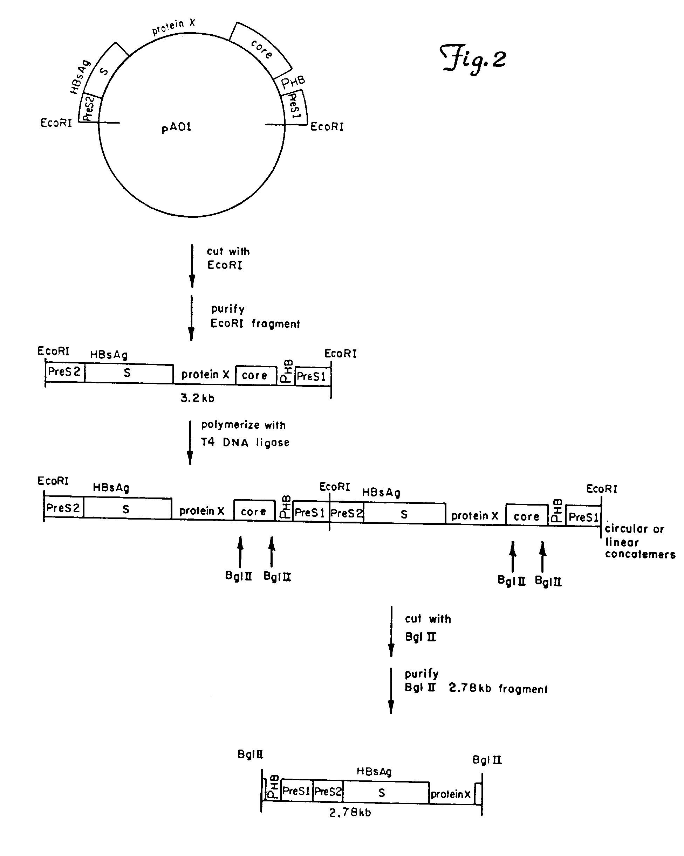

Plasmid pBPV342-12 (see Figure 1) contains gene sequences including the MMT-promoter, the neomycine-resistance gene, an SV40 PAS-t function and the bovine papilloma virus (BPV) genome. During the construction of plasmid pDM1, plasmid pBPV342-12 is subjected to BamHI digestion, with the result that the plasmid is split into two DNA fragments. Following separation of the fragments, the 7.95 kilobase (kb) fragment, which comprises the entire BPV genome, is discarded. The elimination of BPV DNA is highly advantageous since it precludes inclusion of any BPV DNA or proteins in the particle of this invention which is to be used for vaccine formulation. The remaining BamHI fragment (6.65 kb) from pBPV342-12, which contains the gene sequences for the MMT-promoter, the neomycine resistance gene and the SV40 PAS-t region, is then ligated, by conventional techniques, to a DNA sequence derived from plasmid pAOI, but which contains an intact PreS$_1$-PreS$_2$-S protein coding region (discussed below; see also Figure 2).

Plasmid pAOI (see Figure 2), which contains the entire hepatitis B viral genome, is subjected to EcoRI digestion to produce a 3.2 kb fragment. the 3.2 kb fragment is ligated to itself to produce tandem repeats comprising a long concatemeric DNA structure containing gene sequences encoding both the core and surface antigens of hepatitis B virus. The method for this manipulation of the hepatitis B viral gene sequences, which is necessary to overcome the permutation caused by molecular cloning in plasmid pAOI and to restore a functional organization with respect to the PreS$_1$-PreS$_2$-S protein coding region, is that of Cummings et al in Proc. Natl. Acad. Sci. USA, Vol. 77, p. 1842, 1980. Through subsequent BglII digestion, the HBV core antigen gene segment is eliminated, leaving a DNA fragment (2.78 kb) carrying the PreS$_1$-PreS$_2$-S protein coding region of the hepatitis B virus genome together with the natural promoter system (P$_{HB}$) of such region.

The linear pBPV342-12 BamHI fragment (see Figure 1) and the pAOI-derived BglII fragment (see Figure 2) are then ligated to produce the recombinant plasmid, pDM1 (see Figure 3), which retains the natural promoter (P$_{HB}$) for the Pres$_1$-PreS$_2$-S protein coding region.

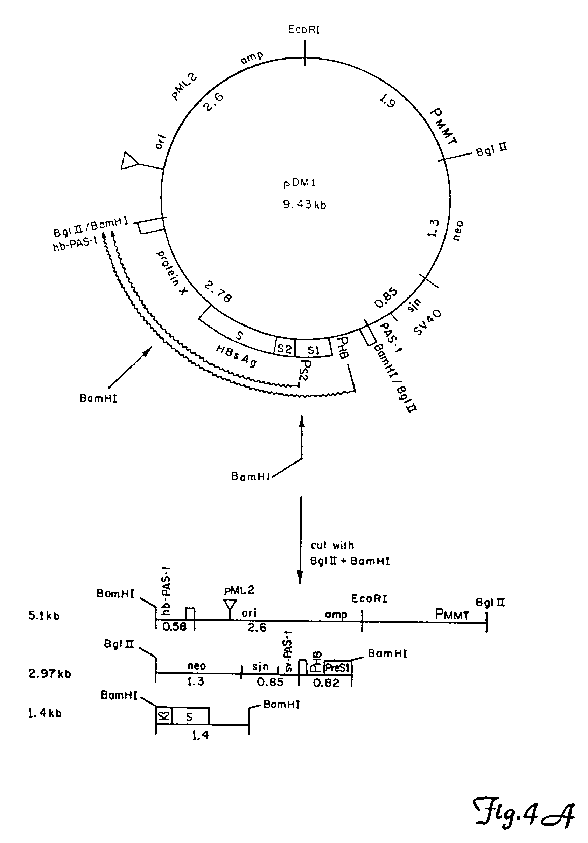

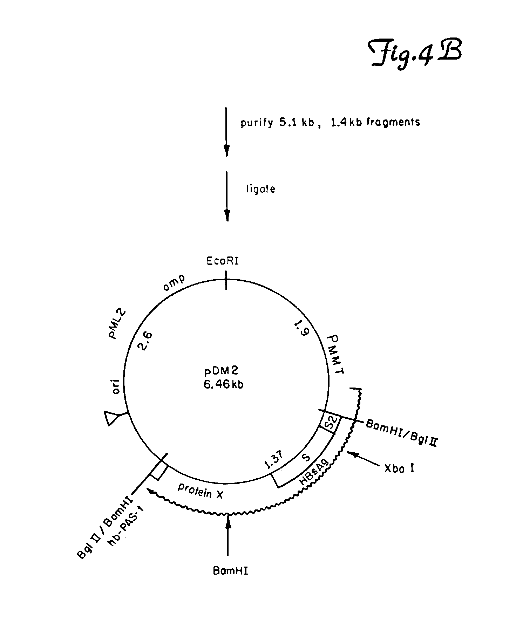

Then, the natural promoter in pDM1 is excised by a BglII and BamHI digestion such that the MMT-promoter is positioned directly ahead of the PreS$_2$-S protein coding region to control transcription of said coding region by the MMT-promoter. Following BamHI + BglII digestion three discrete DNA fragments are obtained (see Figures 4A and 4B):

1) a 5.1 kb fragment containing a polyadenylation site and the MMT-promoter;
2) a 3.0 kb fragment containing the neomycine resistance gene and the natural promoter; and
3) a 1.4 kb fragment containing the PreS$_2$-S protein coding region.

Following purification, the 5.1 and the 1.4 kb fragments are ligated using conventional techniques to produce recombinant plasmids (6.46 kb) containing the PreS$_2$-S protein coding region, now under the control of the MMT-promoter. Because the relative orientations of the two ligated fragments are random and two different plasmid types are produced, only one of which exhibits the correct transcriptional reading orientation from the MMT-promoter into the surface antigen gene sequences, the correct functional plasmid, pDM2, is ascertained by EcoRI + XbaI digestion of purified plasmid candidates. The plasmid with the correct orientation yields a 2.1 and a 4.4 kb fragment following this digestion.

A bacterial strain, HB101, containing pDM2, was deposited at Deutsche Sammlung für Mikroorganismen, Göttingen, under the depository number DSM 3285 on April 4, 1985.

Third Preferred Embodiment

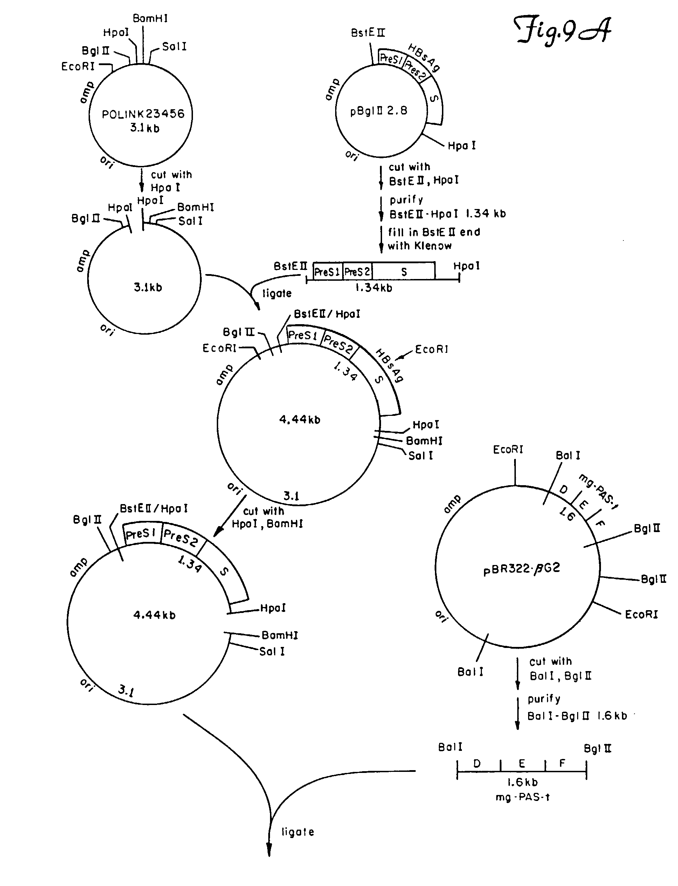

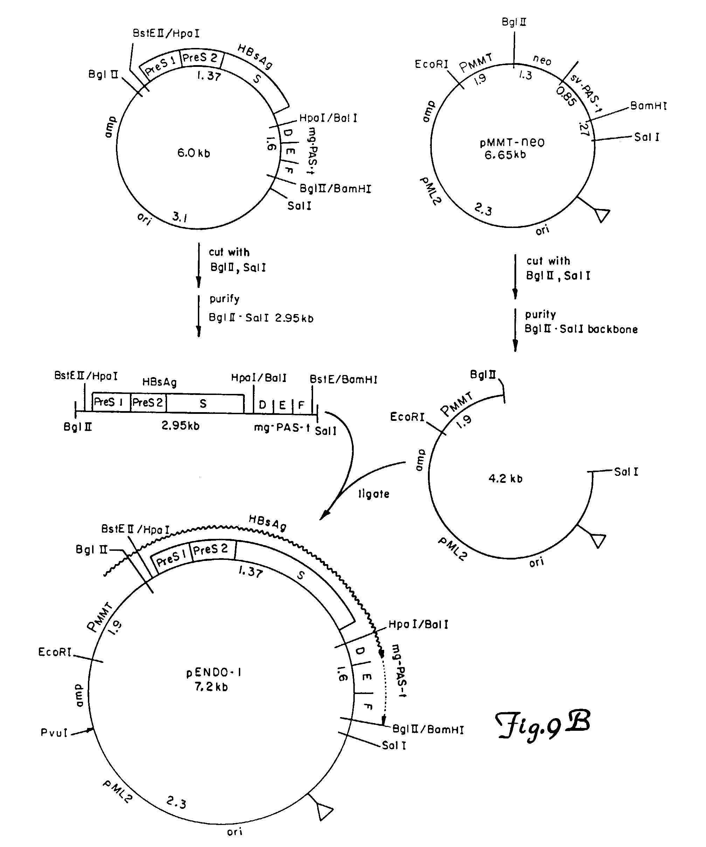

In a third preferred embodiment, the MMT-promoter is spliced to a HBV gene segment containing the $PreS_1$-$PreS_2$-S protein coding region in such a way as to eliminate the natural promoter system and place said coding sequence under the direct control of the MMT-promoter (see Figure 9).

Fourth Preferred Embodiment

In a fourth preferred embodiment, the mouse globin gene mg-PAS-t segment, recovered from plasmid pBR322. G2, is substituted as a polyadenylation-termination (PAS-t) signal for the SV40 PAS-t region. The substitution of mg-PAS-t provides DNA transfer vectors without any non-HPV viral genomic portions (see Figures 9, 10 and 11).

Fifth Preferred Embodiment

A fifth preferred embodiment is a method of preparing plasmid DNA mixed concatamers, amplifying the gene copy number by ligation of high ratios of plasmids containing gene constructs encoding the $PreS_1$-$PreS_2$-S, $PreS_2$-S or S protein coding regions to plasmids containing the drug-resistance marker, for subsequent transfection into cell lines.

Plasmids pBR322. G2, POLINK 23456, and pBlg12.8 were deposited at the American Type Culture Collection, Rockville, Maryland, under the accession numbers <u>67073</u>, <u>67072</u>, <u>67075</u>, respectively, on <u>April 11, 1986</u>.

In the following Examples, the preparation of the recombinant DNA vectors, hosts and particles of this invention, as well as the incorporation into mammalian cell lines is described in more detail. The following examples are for illustrative purposes only and are not intended to limit the invention in any way.

EXAMPLES

MATERIALS AND PROCEDURES

A. <u>ORIGIN AND DESCRIPTION OF MAMMALIAN CELL LINES.</u>

1. <u>L-Cells.</u>

NCTC clone 929 was derived in March 1948 by K.K. Sanford, W.R. Earle and G.D. Likely (<u>J. Nat. Cancer Inst.</u>, Vol. 9, pp. 229, 1948) from the parental strain L established in 1940 by W.R. Earle (<u>J. Nat. Cancer Inst.</u>, Vol. 4, pp. 165, 1943). The parent strain L was derived from normal subcutaneous areolar and adipose tissue of a 100 day-old male C3H/An mouse, and clone 929 was established (by the capillary technique for single cell isolation) from the 95th subculture generation of the parent strain.

LM(TK-), a sub-line of mouse L cells, was isolated by D.R. Dubbs and S. Kit (S. Kit et al, <u>Exp. Cell Res.</u>, Vol. 31, pp. 297-312, 1963; and D.R. Dubbs & S. Kit, <u>Exp. Cell Res.</u>, Vol. 33, p. 19, 1964), and is deficient in thymidine kinase. It is unable to grow in medium containing HAT. No spontaneous reversion of HAT-resistance has been observed in this cell line, and it seems most likely that a deletion involving this gene has occurred.

<u>Number of Serial Subcultures from Tissue of origin:</u> 648; clone, 553.
<u>Freeze Medium:</u> Culture medium, 90%; DMSO 10%; antibiotic-free.
<u>Viability:</u> 81-96% (dye exclusion).
<u>Culture Medium:</u> DMEM + 10% FBS (Dulbecco's and Vogt's Modified Eagle's Medium + 10% FBS).
<u>Growth Characteristics of Thawed Cells:</u> An innoculum of 6-8 x $10^5$ cells in 5 ml of above culture medium per T-25 flask, yields a 500-fold increase within 7 days at 37°C, provided the medium is renewed two tines weekly and the pH is adjusted to 7.3 with a humidified mixture of 5% or 10% carbon dioxide in air. Subcultures are prepared by scraping or shaking. Cells also grow well on other media (Waymouth's, Eagle's, NCTCl35, etc.) supplemented with 10%-30% horse serum.
<u>Plating Efficiency:</u> 70%
<u>Morphology:</u> Fibroblast-like.
<u>Karyology:</u> Chromosome Frequency Distribution 100

| Cells: | 2 | 2 | 1 | 4 | 2 | 9 | 4 | 12 | 16 | 17 | 4 | 13 | 2 |
|---|---|---|---|---|---|---|---|---|---|---|---|---|---|
| Chromosomes: | 56-58 | 59 | 60 | 61 | 62 | 63 | 64 | 65 | 66 | 67 | 68 | 69 |

| Cells: | 2 | 2 | 1 | 1 | 6 |
|---|---|---|---|---|---|
| Chromosomes : | 70 | 71-76-82-125-241 | | | |

Long metacentric chromosome with secondary constriction noted in 77/100 cells.

Sterility: Tests for mycoplasma, bacteria and fungi were negative.

Species: Confirmed as mouse by mixed agglutination and hemagglutination tests.

Virus Susceptibility: Susceptible to pseudorabies virus and vesicular stomatitis (Indian Strain) virus. When the cells were cultured in the above culture medium, Herpes simplex B virus, and vaccinia produced cytopathic effects in the first passage only. The susceptibility to certain viruses may vary with the culture medium employed. Not susceptible to poliovirus type 1, Coxsackie virus type B-5 and polyoma virus.

Tumorigenicity: An innoculum of $1 \times 10^6$ cells per mouse was injected subcuntaneously into nude mice. In non-irradiated mice, 0/25 tumors were produced. In mice x-irradiated (425 r, whole body) 11/18 tumors (sarcomas) were produced at the site of injection.

Reverse Transcriptase: Positive.

Submitted, Prepared and Characterized By: American Type Culture Collection, Rockville, Maryland.

2. Vero Cells.

The Vero cell line was initiated from the kidney of a normal, adult, African green monkey on March 27, 1962, by Y. Yasumuar and Y. Kawakita at the Chiba University in Chiba, Japan (Nippon Rinsho, Vol. 21, p. 1209, 1963).

Number of Serial Subcultures from Tissue of Origin: 121

Freeze Medium: Minimum essential medium (Eagle) with non-essential amino acids and Earle's BSS, 85%; fetal bovine serum, 5% dimethyl sulfoxide (DMSO), 10%; antibiotic-free.

Viability: Approximately 97% (dye exclusion).

Culture Medium: DMEM, 5% FBS

Growth Characteristics of Thawed Cells: An innoculum of $3 \times 10^5$ viable cells in 3 ml of the above culture medium per T-25 flask, yields a 30-fold increase within 7 days at 37°C, provided the medium is renewed three times weekly and the pH is adjusted to 7.4 with a humidified mixture of 5% or 10% carbon dioxide in air. Subcultures are prepared by trypsinization.

Plating Efficiency: Approximately 24% in above culture medium.

Morphology: Epithelial Fibroblast-like.

Karyology: Chromosome Frequency Distribution 50 Cells: 2n = 60.

| Cells: | 2 | 1 | 2 | 2 | 3 | 4 | 5 | 7 | 2 | 1 | 17 | 2 | 3 |
|---|---|---|---|---|---|---|---|---|---|---|---|---|---|
| Chromosomes: | 47-49 | 59 | 51 | 52 | 53 | 54 | 55 | 56 | 57 | 58 | 59 | 60 | |

Sterility: Tests for mycoplasma, bacteria and fungi were negative.

Species: Confirmed as monkey by immunofluorescence test.

Virus susceptibility: Susceptible to poliovirus type 3, Getah, NNdumu, Pixuna, Ross River, Semliki, Paramaribo, Kokobera, Modoc, Murutucu, Germiston, Guaroa, Pongola and Tacaribe Arboviruses. Not susceptible to Stratford, Apeu, Caraparu, Madrid, Nepuyo and Ossa Arboviruses.

Reverse Transcriptase: Not detected.

Submitted, Prepared and Characterized By: American Type Culture Collection, Rockville, Maryland.

3. CHO-KI Cells.

The CHO-KI cells were derived as a subclone from the parental CHO cell line initiated from a biopsy of an ovary of an adult Chinese hamster by T.T. Puck in 1957 (J. Exp. Med., Vol. 108, p. 945, 1958). The CHO-KI cells have a requirement for proline and a modal chromosome number of 20 (Proc. Mat. Acad. Sci., Vol. 60, p. 1275, 1968). The cells apparently lack the active gene form needed for proline synthesis and the block in the biosynthetic chain lies in the step converting glutamic acid to glutamic gamma-semialdehyde. The reversion frequency to proline independence is approximately $10^{-6}$ (Genetics, Vol. 55, p. 513, 1967).

Number of Serial Subcultures from Tissue of Origin: Approximately 400; 7 at ATCC.

Freeze Medium: Culture medium, 92%; glycerol, 8%; antibiotic-free.
Viability: Approximately 90% (dye exclusion).
Culture medium: F-12 Medium (Ham) 90%; fetal bovine serum, 10%; antibiotic-free.
Growth Characteristics of Thawed Cells: An innoculum of $10^5$ viable cells/ml in the above culture medium at 37°C increases 15-20 fold within 7 days.
Plating Efficiency: Approximately 90% in above culture medium.
Morphology : Epithelial-like.
Karyology: Chromosome Frequency Distribution 50 Cells: 2n = 22.

| Cells: | 2 | 2 | 35 | 3 | 4 | 1 | 1 | 1 | 1 |
|---|---|---|---|---|---|---|---|---|---|
| Chromosomes: | 18 | 19 | 20 | 21 | 22-24-32-39-42 | | | | |

Sterility: Tests for mycoplasma, bacteria and fungi were negative.
Species: Confirmed as Chinese hamster by cytotoxic-antibody dye-exclusion test and isoenzyme analysis.
Virus Susceptibility: Susceptible to vesicular stomatitis (Indiana Strain) and Getah arboviruses. Not susceptible to poliovirus type 2, Modoc and Button William arboviruses.
Reverse Transcriptase: Not detected.
Special Characteristics: The reference cells require proline for growth.
Submitted By: T.T. Puck, Eleanor Roosevelt Institute for Cancer Research, University of Colorado Medical Center, Denver, Colorado.

4. NIH/3T3 Cells.

The NIH/3T3, a continuous cell line of highly contact-inhibited cells was established from NIH Swiss mouse embryo cultures in the same manner as the original random bred 3T3 (ATCC CCL 92) and the inbred BALB/c3T3 (ATCC CCL 163). The established NIH/3T3 line was subjected to more than 5 serial cycles of subcloning in order to develop a subclone with morphologic characteristics best suited for transformation assays. The earliest available passages of this subclone are around 120 beyond the primary embryo culture. The NIH/3T3 is highly sensitive to sarcoma virus focus formation and leukemia virus propagation and has proven to be very useful in DNA transfection studies. J. Virol., Vol. 4, pp. 549-553, 1969; cell, Vol. 16, pp. 63-75 and 347-356, 1979.

B. MEDIA, BUFFERS AND SOLUTIONS.

1. For Mammalian Cell Culture.

a. Dulbecco's Modified Eagle Mediun, High Glucose (DMEM).
GIBCO dry powdered medium is prepared and supplemented with:

26 mM $NaHCO_3$
2 mM L-glutamine (Gibco)
1 mM Na-pyruvate (Gibco)
60 µg/ml gentamycine or 100 units/ml penicillin + 100 µg/ml
1 streptomycin

b. Ham's F12.
GIBCO dry powdered medium is prepared and supplemented with:

14 mM $NaHCO_3$
60 µg/ml gentamycin

c. Cell Freezing Medium.

90% (v/v) FBS-DMEM
10% (v/v) Dimethylsulfoxide

Freeze medium is prepared just before use. A11 media are filter sterilized through 0.45 and 0.2 micron filters (Gilman). Sterility of media is assessed by incubating an aliquot of antibiotic-free medium at 37°c for 1 week. Media used in static culture is supplemented with 10% FBS. Fetal bovine serum (FBS) is heat-inactivated at 56°C for 30 minutes. Sterile medium is stored at 40°C prior to use.

d. Trypsin Solution.

0.5 g/l trypsin

0.2 g/l EDTA (tetra sodium) in Hank's Balanced Salt Solution

Since EDTA was found to be toxic for Vero cells, EDTA-free trypsin solution is used with these cells.

e. PBS

(phosphate buffered saline, GIBCO)183 mM NaCl

$\qquad$ 8.6 mM $Na_2HPO_4$

$\qquad$ 2.2 mM $KH_2PO_4$

f.

TNE.   160 mM NaCl

$\qquad$ 10 mM Tris, pH 7.5

$\qquad$ 1 mM EDTA

2. For Molecular Biology.

a. Solutions for SDS-PAGE Procedures.

| | |
|---|---|
| Acrylamide (Stock) | 30% (w/w) Acrylamide (BioRad) |
| | 0.8% (w/w) N'N-Methylene-bisacrylamide (BioRad) |
| 4 x buffer for separation gel | 1.5 M Tris-Cl, pH 8.8 |
| | 0.4% (w/v) sodium-dodecylsulfate (SDS) (Serva) |
| 4 x buffer for spacer gel | 0.5 M Tris-Cl, pH 6.8 |
| | 0.4% (w/v) SDS |
| 3 x sample buffer | 22.3% (v/v) glycerol |
| | 0.03% (w/v) Bromphenolblue |
| | 6.7% (w/v) SDS |
| 10 x electrode buffer | 0.25% M Tris, pH 8.2 |
| | 1.90 M glycine |
| | 1% (w/v) SDS |

b. Solutions for Silver Staining Procedures.

| | |
|---|---|
| Solution I | 50% (v/v) methanol |
| | 10% (v/v) acetic acid |
| Solution II | 10% (v/v) methanol |
| | 5% (v/v) acetic acid |
| Solution III | 10% glutaraldehyde |
| Solution IV | 20% $AgNO_3$ in $H_2O$ (Stock) |
| Solution V | 3% (w/v/) $Na_2CO_3$ 0.1% (v/v) formaldehyde |

c. Solutions for Nick Translation.

| | |
|---|---|
| 10 x Reaction buffer: | 500 mM Tris-Cl, |
| | pH 7.2 |
| | 100 mM $MgSO_4$ |
| | 1 mM DTT |
| Nucleotide Mix: | 100 mM dGTP |
| | 100 mM dATP |
| | 100 mM dTTP |
| | in 10 mM Tris, |
| | pH 7.5 |

d. Solutions for Hybridization.

| | |
|---|---|
| 20 x SSC | 3 M NaCl |
| | 0.3 M $Na_3$-Citrate |
| 50 x Denhardt's solution | 1% (w/w) Ficoll 400 (PL-Pharmacia) |
| | 1% (w/w) Polyvinylpyrrolidone (Sigma) |
| | 1% (w/w) BSA |
| Sterile filtrated and stored at | -20°C |
| Prehybridization mix | 50% (v/v) Formamide |
| | 5 x Denhardt's solution |
| | 5 x SSC |
| | 50 mM Na-phosphate |
| | pH 7.0 |
| | 250 µg/ml denatured salmon sperm DNA |
| Hybridization mix | 50% Formamide |
| | 5 x SSC |
| | 20 mM Na-phosphate |
| | pH 7.0 |
| | 1 x Denhardt's solution |
| | 100 µg/ml denatured |
| | salmon sperm DNA |

e. Buffers for Restriction Enzymes. Manufacturers specifications, or:

| Low Salt Buffer | Medium Salt Buffer | High Salt Buffer |
|---|---|---|
| SmaI | BglII | BamHI |
| HpaI | PstI | PvuI |
| XbaI | EcoRI | SalI |
| SpeI | | BstEII (60°C) |

Restriction Buffers:

| | Low | Medium | High |
|---|---|---|---|
| Tris, pH 7.4 | 10mM | 6.6mM | 6.6mM |
| $MgCl_2$ | 10 | 6.6 | 6.6 |
| salt | 20 (KCl) | 60 (NaCl) | 150 (NaCl) |
| β-mercaptoethanol | 10 | 6.6 | 6.6 |

f. Media.

1. L-Broth: 10 g Bactotryptone
    5 g yeast extract
    10 g NaCl
    1 liter $H_2O$
2. L-Broth agar plates: L-Broth containing 15 g/l Difo agar.
3. L-Broth-amp agar plates: L-Broth agar containing 20-50 µg/ml ampicillin.

C. ENZYMES.

The following enzymes are used:

1. Deoxyribonuclease I (Worthington)
2. DNA-Polymerase I ("Klenow-Fragment") (Boehringer Mannheim)
3. T4-DNA Ligase (Boehringer Mannheim)
4. Restriction enzymes:

ECORI, Xbal, BamHI, BglII, BstEII, Spel, Sall, Hpal, Smal, Pvul, Pstl (Boehringer Mannheim, BRL, New England BioLabs)

5. Lysozyme (SIGMA)

6. Pronase

D. ANALYTICAL AND QUANTITATIVE PROCEDURES.

1. Dye Binding Assay.

The concentration of total protein in preparations of purified particles of this invention is determined using the Dio-Rad protein assay kit. This method utilizes the Bradford assay, a spectrophotometric means of measuring Coomassie Blue binding to protein. Ovalbumin is used as a standard.

2. Radioimmune Assay (RIA).

In the NML RIA 125-I "sandwich" radioimmunoassay, beads coated with mouse antibody to hepatitis B surface antigen (anti-HBs) are incubated with serum or plasma and appropriate controls. Particles containing polypeptides encoded by the $Pres_1$-$PreS_2$-S protein coding region are bound to the solid phase antibody. After aspiration of the unbound material and washing of the bead, human $^{125}$I-Anti-KBS is allowed to react with the antibody-antigen complex on the bead. The beads are then washed to remove unbound $^{125}$I-Anti-HBS.

The radioactivity remaining on the beads is counted in a gamma scintillation counter. Specimens giving counts per minute (cpm) greater than or equal to the cut-off value determined by multiplying the negative control mean count rate (NCx) by a factor are considered reactive.

3. Immunoprecipitation.

Cells are grown to 100% confluency in a T75 flask. The culture medium is then replaced with 5 ml of methionine-free medium containing 400 uCi of L-[35S] methionine and 400 uCi of L-[35S] cysteine (NEN) for overnight incubation. The medium is concentrated 10-fold by fractional precipitation with polyethylene glycol. Concentrated protein (about $10^6$ cpm) is incubated with 10 µl of pre-immune guinea pig serum or with 1 and 10 µl of anti-HBsAg guinea pig serum in 50 µl of TDN (10mM Tris, pH 7.4, 1mM EDTA, 130 mM NaCl) - 0.5% Tween 20 for 1 hour at 37°c. The immunoglobulin is bound to 20 µl of protein A-Sepharose C1-4B (Pharmacia) for 1 hour at 37°c, washed once with TEN-Tween 20 and 500 mM LiCl and again with TEN-Tween 20. Samples are electrophoresed in the SDS-PAGE system described below. Gels are fixed for 60 minutes in a solution of 10% tricholoro-acetic acid, 10% glacial acetic acid, and 30% methanol and incubated in Enlightning (NEN) for 30 minutes. Gels are dried and autoradiographed with KODAK XAR-5 film.

4. SDS-Polyacrylamide Gel Electrophoresis (PAGE).

Sample and Gel Preparation. For analysis by PAGE, aliquots of purified particles of this invention are adjusted to 10 µg/ml. From each sample, 10 µl are taken and mixed with 10 µl 1 M DTT in 10% SDS and 10 µl sample buffer. This mixture is boiled 5, 10 or 15 minutes just before loading in order to break the 20 nm particles and to generate monomeric molecules. After adding 10 µl of loading buffer, the mixture is electrophoresed in a 0.75 mm thick slab gel (12 x 18 x 0.1 cm) of 12.5% polyacrylamide - 0.4% bisacrylamide, using the Laemmli (1970) buffer system at 15mA and 100 V for 6 hours. Protein bands are visualized by silver staining (see Silver Staining section below).

5. Silver Staining.

The PAGE gel is stained with silver according to Merril et al. (1981). The protein is fixed in the gel with a solution of 50% methanol and 10% acetic acid for 30 minutes. The gel is washed with a solution of 10% methanol and 5% acetic acid for 30 minutes. After incubation in 10% glutaraldehyde for 30 minues, the gel is washed in deionized $H_2O$ overnight. Following incubation in 0.1% $AgNO_3$ for 30 minutes, the gel is subjected to a short wash in $H_2O$. The stained gel is developed in 100 ml of 3% $Na_2CO_3$ and 30 µl formaldehyde and fixed in 1% acetic acid. The gel is sealed in a plastic bag and photographed.

E. Genetic Engineering Procedures.

1. Recombinant DNA Procedures.

a. Restriction Endonuclease Digestion of Purified DNA.

Per manufacturer's specifications of the particular endonuclease.

b. Plasmid DNA Isolation.

Large Scale CsCl Plasmid Preparation:

1. Grow 1 liter of plasmid-bearing cells to 0.5 $OD_{600}$ in L-Broth, amplify 12-20 hours with 200 µg/ml chloramphenicol.

2. Centrifuge in Sorval RC5b, 8,000 rpm for 20 minutes.

3. Resuspend in 18 ml cold 25% sucrose, 50mM Tris, pH 8.0.

4. Transfer to 250 ml Erlenmeyer flask. Keep on ice.

5. Add 6 ml 5mg/ml lysozyme in 250mM Tris, pH 8.0, let stand 10-15 minutes.

6. Add 6 ml 250mM EDTA, pH 8.0 and mix gently; incubate 15 minutes on ice.

7. Add 30 ml detergent mix:

    0.01% Triton X-100

    60mM EDTA, pH 8.0

    50mM Tris, pH 8.0

8. Incubate 30 minutes on ice.

9. Centrifuge 25,000 rpm. 90 minutes in SW28 rotor, 4°C.

10. To supernatant fluid, add pronase to 250 $\mu$g/ml and incubate 30 minutes, 37°C.

11. Phenol extract once with 1/2 volume phenol equilibrated with TE (10mM Tris, pH 8.0, 1mM EDTA).

12. Remove aqueous layer; add sodium acetate to 300mM; add 2 volumes cold 100% ethanol; mix thoroughly. Hold at -20°C overnight.

13. Centrifuge; resuspend in 6 ml TE-10 (10mM Tris, 10mM EDTA, pH 8.0).

14. Add 9.4g CsCl, 0.65 ml of 6 mg/ml ethidium bromide; make up to 10 ml volume with sterile double-distilled water.

15. Fill Beckman heat-sealable gradient tubes; centrifuge 48,000 rpm, 40 hr. in Ti70.1 Beckman rotor.

16. Visualize plasmid bands with UV and remove plasmid DNA with syringe and 18 gauge needle by piercing the side of the tube.

17. Remove ethidium bromide from the plasmid fraction by 3 successive extractions with equal volumes of isobutanol.

18. Dialyze against one 2-liter lot of 10mM Tris, pH 7.4, 1mM EDTA, pH 7.5, 5mM NaCl for 2 hours or more at 4°C.

19. Phenol extract once with 1/3 vol. phenol equilibrated with TE as above.

20. Add NaAc to 300mM, add 2 vol. 100% ethanol; precipitate at -20°C overnight, or at -70°C for 30 minutes.

c. <u>Nick Translation</u>. Nick-translation is performed according to Rigby et al (J. <u>Mol. Biol.</u>, Vol. 113, pp. 237-251, 1977). The reaction mixture for $^{32}$P-labeling of DNA usually contains 0.5 $\mu$g of, for example, the <u>ECORI</u>-<u>Xbal</u> fragment of pDM2, in a total volume of 30 $\mu$l with 50 mM Tris, pH 7.8, 5 mM MgCl$_2$, 10 mM mercaptoethanol, 0.1 mM dATP, 0.1 mM dGTP, 0.1 mM dTTP, 50 uCi $^{32}$P-dCTP, 10 units DNA polymerase I, 3 $\mu$l of a $2 \times 10^{-5}$ fold dilution of 1 mg/ml DNase I and is incubated for 90 minutes at 15°C, yielding $3 \times 10^6$ to $12 \times 10^6$ total cpm, i.e. $1 \times 10^7$ to $5 \times 10^7$ cpm/$\mu$g DNA.

d. <u>Transformation of Competent Bacterial cells.</u> From a dense overnight culture, 1 ml of the bacterial cell suspension is taken for innoculation of 100 ml growth medium (L-broth). The cells are grown at 37°C to a density of OD$_{550}$ = 0.7 which is reached within 2 hours with vigorous shaking in a 500 ml Erlenmeyer flask. Growth is stopped by chilling the culture on ice for 10 minutes. From this culture, 3 ml is taken for harvesting the exponential bacterial cells at 3,000 rpm for 5 minutes. The cells are resuspended in 1.5 ml of 50 mM CaCl$_2$ in 10 mM Tris, pH 8.0, and incubated on ice for another 15 minutes. The cells are harvested once more by centrifugation of 3,000 rpm for 5 minutes and resuspended in 200 $\mu$l of 50 mM CaCl$_2$ in 10 mM Tris, pH 8.0, and kept at 4°C overnight.

Thereafter, the ligation mixture was filled up with 10 mM tris, pH 7.5, 1 mM EDTA, to a total volume of 70 $\mu$l and added to the 200 $\mu$l bacterial cell suspension for DNA take-up.

The mixture was incubated on ice for 30 minutes, then 1 ml L-broth was added and the mixture was incubated at 42°C for 2 minutes and at 37°C for 40 minutes. After the incubation, the cells were spread on agar plates containing 50 $\mu$g ampicillin/ml agar at volumes of 50 $\mu$l up to 300 $\mu$l of the cell suspension per plate. The agar plates were incubated at 37°c overnight. After this incubation period, single isolated bacterial colonies were formed.

e. <u>Southern Blot Analysis.</u> To characterize the organization within the host cell gerome of the vectors of this invention, chromosomal DNA from cell lines producing particles of this invention is isolated and digested with the appropriate restriction enzyme(s) and analyzed by the method of Southern (<u>J. Mol. Biol.</u>, Vol. 98, pp. 503-517, 1975) using a $^{32}$P-labeled DNA probe. Following digestion of the chromosomal DNA (20 $\mu$g) with restriction enzymes <u>EcoRl</u> and <u>Xbal</u>, the resulting fragments are separated by 0.7% agarose gel electrophoresis. Thereafter, the DNA is denatured by exposing to 366 nm UV light for 10 minutes and by incubation in a solution of 0.5N NaOH and 1 M Nacl for 45 minutes. The gels are neutralized by incubation in 0.5 M Tris, 1.5M NaCl, pH 7.5 for 60 minutes. The DNA is transferred to a nitrocellulose filter by soaking in 3 M NaCl, 0.3 M NaCitrate (20XSSC) for 20 hours through the gel by covering the top of the nitrocellulose filter with a staple of dry paper towels. The nitrocellulose filter is kept for 2 hours in a vacuum oven at 80°c.

The radioactive DNA probe from the ECORI-XbaI fragment of the pDM1 plasmid (4.3 kb) is prepared by nick-translation.

For hybridization with the DNA probe, the nitrocellulose filter is sealed in a plastic bag containing 10 ml of prehybridization mixture : 50% formamide, 5 x SSC, 50 mM sodium-phosphate, pH 7.0, 5 x Denhardt's solution, 250 µg/ml denatured salmon sperm DNA. The filter is incubated in this mixture for 4 hours at 45°C, after which the pre-hybridization mixture is replaced by the hybridization mixture: 50% formamide, 5 x SSC, 20 mM Na phosphate, pH 7.0, 1 x Denhardt's solution, 100 µg/ml denatured salmon sperm DNA, 5 x $10^5$ cpm/ml $^{32}$p-probe. The filter, after incubating in the hybridization mix for 18 hours at 45°C, is washed three times, 5 minutes each, in 0.1 x SSC, 0.1% SDS at 50°c. The filter is dried at 60°c for 10 minutes and exposed to two X-ray films (XAR-5, KODAK) between two intensifying screens and kept at -80°C. The first X-ray film is developed after 3 days' exposure; the second film after 7 days' exposure. Hybridization of the labeled DNA probe to one of cellular DNA restriction fragments from transfectants which produce particles of this invention demonstrates that the cellular DNA does in fact contain the integrated vector of this invention. Since the cellular DNA restriction pattern, as elucidated by the Southern blot procedure, is identical to that of the original plasmid construct, no major rearrangement of plasmid DNA occurred upon integration into the host cell DNA.

## 2. Transfection of Mammalian Cell Lines and Identification of Clones Producing Particles of this Invention.

Cells are transfected using the calcium phosphate precipitation method of Wigler et al (Cell, Vol. 14, p. 725, 1978). Cells are then split 1:6 into new culture dishes, and cells which have received the drug-resistance marker gene (neo) are selected by growth in G418-containing medium. After 10 days of growth in selective medium, drug-resistant colonies are cloned into micro-titer plates. Levels of expression of the particles of the present invention are assessed by the RIA technique after the cells have reached confluency.

Tissue culture dishes (100 mm diameter) are innoculated with 5 x $10^5$ cells and incubated overnight at 37°C. Four hours prior to transfection, the culture medium is replaced with fresh medium. DNA to be transfected is suspended in 1 ml of solution containing 250 mM $CaCl_2$, 140 mM Nacl, 25 mM Hepes, pH7.1 and 0.75 mM $NaHPO_4$. This calcium phosphate - DNA precipitate is added to the cells in culture. After an overnight incubation, fresh medium is added to the cell culture and the cells are incubated for an addition two days.

## F. Procedures for static Cell Culture of Mammalian Cells.

An ampule of cells held at 196°C in liquid nitrogen is thawed quickly by placing the ampule in a 37°C water bath for 5 minutes. One ml of the freshly-thawed cell suspension is diluted by slowly adding 10 ml of the appropriate culture medium. Thereafter, the cells are harvested by centrifugation, resuspended in 10 ml of culture medium. Thereafter, the cells are harvested by centrifugation, resuspended in 10 ml of culture medium, transferred to a T25 culture flask, and grown in an incubator at 37°C and 5% $CO_2$. With these conditions, the doubling time is in the range of 15 to 20 hours for L-cells and CHO cells, and 30 to 40 hours for Vero cells. L-cells and CHO cells can be kept alive and growing beyond 100% confluency, whereas Vero cells are passaged at 100% confluency.

## G. Procedures for Mammalian Cell Growth in ACUSYST-Pand Harvest of Particles of this Invention.

### 1. Growth and Preparation of Innoculum.

Cells are maintained in T75 or T150 flasks containing DMEM-10% FBS. Roller bottles (850 cm$^2$) are innoculated with 10-15 x $10^6$ cells harvested from T-flasks. After 3 to 5 days of growth, approximately $10^9$ total cells are harvested from six roller bottles and used to innoculate six hollow fiber cartridges (HFC's) in an ACUSYST-P made by Endotronics, Inc. of Minneapolis, Minnesota U.S.A.

### 2. ACUSYST-P Culture and Harvest of Particles of this Invention.

After inoculation, the HFC's are inserted into the ACUSYST-P culture system, and the medium flow rate through the cartridges is adjusted to and mintained at 50 ml/hr. Medium is assayed daily for pH, $pO_2$, glucose and lactate. After 2 to 3 weeks of growth, a 600 ml fluid volume is harvested two times weekly from the extracapillary space of each ACUSYST-P hollow fiber cartridge. Production of particles of the subject invention is assayed by the radioimmunoassay (RIA). Since freezing destroys RIA assayable activity, harvests are stored at 4°C prior to purification. No protease activity can be detected under these conditions as assayed by Azocoll substrate proteolysis (SIGMA).

### 3. Quality Control Procedures.

a. Tests for the Presence of Bacteria and Fungi. A pool of cells containing about 5 x $10^5$ cells per ml, suspended in the cell culture medium from which they were harvested, is tested for the presence of bacteria and fungi. One ml of cells is streaked onto trypticase soy agar (BBL) plates, and another 1 ml is innoculated into thioglycolate medium (DIFCO) to test for the presence of aerobic as well as anaerobic bacteria. Fungal contamination is assayed by streaking 1 ml of cells onto Sabouraud (DIFCO) agar plates. These tests were performed on a regular basis to ensure sterility of growth medium and absence of bacterial and fungal contamination in cell cultures.

b. Test for the Presence of Mycoplasma: Fluorescence Staining. The presence of mycoplasmal DNA in the cell cytoplasm is determined by a staining method using the fluorochrome, Hoechst 33258. This DNA-staining dye fluoresces under ultraviolet light and provides the basis for a very rapid and sensitive test for mycoplasmas. The procedure involves coverslip cultures of the cells to be tested which are used when 50-70% confluent. After fixing and staining, the coverslips are examined with a fluorescent microscope. Cytoplasmic fluorescence indicates the presence of mycoplasma.

The stock of the Hoechst 33258 bisbenzamide fluorochrome solution is made by dissolving 5 mg in 100 ml PBS using a magnetic stirrer. It is sterilized by filtration through a 0.22 um membrane stored in the dark at 4°C, and diluted a thousand-fold with PBS before use. The medium from coverslip cultures, which are 50-70% confluent, is aspirated from the cells and fixed with two changes of ethanol:acetic acid (3:1) at 4°C. Following one wash with deionized water and incubation for 30 minutes at 37°C with diluted bisbenzamide fluorochrome (Hoechst-33258), the coverslips are rinsed with deionized water. The coverslips are mounted, cell-side down, on a slide using a glycerol mountant (22.2 ml 2.1% citric acid; 1 ml $H_2O$; 27.8 ml 2.8% $Na_2HPA_4$; 50 ml glycerol pH 5.5).

c. Tests for the Presence of Viruses.

1) Tests in Cell Cultures. Tester cells are examined for normal morphology during an incubation period of at least 14 days after innoculation with a suspension of the cell line being tested. In addition, on days 3-5, and again after the 12th day, at least 4% of the inoculated tester cultures is washed and tested for hemadsorption using red cells from sheep or humans. Tests are read after the cultures have incubated for 30 minutes at 3°-4°C and again after 30 minutes at 34°-37°C. Results of tests for virus-infected cells which absorb erythrocytes indicate no viral contamination.

2) Tests in Animals. Cells to be tested are suspended in 140 mM NaCl, 2.7 mM KCl, 8.1 mM $Na_2HPO_4$, pH 7.2 at a concentration of $10^6$ per ml and are inoculated into different groups of animals. In one group, at least 10 animals from at least two litters of suckling mice are innoculated. Each animal is inoculated with 0.1 ml cells intraperitoneally and 0.01 ml intracerebrally. The mice are observed daily for at least 14 days. Each mouse that dies after the first 24 hours of the test, or is sacrificed because of illness, is necropsied and examined for evidence of viral infection. Such examination involves additional testing by intracerebral and intraperitoneal subinnoculation of appropriate tissue suspensions into an additional group of at least five suckling mice following by daily observations for 14 days. In addition, a blind passage is made from a single pool of the emulsified tissue (minus skin and viscera) of all mice surviving the original 24-day test.

In another group, 10 adult mice are also innoculated. Each animal is inoculated with 0.5 ml cells intraperitoneally and 0.03 cells intracerebrally. The animals are observed for four weeks and any that become sick or show abnormality is examined to establish the cause of illness. Results of the tests in animals for the presence of contaminating viruses indicate no viral contamination.

d. Testing for Tumorigenicity. A suitable test for tumorigenicity using nude mice (nu/nu) involves innoculating 20 animals within 24 hours of birth with 0.1 ml of potent serum. The injection is given either by the intramuscular or subcutaneous route and is repeated on days 2, 7 and 14 of life. One million reference tumor cells routinely produces progressively growing tumors and metastases. One trillion viable cells of the candidate cell line are inoculated by the subcutaneous route at any site at which developing tumors can be palpitated (the limbs are suitable). The animals are observed for 21 days for evidence of nodule formation at the site of injection, and measurements are made periodically to determine whether there has been progressive growth. At the end of the 21-day observation period, all animals are sacrificed and examined for gross evidence of tumor formation at the site of injection and in other organs such as the lymph nodes, lungs, kidneys and liver. All tumor-like lesions and all innoculation sites are examined histopathologically. In addition, since some cell lines may form metastases without evidence of local tumor growth, the lungs and regional lymph nodes of all animals are examined histologically.

For the purpose of this test, a progressively growing tumor is defined as a palpable nodule that increases in size over the 21-day observation period and that shows viable and mitotically active innoculated cells when examined histologically. The presence of microscopically viable cells in association with a stationary or regressing nodule is not considered a progressively growing tumor.

### H. Purification of the Particles of this Invention.

#### 1. Fractional Precipitation with Polyethylene Clycol (PEG).

Medium from the extracapillary space is harvested from the ACUSYST-P culture system and pooled into volumes of 3000 ml. To each volume, 180 g of PEG 8000 (SIGMA) are added, dissolved by stirring at room temperature for 20 minutes and stirred for another 3 hours at 4°C. The precipitate is collected by centrifugation in 500 ml bottles in a GS 3 rotor at 4500 rpm (3000 x g) for 30 minutes at 10°C. The supernatant is collected and 180 g of PEG 8000 are again added and dissolved at room temperature as described above. The solution is stirred at 4°C for an additional 3 hours. The precipitate from this solution is collected as described above except that centrifugation is performed at 9000 rpm (15,000 x g) for 60 minutes. The pellet is resuspended in 20 ml of phosphate buffered saline (PBS).

#### 2. Gel Filtration Chromatography.

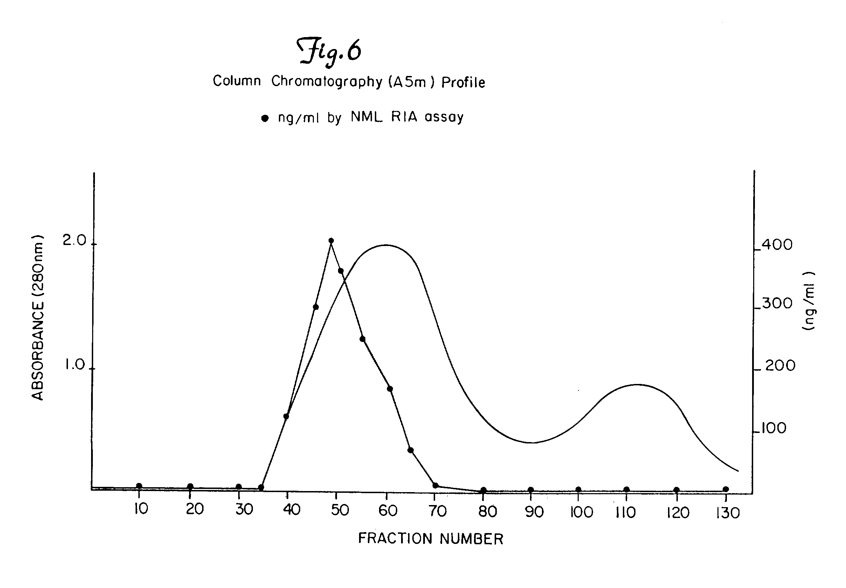

The material obtained after PEG precipitation, and redissolved in PBS, is submitted to gel filtration chromatography using BioRad A-5m resin at 4°C. Column dimensions are 25 x 1000 mm, and the bed volume is 480 ml. In a typical fractionation run, 1000 $\mu$g PEG-precipitated particles of this invention in a volume of 10 to 15 ml is loaded and eluted with PBS or TNE at a speed of 6 drops/minute (18 ml/hr) in fractions of 3 ml. Figure 6 shows the profile of RIA-assayable material eluted from an A-5m column. The solid line indicates the 280 nm absorbance of each fraction, and the dots indicate amount of material calculated from the RIA results.

#### 3. Isopycnic Centrifugation in CsCl.

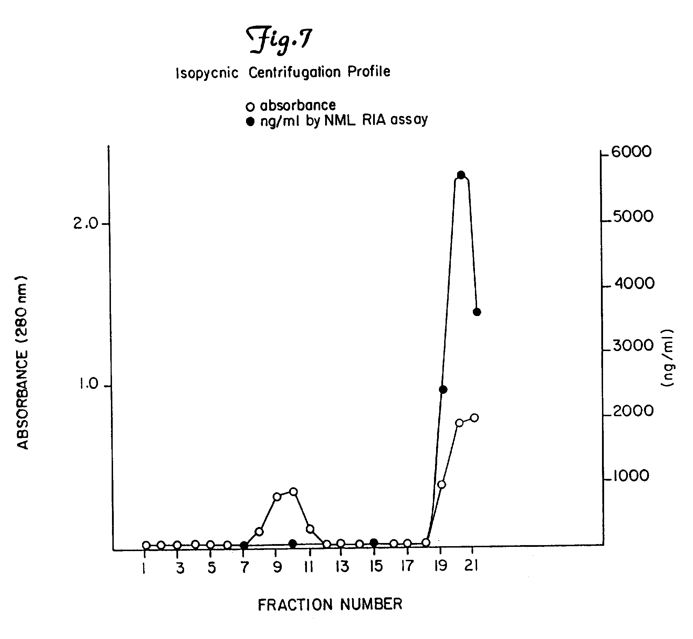

About 30 fractions covering the first peak resulting from gel filtration column chromatography and containing partially purified particles of this invention are pooled (approximately 100 ml). This solution is adjusted to a density of 1.30 g/cc with CsCl and subsequently transferred to nitrocellulose tubes fitting into a SW 27/28 rotor (Beckman). Gradients are set by underlaying 4 ml of a CsCl solution of 1.35 g/cc and by overlaying 4 ml of 1.25 g/cc and 4 ml of 1.20 g/cc density. Gradients are run at 28,000 rpm for 50 hours at 10°C, fractionated, and the purified particles in the 1.20 g/cc density layer at the top of the gradient (Figure 7) is collected. Purified particles are well separated from the small amount of contaminating protein banding in the middle of the gradient. The solution was desalted by dialysis, first against water, then against 3 changes of saline, over a 24-hour period.

As a further quality control measure, a portion of this gradient-purified material is submitted to linear CsCl-gradient centrifugation. A single peak of purified particles appears as expected at 1.2 g/cc.

### I. Preparation of the Adjuvant of Purified Particles of this Invention and Stability Testing.

In order to prepare an adjuvant of the vaccine of this invention, 1/10,000 volume Thimerosol, and 1/10 volume of filter-sterilized 0.2 M Al K(So$_4$)$_2$:12 H$_2$O are added to the desired concentration of antigen in sterile saline. The pH is adjusted to 5.0 with sterile 1N NaOh and the suspension is stirred at room temperature for 3 hours. The alum-precipitated antigen is recovered by centrifugation for 10 minutes at 2000 rpm, resuspended in sterile normal saline containing 1:10,000 Thimerosol, and aliquoted under sterile conditions.

To determine the stability of the particles of this invention absorbed to alum, the antigen was recovered by resolubilizing the alum in 3% sodium citrate followed by successive dialyses against 3% sodium citrate and PBS. The quantity of particles of this invention is then determined by parallel-line radioimmune assay against dilutions of a purified HBSAg standard in PBS-50% newborn calf serum.

Table 1

Stability Testing Parameters

Samples: two each of bulk and absorbed

Sample Concentration: 5 $\mu$g/ml

Storage Container: glass vials

Storage Temperature: 2°-8°C

Tests : (a) Qualitative: SDS-PAGE

(b) Quantitative: Radioimmunoassay

Example 1

Expression of Particles Comprising Pres1-PreS2-S Protein Coding Region Polypeptides

A. Preparation of Recombinant Plasmid pMMT-neo.

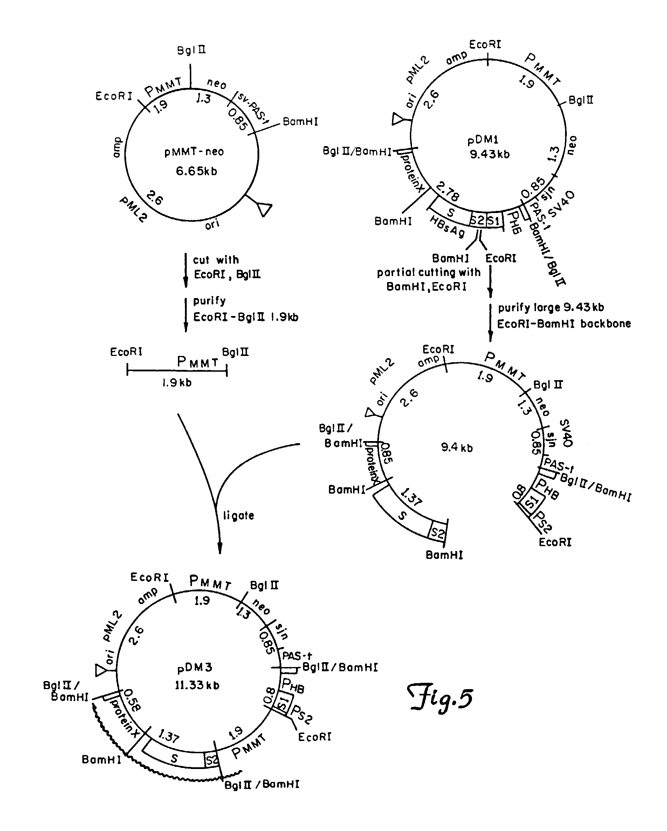

The plasmid pBPV342-12 was digested with the restriction-endonuclease BamHI (see Figure 1 and Materials and Procedures). Two DNA molecules were generated: one molecule (7.95 kb) comprises the entire genome of bovine papilloma virus (BPV); the other molecule (6.65 kb) contains part of the bacterial plasmid pML2 (pBR322 deleted for the "poison sequence"), the mouse metallothionein promoter (pMMT), the neomycine resistance gene (neo) and the SV40 PAS-t (see Figure 1). When ligated to itself (circularized), this fragment gives rise to plasmid pMMT-neo (see also Figure 5) (ATCC No. 67074).

The reaction was performed in a total volume of 400 µl of reaction buffer (see Materials and Procedures) at a final concentration of 0.2 µg/µl pBPV342-12 DNA; and 80 units of BamHI at 37°C for 4 hours. The completion of the digest was checked by agarose gel electrophoresis in a 0.7% agarose gel (see Materials and Procedures). The reaction was stopped by adding 40 µl of 8 M LiCl and the DNA was precipitated with 1 ml of ethanol at -80°C for 30 minutes. The precipitated DNA was resuspended in 100 µl of 10 mM Tris, pH 7.8.

B. Isolation of a Fragment Containing the Entire PreS1-PreS2-S Protein coding Region Along with the Natural Promoter of Transcription.

The preparation and cloning of the HBV genome, subtype adw, is described by Cummings, I.W. et al in Proc. Natl. Aca. Sci., U.S.A., Vol. 77, p. 1842, 1980. The circular HBV genome was linearized at the unique EcoRI site for cloning into bacteriophage lambda gtWES and for subcloning in a hacterial plasmid to yield plasmid pAOl (see Figure 2). Since the ECORI restriction site lies within the $Pres_1$-$PreS_2$-S protein coding region, the latter is interrupted when in the EcoRI linear form. To regenerate an intact $PreS_1$-$PreS_2$-S protein coding region, the 3.2 kb EcoRI insert of pAO1 was isolated and separated from the plasmid DNA by digesting 100 µg of pao1 in a total volume of 400 µl of reaction buffer (see Materials and Procedures) containing 200 units EcoRI for 4 hours at 37°C. The 3.2 kb insert DNA was separated from the plasmid DNA by preparative 0.7% agarose gel electrophoresis (see Materials and Procedures). The DNA was electro-eluted from the agarose gel on DE 81 Whatman ion exchange filter from which the DNA is removed in a high salt solution. The DNA was purified by one phenol/chloroform extraction and two ethanol precipitations. The purified linear 3.2 kb EcoRI fragment encoding the complete HBV genome was then subjected to self-ligation at a high DNA concentration in order to favor formation of concatamers: 30 µg of EcoRI fragment DNA was ligated in a total volume of 50 µl of reaction buffer containing 1.8 units T4 DNA ligase and 2 mM ATP at 15°C for 1 hour and at 4°C overnight; thereafter, the DNA was purified by two phenol/chloroform extractions, one chloroform extraction, followed by two ethanol precipitations. The pelleted DNA was resuspended in 20 µl of 10 mM Tris, pH 7.8, and digested with restriction enzyme BglII in a total volume of 50 µl of reaction buffer (see Materials and Procedures) containing 50 units of BglII at 37°C for 4 hours.

The DNA fragments generated by this reaction were separated by 1.5% agarose gel electrophoresis. The 2.78 kb size BglII fragment containing the intact $PreS_1$-$PreS_2$-S protein coding region, together with its natural promoter system necessary for expression of the surface antigen polypeptides, was isolated from the agarose gel and purified as described above (see Figure 2).

C. Insertion of the Fragment Containing the PreS1-PreS2-S Protein Coding Region into the pMMT-neo Plasmid: Construction of pDM1.

From a DNA solution prepared as described under part A. above, 1 µl was taken and mixed with 5 µl of the 2.78 kb BglII fragment (0.25 µg/µl) described under part B. above.

The mixture was submitted to a ligation reaction at a total volume or 10 µl of reaction buffer containing 0.9 units of T4 DNA ligase at 15°C for 1 hour and at 4°C overnight.

Bacterial cells, preferably HB101, were made competent for taking up DNA in a transformation reaction according to the method described in Materials and Procedures. The ligation mixture was made 10 mM Tris, pH 7.5, 1 mM EDTA, in a total volume of 70 µl and added to 200 µl competent bacterial cell suspension for DNA take-up.

The mixture was incubated on ice for 30 minutes, then 1 ml L-broth was added and the mixture was incubated at 42°C for 2 minutes and at 37°C for 40 minutes. After the incubation, the cells were spread on LB agar plates containing 50 µg ampicillin/ml at volumes of 50 µl up to 300 µl of the cell suspension per plate. The agar plates were incubated at 37°C overnight. After this incubation period, single isolated bacterial colonies were screened for the presence of the pMMT-neo bacterial plasmid containing the desired 2.78 kb BglII DNA fragment insert (see Figure 3).

Screening was preferentially performed by the colony hybridization procedure. For this procedure, single colonies were picked with a toothpick and transferred to an LB-ampicillin-containing agar plate in a grid format to allow identification of the clones. The plate was incubated overnight at 37°C.

The colonies were transferred to a nitrocellulose filter by layering the filter on the agar surface until the filter was wet. The filter was quickly peeled off and sequentially transferred to three layers of Whatman 3M paper, each of them either soaked in 0.5 M NaoH, or 1 M Tris, pH 7.0; 1.5 M Nacl/0.5 M Tris, pH 7.4, or 2 X SSC. The filters were placed on the soaked paper, colony-side up, during the lysis of the cells and the following neutralization and fixing procedures.

After the filters were dried in air, they were baked at 80°C in vacuum. Thereafter, the nitrocellulose filters were submitted to DNA hybridization with a radioactively labeled DNA probe made from the 2.78 kb $Bgl$II fragment containing the Pres$_1$-PreS$_2$-S Protein coding region, Prepared as described in part B above, by nick-translation (see Materials and Procedures).

The nitrocellulose filter was incubated in a pre-hybridization mix containing 50% formamide, 20 mM sodium phosphate buffer, pH 6.6; 1 X Denhardts solution; 100 µg/ml denatured salmon sperm DNA and 1 x $10^7$ cpm $^{32}$P-labeled DNA which was melted in 0.2 N NaOH at 68°C for 10 minutes.

The filter was incubated in this mixture for 16 hours at 45°C. Thereafter, the filter was washed twice in 2 X SSC, 0.1% SDS, for 5 minutes each cycle at room temperature and twice in 0,1XSSC, 0.1% SDS, for 15 minutes each cycle at 50°C. The filters were exposed to an X-ray film (preferably 3M) between two screens at -80°C for two days.

Colonies containing the recombinant plasmid carrying the cloned 2.78 kb $Bgl$II fragment appeared as black spots. Four out of 50 colonies were identified and the plasmid DNA of these colonies was isolated in minipreparations according to Birnboim and Doly, $Nucl.\ Acids\ Res.$, Vol. 7, p. 1513, 1979, and analyzed by restriction endonuclease digestion by a $Bgl$II and $Xba$I double digest. The analyses confirmed the intended construction explained above (see Figure 3).

### D. Substitution of the Natural Promoter for the PreS1-PreS2-S Protein Coding Regions by the Metallothionein Promoter: Construction of pDM2.

The plasmid pDMI described above was submitted to complete digestion with the restriction enzymes $Bgl$II and $Bam$HI in a total volume of 100 µl containing 20 µg of plasmid DNA and first with 50 units $Bgl$II in reaction buffer containing 6.7 mM Tris, pH 7.8; 6.7 mM MgCl$_2$; 6.7 mM β-mercaptoethanol, at 37°C for 4 hours; then the salt concentration was raised to 150 mM Nacl and the digestion was completed by addition of 40 units $Bam$HI at 37°C overnight. Three fragments were generated, the large fragment being 5.1 kb, the medium fragment being 2.97 kb and the small fragment being 1.4 kb. These fragments were separated by preparative 0.7% agarose gel electrophoresis from which the (5.1 kb and 1.4 kb) fragments were isolated electrophoretically using DE 81 Whatman ion exchange filter.

The DNA was removed from this filter by incubation in high salt for 4 hours at 4°C and purified by phenol/chloroform extraction and two ethanol precipitations. The DNA was resuspended in 20 µl of 10 mM Tris, pH 7.8. one µl was checked for purity and estimation of quantity by agarose gel electrophoresis.

The large (5.1 kb) fragment containing the metallothionein promoter at the $Bgl$II end was ligated to the small (1.4 kb) fragment containing the PreS$_2$-S protein coding region, but not the natural promoter or the PreS$_1$ portion of that gene. The large fragment also contained the natural termination-polyadenylation signal (hb-PAS-t) necessary for expression of the hepatitis B PreS$_2$-S protein coding region (see also Figure 4).

Because the large fragment also contains the bacterial plasmid, it is possible that self-ligation of this fragment will propagate itself without incorporating the small size $Bam$HI fragment. Therefore, 10 µl of the total 20 µl preparation of the large fragment were subjected to treatment with alkaline phosphatase by adding 28 units of this enzyme and incubating at 37°C for 20 minutes. The reaction was stopped by adding 1 µl 50 mM EGTA and incubating at 68°C for 10 minutes. The DNA was purified by two ethanol precipitations in 0.8 M LiCl.

The pelleted DNA was resuspended in 10 µl 10 mM Tris, pH 7.8, of which 5 µl was taken as a control and checked for self-ligation and another 5 µl was mixed with 5 µl of the electroeluted small (1.4 kb) $Bam$HI fragment. Ligation of each sample was performed in a total volume of 20 µl as described above. Transformation on HB101 bacterial cells was carried out as above.

Twelve single colonies were picked and grown in 2 ml cultures and the plasmid DNA was isolated according to Birnboim and Doly, $supra$. The plasmid DNA was checked for the insertion and orientation of the small size $Bam$HI fragment by a double digest with the restriction enzymes $Eco$RI + $Xba$I.

Two of the twelve plasmid DNAs have the small (1.4 kb) $Bam$HI fragment inserted in the same orientation as the netallothionein promoter.

These plasmid DNAs are grown in mass culture and prepared for introduction by co-transfection into eukaryotic cells.

Alternatively, the $Eco$RI/$Bgl$II (1.9 kb) fragment from plasmid pMMT-$neo$ carrying the metallothionein promoter is used for substituting the short (32 b.p.) $Eco$RI-$Bam$HI fragment in front of the PreS$_2$-S protein coding region in pDM1 to give plasmid pDM3, as illustrated in Figure 5.

E. Introduction of the Recombinant DNA Vectors Obtained Under Parts C. and D. above into Mammalian Cells and the Establishment of Cell Lines Producing Particles of this Invention.

The recombinant plasmids, pDM1 and pDM2, were co-transfected into mouse L cells, vero (African green monkey) cells, CHO cells, and 3T3 mouse fibroblasts by standard transfection procedures (Graham and van der Eb, Virology, Vol. 52, p. 456, 1973, supra) (See Materials and Procedures). Cells which take up and maintain the plasmid DNA are resistant to the drug G418 and survive in the selective medium containing this drug. Cells which do not take up the DNA do not survive in the selective medium. These cells are detected as single clones on the surface of a culture plate. The cells were picked with a cloning cylinder and propagated to prepare for mass culture in a conventional manner in a usual nutrient medium, e.g., Dulbecco's modified Eagle medium supplemented with 10% calf serum and 500 µg G418 per ml (see Materials and Procedures). Five of these clones were established as cell lines, and designated ENDO-I, -II, -III, -IV, and -V, and frozen stocks were prepared (see Materials and Procedures). The production rate of these cell lines in static culture was determined by radioimmune assay (RIA; see Materials and Procedures) and compared to the production rate of known cell lines. On the average, the cell lines according to the invention produced 500 ng to 1,000 ng of the particle of this invention per ml of culture medium per day.

F. Acusyst-P Culture of Cell Lines Producing the Particles of the Invention.

Approximately $10^9$ total cells grown in DMEM+10% FBS in six roller bottles (see Materials and Procedures) were used to innoculate six hollow fiber cartridges in the Acusyst-P culture system (see Materials and Procedures). Approximately 600 ml fluid volume was harvested two times weekly from the extracapillary space of each Acusyst-P hollow fiber cartridge and stored at 4°C prior to purification. The concentration of the particles of this invention was assayed by RIA (see Materials and Procedures).

G. Purification Particles of the Invention from Acusyst-P Culture Medium.

Particles containing proteins coded for by the $Pres_1$-$PreS_2$-S protein coding region produced by the novel cell lines were purified by polyethylene glycol (PEG) precipitation, gel filtration and isopycnic ultracentrifugation in CsCl gradients (see below).

1. Fractional Precipitation with Polyethylene Glycol (PEG).
Medium from the extracapillary space was harvested from the ACUSYST-P culture system and pooled into volumes of 3000 ml. To each, volume, 180 g of PEG 8000 (SIGMA) were added, dissolved by stirring at room temperature for 20 minutes and stirred for another 3 hours at 4°C. The precipitate was collected by centrifugation in 500 ml bottles in a GS 3 rotor at 4500 rpm (3000 x g) for 30 minutes at 10°C, lhe supernatant was collected and 180 g of PEG 8000 are again added and dissolved at room temperature as described above. The solution was stirred at 4°C for an additional 3 hours. The precipitate from this solution was collected as described above except that centrifugation was performed at 9000 rpm (15,000 x g) for 60 minutes. The pellet was resuspended in 20 ml of phosphate buffered saline (PBS).
2. Gel Filtration Chromatography.
The material obtained after PEG precipitation, and redissolved in PBS, was submitted to gel filtration chromatography using BioRad A-5m resin at 4°C. Column dimensions were 25 x 1000 mm, and the bed volume was 480 ml. In a typical fractionation run, 1000 µg of PEG-precipitated particles of this invention in a volume of 10 to 15 ml is loaded and eluted with PBS or TNE at a speed of 6 drops/minute (18 ml/hr) in fractions of 3 ml. Figure 6 shows the profile of the particles of this invention eluted from an A-5m column. The solid line indicates the 280 nm absorbance of each fraction, and the dots indicate the quantity of the particles of this invention in each fracton as calculated by RIA.
3. Isopycnic Centrifugation in CsCl.
About 30 fractions covering the first peak resulting from gel filtration column chromatography (see Figure 6) and containing partially purified particles of this invention were pooled (approximately 100 ml). This solution was adjusted to a density of 1.30 g/cc with CsCl and subsequently transferred to nitrocellulose tubes fitting into a SW 27 or SW 28 rotor (Beckman). Gradients were set by underlaying 4 ml of a CsCl solution of 1.35 g/cc and by overlaying 4 ml of 1.25 g/cc and 4 ml of 1.20 g/cc density. Gradients were run at 28,000 rpm for 50 hours at 10°C, fractionated, and the purified antigen in the 1.20 g/cc density layer at the top of the gradient was collected. The particles of this invention were well separated from the small amount of contaminating protein banding in the middle of the gradient (see Figure 7). The solution was desalted by dialysis against three changes of saline over a 24-hour period.

As a quality control measure, a portion of this gradient-purified material was submitted to linear CsCl-gradient centrifugation. A single peak of the particles of this invention appeared as expected at 1.2 g/cc.

H. Characterization of the Particles of this Invention Produced by Cell Lines.

The purity and physical identity of the polypeptides of the particles of this invention in preparations are established by conventional laboratory techniques, such as radioimmune assay, immunoprecipitation and SDS-PAGE (see Materials and Procedures).

1. Purity Determinations.

a. Determination of Levels of Contaminating Plasma Proteins.

The levels of various serum proteins in preparations of purified particles of this invention are assessed by the standard RIA technique using specific antibodies against bovine serum albumin, IgA, IgG, IgM, etc. The results shown in Table 2 show no detectable immunoglobulin contamination and only minor albumin contamination.

Table 2

| Determination of Contaminating Plasma Proteins by RIA | |
| --- | --- |
| Protein | % of Total Proteins |
| Albumin | 0.05 |
| IgG | 0.2 |
| IgA | 0.2 |
| IgM | 0.2 |

b. Determination of Levels of Contaminating Host Cell or PreS1-PreS2-S Nucleic Acid Sequences in Purified Preparations of the Particles of this Invention by the Dot Blot Procedure.

A dot-blot hybridization is performed on purified particles of this invention in order to check for contaminating $PreS_1$-$PreS_2$-S DNA sequences or host chromosomal DNA sequences. For each spot, a 100 ng quantity of purified particles of this invention in 40 $\mu$l 0.25 N NaOH is heated at 68°C for 10 minutes. As a positive control, 20 pg of recombinant plasmid pDM1 DNA of this invention in 40 $\mu$l 0.25 N NaOH is treated the same way. Immediately after denaturation in NaOH, the solution is spotted onto a nitrocellulose filter. The following treatment of the filter and the hybridizing procedures are analogous to those described for the Southern Blot procedure, except that the $^{32}$p-labeled probe is a 50:50 mixture of chromosomal DNA and plasmid DNA of this invention containing the $Pres_1$-$Pres_2$-s protein coding region. As expected, the hybridizing probe gives a strong signal with the recombinant plasmid DNA of this invention, but only a weak background signal with the purified particle preparation. This hybridization pattern indicates no $PreS_1$-$PreS_2$ -S DNA or chromosomal DNA in the sample.

2. Immunoprecipitation and SDS-Polyacrylamide Gel Analysis.

To determine the pattern of $PreS_1$-$PreS_2$-S polypeptides produced by transfectants of this invention, proteins produced by such transfectants are biosynthetically labeled, immunoprecipitated and analyzed on SDS-PAGE gels (see Materials and Procedures). The electrophoretic pattern of proteins, isolated from culture medium by immunoprecipitation with antibodies to naturally derived HBV, was visualized by the silver staining procedure. The two major proteins correspond in size to nonglycosylated and glycosylated polypeptides encoded by the S protein coding region (24 kd and 27 kd, respectively). Two minor proteins of 33 kd and 36 kd correspond in size to the two polypeptides encoded by the $PreS_2$-S protein coding region. This method is not sensitive enough to detect the small quantities of polypeptides containing the $PreS_1$-$PreS_2$-S polypeptides, but the presence of the proteins encoded by the entire $PreS_1$-$PreS_2$-S protein coding region was detected by the western immunoblot procedure (see, infra). The weak bands at the top of the gel result from aggregated protein and are also visible with naturally-derived HBV particles. Thus, cells transfected with the $PreS_1$-$PreS_2$-S protein coding region secrete proteins which not only react with antibodies against the natural product, but also are identical in size. The presence of proteins which are identical in size to the naturally-occurring glycosylated $PreS_1$-$PreS_2$-S, $PreS_2$-S and S polypeptides from the hepatitis B virus suggests that glycosylation pathways function normally in these cells.

3. Immunoblot (Western).

To establish that each of the polypeptides species appearing in the silver stained SDS-PAGE gel system reacts with specific anti-HBV antibody, the Western immunoblot procedure was employed. Proteins separated in a SDS-PAGE were transferred to a nitrocellulose filter (Schleicher & Schull) by electroblotting (BioRad) at 4°C, 100 V, 3 hours. After transfer, the filter is saturated with proteins in order to avoid nonspecific binding of peroxidase-labeled antibody. To do this, the filter is incubated for 1 hour at room temperature in 20% newborn calf serum in PBS, and the polypeptides of the particle of this invention were made visible by HBV-specific antibodies conjugated with peroxidase. The filter is placed on parafilm and covered with a solution of the conjugated antibodies for 3 hours at room temperature in a wet chamber. The filter is washed six times with 0.5% Tween in 10mM Tris and 5 mM CaCl and subsequently covered with the chromogen, POD (o-phenylenediaminedihydrochloride) in hydrogen peroxide (0,3g/l) in citrate-phosphate-buffer. Stopping solution is 0.5 N sulfuric acid. The protein bands corresponding to all six viral polypeptides appear, i.e., all the proteins coded for by the $Pres_1$-$PreS_2$-S protein coding region.

4. Amino Acid Composition of Purified Proteins Coded for by the PreS1-PreS2-S Protein Coding Region Comprising the Particles of this Invention.

Following acid hydrolysis, the amino acid composition of the purified proteins coded for by the $Pres_1$-$PreS_2$-S protein coding region is determined and compared with the polypeptide composition calculated from the nucleotide sequence of the S protein coding region, the $PreS_2$-S protein coding region or the $PreS_1$-$PreS_2$-S protein coding region. The results presented in Table 3 correlate well with values predicted from the DNA sequences as well as with published results (Shiraishi et al, J. Gen. Virol., Vol. 48, p. 31, 1980). In particular, the relatively high percentage of serine, proline and leucine is characteristic of these polypeptides.

Table 3

| Amino Acid Composition of Purified Particles of this Invention | | | | |
|---|---|---|---|---|
| Residue Percent | | | | |
| AMINO ACIDS* | DETERMINED | EXPECTED | | |
| | | S | $PreS_2$-S | $PreS_1$-$PreS_2$-S |
| ASN + ASP | 5.5 | 4.2 | 5.2 | 7.3 |
| THR | 7.5 | 8.0 | 7.9 | 7.8 |
| SER | 12.5 | 11.3 | 12.0 | 10.8 |
| GLN + GLU | 7.5 | 4.2 | 4.5 | 5.1 |
| PRO | 12.5 | 10.8 | 10.5 | 12.6 |
| GLY | 8.6 | 6.6 | 7.1 | 8.3 |
| ALA | 5.0 | 2.8 | 4.5 | 5.4 |
| VAL | 4.2 | 5.2 | 5.2 | 4.8 |
| CYS | 3.2 | 6.6 | 5.2 | 3.8 |
| MET | 1.9 | 2.3 | 2.2 | 1.9 |
| ILE | 4.9 | 7.5 | 7.5 | 6.7 |
| LEU | 12.8 | 15.5 | 13.9 | 12.1 |
| TYR | 2.0 | 2.8 | 2.6 | 1.9 |
| PHE | 5.6 | 7.5 | 6.0 | 5.4 |
| LYS | 2.1 | 1.9 | 1.5 | 1.3 |
| HIS | 0.9 | 0.5 | 1.1 | 1.9 |
| ARG | 2.1 | 2.4 | 3.0 | 3.0 |
| | 98.8% | 100.1% | 99.9% | 100.1% |

*Tryptophan is lost in the acid hydrolysis procedure.

I. Electron Microscopy of Purified Hepatitis B Particles of this Invention.

In the electron microscope, the purified Particles of this invention were visualized as splerical 22 nm particles which are similar to those typically formed by the assembly of hepatitis 3 S protein polypeptides.

J. Preparation of the Adjuvant of Purified Particles of this Invention and Stability Testing.

In order to prepare an adjuvant of the vaccine of this invention, 1/10,000 volume Thimerosol, and 1/10 volume of filter-sterilized 0.2 M Al K(SO$_4$)$_2$:12 H$_2$O are added to the desired concentration of antigen in sterile saline. The pH is adjusted to 5.0 with sterile 1N NaOH and the suspension is stirred at room temperature for 3 hours. The alum-precipitated antigen is recovered by centrifugation for 10 minutes at 2000 rpm, resuspended in sterile normal saline containing 1:10,000 Thimerosol, and aliquoted under sterile conditions.

To determine the stability of the particles of this invention absorbed to alum, the antigen was recovered by resolubilizing the alum in 3% sodium citrate followed by successive dialyses against 3% sodium citrate and PBS. The quantity of particles of this invention was then determined by parallel-line radioimmune assay against dilutions of a purified standard in PBS-50% newborn calf serum. Stability testing results (Table 4) show the purified particles (either bulk or alum-absorbed) to be stable at 2°-8°C for at least 7 months.

Table 4

| Stability Testing | | | | |
|---|---|---|---|---|
| **SDS-PAGE:** | | | | |
| Storage Month | Banding Pattern Results | | | |
| | Bulk 1 | Sample 2 | Alum Absorbed 1 | Sample 2 |
| Start | normal | normal | normal | normal |
| 1 | normal | normal | normal | normal |
| 2 | normal | normal | normal | normal |
| 3 | normal | normal | normal | normal |
| 4 | normal | normal | normal | normal |
| 5 | normal | normal | normal | normal |
| 6 | normal | normal | normal | normal |
| 7 | normal | normal | normal | normal |
| **Quantitative Determination by RIA :** | | | | |
| Storage Month | Percent (%) Activity | | | |
| | Bulk 1 | Sample 2 | Alum Absorbed 1 | Sample 2 |
| Start | 101 | | 101 | |
| 1 | 101 | 101 | 100 | 100 |
| 2 | 99 | 99 | 99 | 100 |
| 3 | 100 | 100 | 99 | 101 |
| 4 | 99 | 100 | 101 | 99 |
| 5 | 102 | 99 | 99 | 98 |
| 6 | 100 | 101 | 100 | 100 |
| 7 | 99 | 98 | 98 | 101 |

K. Seroconversion and ED$_{50}$ of the particles of this Invention.

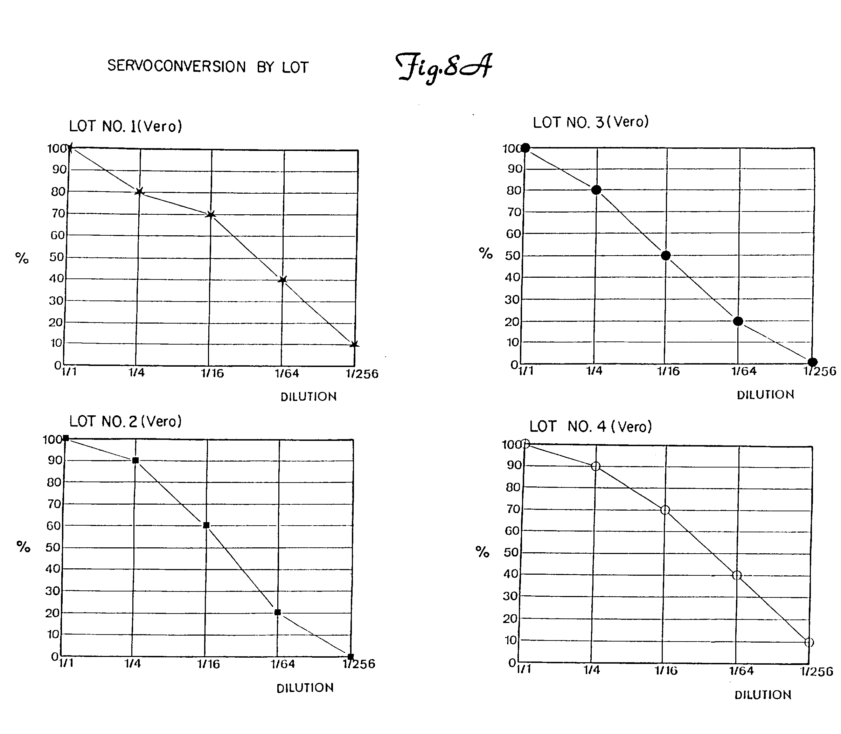

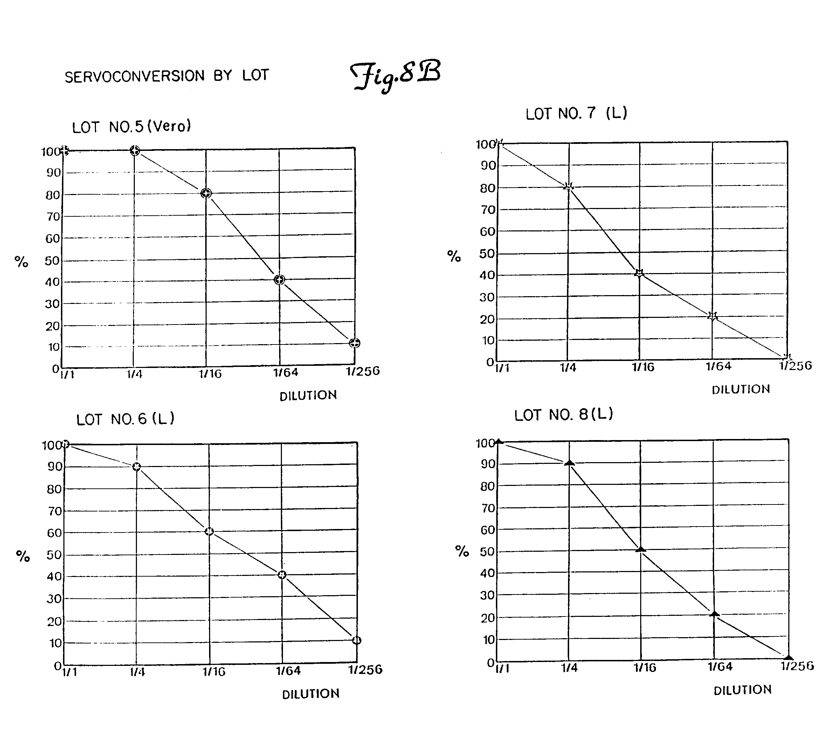

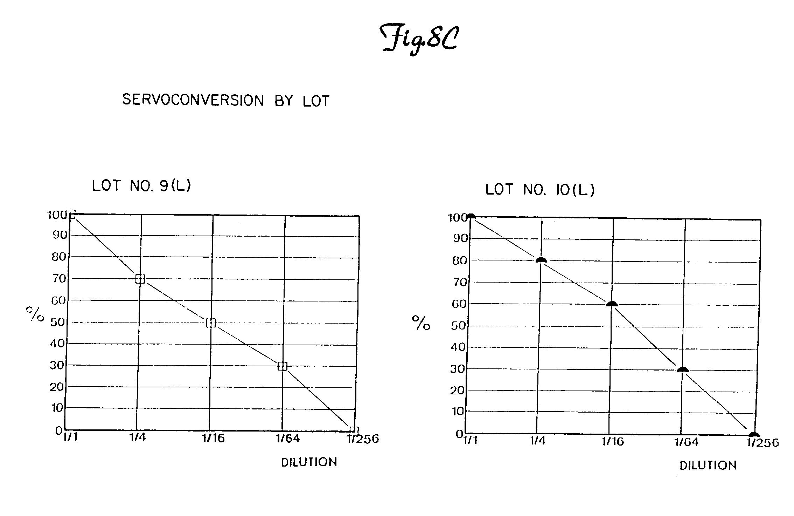

The seroconversion tests are carried out on five groups of ten adult white mice (Figures 8A, 8B and 8C). Gm female ICR strain swiss mice are injected subcutaneously with 1 ml of alum adjuvant vaccine containing particles of this invention at the following dilutions: 1/1 (5.0 μg), 1/4 (1.3 μg), 1/16 (0.31 μg), 1/64 (0.16 μg), and 1/256 (0.078 μg), I-lice are bled after 28 days, and anti-body titer is quantitated by the AUSAB test in comparison with the HBIG standard. The serum samples from each animal were diluted four-fold serially and tested in triplicate.

The percentage of mice exhibiting a positive serotype declined with increasing dilution of particles of this invention. The dose resulting in 50% of the animals exhibiting a seropositive response (ED$_{50}$) is about 0.05-0.25 μg of vaccine containing particles of this invention. In general, the seroconversion results obtained with the vaccine of this invention are indistinguisable from those obtained with the naturally derived vaccine.

Example 2

Expression of Particles Comprising PreS1-PreS2-S Protein Coding Region Polypeptides

A. Preparation of Recombinant Plasmid pENDO-1.

Gene sequences from recombinant plasmids, pBglII2.8, pBR322.βG2 (a plasmid made by subcloning into the EcoRI site of pBR322 the 7 kb EcoRI βG2 fragment from plasmid pMB9.βG2 described by Tilghman, S.M. et al, Proceeding Natl. Acad. Sci., Vol. 75, p. 725, 1978), pDM2, POLINK 23456 (see infra), and the pMMT-neo plasmid, are used in the steps to prepare vector constructions to produce recombinant expression vectors having no non-HPV viral genomic portions.

Referring to Figures 9A and 9B, the pBglII2.8 plasmid contains a gene segment (1.34 kb BstEII-HpaI fragment) containing the PreS$_1$-PreS$_2$-S protein coding region and carries the natural strong promoter for transcription of the PreS$_2$-S protein coding region, but lacks the natural (weak) promoter for transcription of the PreS$_1$-PreS$_2$-S protein coding region, as well as the transcription termination functions and the polyadenylation signal. Plasmid pBglII2.8 was subjected to BstEII and HpaI digestion to produce two DNA fragments. Following separation by 3.5% polyacrylamide gel electrophoresis of the fragments, one of them (1.34 kb), which contains the PreS$_1$-PreS$_2$-S protein coding region, was purified and retained. The HpaI end is blunt. The BstEII end was made blunt by filling in (made double-stranded) using DNA polymerase I (Klenow fragment) and the four dNTP's using conventional techniques to give rise to a DNA fragment with two blunt ends.

A polylinker plasmid, POLINK 23456, having a number of restriction sites contained within the polylinker region (below) was employed.

```
         XbaI      BclI    HpaI    ClaI   HindIII  KpnI    BamHI          SphI    SacI         SalI
GAATTCTAGATCTGATCAGTTAACATCCATAAGCTTGGTACCGGATCCCGGGCATGCGAGCTCGAGTCGAC
EcoRI    BglII                                                  SmaI          XhoI
                                                                XmaI          AvaI
                                                                AvaI
```

The polylinker adapter is carried within the EcoRI-SalI back-bone fragment (3.1 kb) of pBR328.

POLINK 23456 is digested with HpaI to linearize the plasmid. Such HpaI linearized plasmid bears two blunt ends. The linear plasmid is then blunt-end ligated with the 1.34 kb DNA fragment containing the PreS$_1$-PreS$_2$-S protein coding region obtained from the pBglII2.8 plasmid (above). Since the 1.34 kb fragment can be incorporated into the polylinker plasmid in either orientation, the correct orientation (clockwise with respect to the natural direction of transcription) is ascertained by digestion with EcoRI, followed by size analysis on 1% agarose gels. EcoRI digestion of the plasmid carrying the PreS$_1$-PreS$_2$-S protein coding region in the correct orientation gives rise to two fragments of 0.4 kb and 4.1 kb; the incorrect orientation gives rise to two fragments of 1.0 kb and 3.5 kb. The new plasmid containing the PreS$_1$-PreS$_2$-S protein coding region is then digested with HpaI and "partially" digested with BamHI. the short (24 b.p.) sequence between the HpaI and BamHI restriction sites is separated from the HpaI-BamHI "partial" back-bone (5.0 kb) and discarded along with the fragments in the reaction mixture resulting from complete digestion.

The pBR322.βG2 plasmid is digested with BalI (blunt end) and BglII ("sticky" end) to obtain a gene sequence (1.6 kb) containing the mg-PAS-t polyadenylation-termination sequence from the mouse globin gene. The mg-PAS-t fragment is then ligated into the opened HpaI-BamHI linearized plasmid from above, BalI-to-HpaI and BglII-to-BamHI (BglII and BamHI "sticky" ends are identicle and therefore are compatible, and suitable for ligation to each other), to form a new intermediate recombinant plasmid (6.0 kb) containing both the Pres$_1$-PreS$_2$-S protein coding region and the mg-PAS-t sequence in the correct orientations and order. The PreS$_1$-PreS$_2$-S protein coding region/mg-PAS-t-containing plasmid is then digested with BglII and SalI, splitting the plasmid into two DNA fragments (2.95 kb and 3.05 kb), as ascertained by 1% agarose gel electrophoresis. Following digestion of the 3.05 kb fragment with PvuI to produce two smaller fragments (1.08 kb and 1.98 kb), the remaining 2.95 kb BglII-SalI DNA fragment (containing no PvuI site) containing the fused PreS$_1$-PreS$_2$-S protein coding region/mg-PAS-t segments is purified and retained.

The pMMT-neo plasmid, the plasmid formed from ligation of the 6.65 fragment obtained in Example 1, Section A (see Figure 9) is then digested with BglII and sall resulting in two DNA fragments (4.23 kb and 2.42 kb). The 2.42 kb fragment containing the neomycine selection marker and the SV40 PAS-t is discarded. The remaining 4.23 kb fragment is purified using 0.8% agarose gel electrophoresis and is then ligated with the BglII-SalI 2.95 kb PreS$_1$-PreS$_2$-S protein coding region/mg-PAS-t DNA fragment obtained above to form a 7,15 kb recombinant plasmid containing in order: the MMT-promoter, the PreS$_1$-PreS$_2$-S protein coding region and the mg-PAS-t polyadenylation and termination sequence, the plasmid being referred to as pENDO-1. The pENDO-1 plasmid contains no non-HBV viral genomic portions (see Figure 9).

B. Preparation of Recombinant Plasmid pENDO-2.

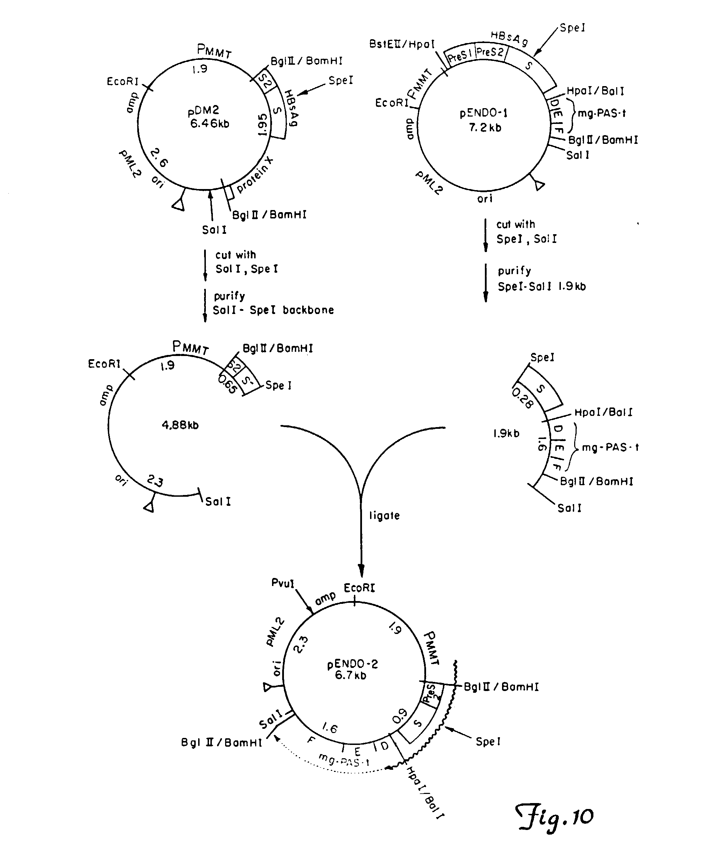

Referring to Figure 10, pDM2, which includes the MMT-promoter, the $PreS_2$-S protein coding region and the protein X coding region, is digested with restriction enzymes SpeI and SalI. The SpeI restriction site is in the S protein region of the $PreS_2$-s protein coding region and is unique in pDM2, as is the SalI site downstream of the gene. Using these two enzymes, the plasmid is split into two DNA fragments (1.58 kb and 4.88 kb) and the 1.58 kb fragment, containing the carboxy end of the $PreS_2$-S protein coding region and the protein X coding region, is discarded, while the 4.88 kb fragment is purified and retained.

The pENDO-1 plasmid is also subjected to SpeI and SalI digestion, splitting the plasmid into two DNA fragments, 1.9 kb and 5.25 kb. The 1.9 kb fragment containing the carboxy end of the S protein region of the $PreS_2$-S protein coding region, plus the mg-PAS-t region is purified and retained.

The two retained DNA fragments (1.9 kb, and 4.88 kb) are then ligated to produce a new recombinant plasmid (6.8 kb) designated pENDO-2 which contains the MMT-promoter, the $PreS_2$-S protein coding region and the mg-PAS-t sequence. Plasmid pENDO-2 contains no non-HBV viral genomic portions (see Figure 10).

C. Preparation of Recombinant Plasmid pENDO-0.

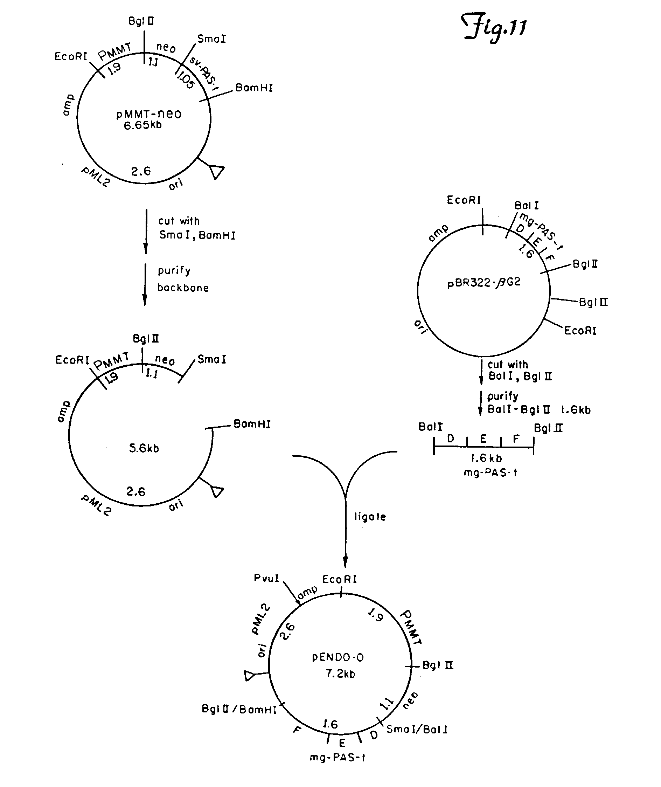

A third new plasmid, referred to as pENDO-0 (see Figure 11), containing a neomycine selection marker, is formed by digesting pMMT-neo, the plasmid formed from ligation of the 6.65 kb fragment obtained in Example 1, Section A (see Figure 11), with restriction enzymes SmaI (blunt end) and BamHI. The plasmid is split into two DNA fragments (5.5 kb and 1.15 kb). The 5.5 kb fragment containing the promoter and the neomycine selection marker from the pMMT-neo plasmid backbone is retained. The latter fragment is then ligated with the DNA (1.6 kb) fragment containing the BalI-BglI-PAS-t sequence as described with reference to the formation of pENDO-1. The resulting 7.2 kb recombinant plasmid, pENDO-0, now contains the MMT-promoter, the neomycine selection marker and the mg-PAS-t sequence and no viral DNA sequences (see Figure 11).

D. Introduction of Plasmid Vectors pENDO-0, pENDO-1 and pENDO-2 into Mammalian Cells by Co-Transfection.

The recombinant plasmid vectors pENDO-0, pENDO-1 and pENDO-2 are co-transfected into mammalian cells, such as mouse L-cells, Vero (African green monkey) cells, CHO cells and 3T3 mouse fibroblasts, using standard co-transfection procedures. G418-resistant transfectants are isolated and screened for production of the particles of this invention using the RIA method.

E. Preparation of Mixed Concatamers of pENDO-0, pENDO-1, pENDO-2 Plasmid DNAs for Transfection of Cell Lines.

Each of the plasmids, pENDO-1, pENDO-2 and pENDO-0, contain a unique restriction site, PvuI, which exists within the Amp gene, counterclockwise to the EcoRI restriction site (see Figures 9, 10 & 11). Each of the plasmids is digested separately with PvuI to linearize. Then such plasmids are polymerized to form mixed concatamers with various ratios of the different plasmids. For example, polymerization of the pENDO-1 and pENDO-0 linearized plasmids using ligase, such as T4 DNA ligase, can form a mixed concatamer with a ratio of 10:1 pENDO-1:pENDO-0, if the input ratio of the component linearized plasmids is 10:1 pENDO-1:pENDO-0. The resulting pENDO-1/pENDO-0 mixed concatamer is transfected into a eukaryotic cell, such as a mammalian cell, and the cell subjected to G418 selection. It is believed, on the average, that every G418-resistant (transfected) cell should contain about ten copies of the $PreS_1$-$PreS_2$-S protein coding region per one copy of the neo gene.

Similarly, the pENDO-0 fragment is polymerized with the pENDO-2 fragment by ligase, such as T4 DNA ligase, at a ratio of 10:1 pENDO-2:pENDO-0, for example, to form a pENDO-2/pENDO-0 mixed concatamer. The concatamer is then transfected into a eukaryotic cell, such as a mammalian cell. The cells will then be subjected to G418 selection and on the average each G418-resistant (transfected) cell will contain about ten copies of pENDO-1 to pENDO-0, in this example.

Similarly, pENDO-1, pENDO-2 and pENDO-0 are polymerized by ligase, such as T4 DNA ligase, to form a mixed concatamer at a ratio of 10:10:1, pENDO-1:pENDO-2:pENDO-0, for example. The resulting mixed concatamer is transfected into a eukaryotic cell, such as mammalian cell, and the cell subjected to G418 selection. On the average, the resistant (transfected) cells should contain ten copies of pENDO-1 to ten copies pENDO-2 to one copy pENDO-0.

Each of the resulting resistant cells should result in higher yields of the particles containing the proteins encoded by the $PreS_1$-$PreS_2$-S protein coding region.

Applications of Non-Replicating Vectors Comprising the Mouse Metellothionein Promoter to the Expression of Of Other Proteins in Transfected Eukaryotic Host Cells

The invention also comprises recombinant DNA transfer vectors comprising the MMT promotor, any functional DNA sequence and additional DNA other than viral DNA.

By the term functional DNA sequence is meant any discrete region of DNA derived directly or indirectly from any source which functions in a host eukaryotic cell transfected with the vector of this invention as a complete gene expression unit, a structural gene, a promoter or regulatory region.

By complete gene expression unit is means a structural gene and the promoter and regulatory regions required for its transcription and translation.

By promoter is means any region upstream of a structural gene which permits binding of RNA polymeraze and transcription to occur.

By regulatory region is meant any region which regulates the rate of transcription of the structural gene.

By structural gene is meant a coding sequence which serves to be the template for the synthesis of messenger RNA.

Preferred structural genes include those coding for polypeptides of pharmaceutical importance such as a viral antigen, insulin, interferon, and lynphokines, rat or human growth hormones, tissue Plasminogen Activator, alpha-l-antiprypsin and the like. Most preferred is rat or human growth hormone.

This invention relates to a recombinant DNA vector comprising the function DNA sequence and a MMT-promoter. The MMT-promoter may be incorporated into the DNA vector either in addition to the natural promoter for the DNA sequence or in place of the natural promoter. Preferably, the MMT-promoter is located in the DNA vector immediately upstream of the DNA sequence protein coding region. Preferably, such vector also comprises a transcription termination sequence and a selection marker. Preferably, such selection marker is a drug-resistance marker, such as the neomycine gene. Preferably, such transcription termination sequence is an SV40 termination site (sv-PAS-t) or more preferably the DEF region (mg-PAS-t) of the mouse globin gene. Such vector may be prepared by conventional recombinant DNA and other molecular biological techniques. In the most preferable case, using the mg-PAS-t region, the vector does not contain any viral DNA segments and is not oncogenic, i.e., it will not transform (make cancerous) any host cell into which it is introduced. Such vector, upon transfection into a host, if it does not contain an autonomous replication sequence (replicon) capable of functioning in a host transfected therewith, integrates into the host chromosome and replicates passively with the host genome.

Preferably, the recombinant DNA vector of this invention should comprise the following characteristics:

1) The DNA sequence of interest.
2) A MMT-promoter located immediately upstream of the DNA sequence of interest.
3) The vector should be able to replicate in bacteria, or other procaryotic host into which it is transformed, for growth, amplification and preparation of large quantities of the recombinant vector. Thus, such vector should include a bacterial or other procaryotic replicon, i.e., a DNA segment bearing all the functions required for autonomous replication and maintenance of the vector extrachromosomally in a procaryotic host cell, such as a bacterial host cell, transformed therewith. Such replicons are well known in the art.
4) The vector replicon should be small (i.e., probably smaller than 6-8 kilobase pairs) to enable easy genetic and molecular biological manipulation thereof.
5) The vector should carry a selection marker, preferably a drug-resistance marker such as ampicillin, for use in bacterial host cells transformed therewith.
6) The vector should carry a second selection marker, preferably a drug-resistance marker such as neomycine, for use as such in eukaryotic host cells transfected therewith.
7) The vector should contain convenient endonuclease restriction sites for cloning.
8) The vector should contain a tran- scription termination and polyadenylation sequence.
9) The gene expression unit does not contain any viral segments and is not oncongenic.

Most preferably, the vector of this invention does not comprise an autonomous replicating sequence (replicon) capable of functioning in a eukaryotic host cell transfected therewith. A primary reason for using a non-replicating vector system in eukaryotic host cells is that all vector systems capable of autonomous and extrachromosomal replication in a mammalian host eukaryotic cell transfected therewith comprise replicons which are derived from oncogenic viruses. It is desirable to employ vectors comprising DNA not derived from oncogenic viruses for expressing DNA sequences encoding polypeptides of pharmaceutical importance.

This invention also relates to a transfected host eukaryotic cell transformed with a recombinant DNA vector of containing the functional DNA sequence this invention. Preferably, such host is a mammalian cell, most preferably a Chinese hamster ovary (CHO) cell line, a vero cell line, an L-cell line, or a mouse or rat fibroblastic cell line. Such host may be prepared by transfecting a eukaryotic cell with a recombinant DNA vector of this invention by conventional techniques.

This invention also relates to a method for preparing the proteins coded for by the DNA sequence of interest which comprises: a) cultivating the transfected host of this invention under culture medium conditions which enable such host to express such proteins, and b) isolating such proteins. Preferably, such transfected host is able to secrete such proteins into the medium. Preferably, heavy metal ions or steroid hormones, such as dexamethasone, are added to such culture medium to induce the MMT-promoter and thereby enhance expression of such coding region. Heavy metal ions such as cadmium or zinc are most preferred. The optimal concentration of heavy metal ions or steroid hormone contained in the medium can be determined by conventional techniques.

This invention also relates to the proteins prepared by such method. If the transfected host cell of this invention secretes such proteins directly into the culture medium, such proteins can then be isolated from the culture medium of the transfected host of this invention by conventional protein isolation techniques. If the transfected host cell of this invention does not secrete such proteins , they are obtained from a culture lysate of such host by conventional culture lysate techniques.

Example 3

Expression of Human Growth Hormone

Preparation of a Recombinant DNA Vector Encoding Human Growth Hormone, Said Vector Comprising the Metallothionein Promoter

A. Isolation of a DNA Fraament Containing the Human Growth Hormone Gene Without the Natural Promoter for Transcription.

The recombinant plasmid pBR322 containing a 2 kb EcoRI DNA fragment insert encoding the human growth hormone gene (in a counterclockwise orientation) is digested with the restriction endonuclease BamHI in a total volume of 500 ul containing 100 ug of plasmid DNA, 240 units BamHI, in BamHI (high salt) buffer. Two fragments are generated, one being about 5.6 kb and another fragment 0.8 kb. The fragments are separated by electrophoresis in a 0.8% preparative agarose gel. The larger fragment, containing the structural gene for human growth hormone without the natural promoter for gene, is electro-eluted from the gel and purified by two cycles of phenol/chloroform extraction, two cycles of chloroform extraction and two cycles of ethanol precipitation. The pelleted DNA is resuspended in 20 ul 10 mM Tris, pH 8.0.

B. Isolation of a DNA Fragment Containing the Metallothionein Promoter and the pML2 Vector Backbone.

Plasmid pMMT-neo (see Figure 5) is digested with restriction endonucleases BglII and BamHI to produce two fragments, one being 4.5 kb and containing the MMT-promoter and the pML2 vector backbone, and the other being 2.15 kb and containing the neo gene coupled to the sv-PAS-t segment. In order to prevent self-ligation, the linear DNA is treated with alkaline phosphatase in a total volume of 100 ul containing 25 ug of the DNA fragment, and, 28 U of alkaline phosphatase. The mixture is incubated 20 minutes at 37°C, and the reaction is stopped by adding 10 ul 50 mM EDTA and heating for 10 minutes at 68°C. The fragments are separated by electrophoresis in a 0.8% agarose gel. The 4.5 kb fragment is electro-eluted from the gel as described and is purified by two cycles of phenol/chloroform extraction, two cycles of chloroform extraction and two cycles of ethanol precipitation. The pelleted DNA is resuspended in 20 ul 10 mM Tris, pH 8.0.

C. Ligation of the DNA Fragments Isolated in A. and B. Above.

A 1.5 ug quantity of the 5.6 kb fragment from A. above is taken and mixed with 0.3 ug of the fragment from B. above in a total volume of 10 ul containing 0.9 units of T4 DNA-ligase and 1X ligation buffer, plus 100 mM ATP. The mixture is incubated for 1 hour at 15°C and overnight at 4°C. The ligation mixture is then introduced into the bacterial strain, HB101, using the procedure described in Materials and Procedures. Cells from the transformation mixture are spread onto LB-ampicillin agar plates and isolated colonies are screened for those containing plasmids of 10.1 kb in size that can be linearized with restriction endonuclease BamHI. The orientation is determined by EcoRI digestion. Plasmids containing the insert in the correct orientation yield two EcoRI fragments, one of 3.5 kb and the other of 6.6 kb. Such plasmids, designated pMMT-hGH, are subjected to large scale growth and purification as described.

D. Introduction of the Recombinant DNA Vector Obtained in C. Above into Mammalian Cells and Establishment of Cell Lines Producing Human Growth Hormone.

The vector, pMMT-hGH, is then transfected into eukaryotic host cells (mammalian) by standard co-transfection techniques to yield transfectants that synthesize the human growth hormone polypeptide. Such transfectants are identified by the RIA technique using antibodies to human growth hormone. Transfectants showing secretion of human growth hormone are established as cell lines using conventional techniques.

Example 4

Expression of Rat Growth Hormone

Preparation of a Recombinant DNA Vector Comprising the Rat Growth Hormone, Said Vector Containing the Metallothionein Promoter.

A. Conversion of the BglII Restriction Endonuclease Site in pMMT-neo to XhoI.

A 200 ug quantity of pMMT-neo is digested with 500 U of restriction endonuclease BglII in medium salt buffer in a total reaction volume of 1,000 ul. The ends of the 6.65 kb linear plasmid DNA are first made blunt by filling in the BglII ends using DNA polymerase I (Klenow fragment) and the four dNTP's, as described previously, followed by attachment of the following synthetic DNA linker fragment containing the XhoI restriction endonuclease recognition and cleavage site (from PL Biochemicals, Milwaukee, Wisconsin 53205):

$$5'-CCTCGAGG-3'$$
$$3'-GGAGCTCC-5'$$

The filling in reaction is performed in a total volume of 30 ul containing 2.5 ug of the 6.65 kb fragment, 200 mM of the four dNTP's, 3 ul NT-buffer, 1 ul Klenow fragment. The mixture is incubated at room temperature for 30 minutes. The reaction is stopped by the addition of 2 ul 0.25 M EDTA and the DNA is washed once with an equal volume of phenol/chloroform and then with an equal volume of chloroform alone. The DNA in the aqueous phase is further purified by two ethanol precipitations. The precipitated DNA is resuspended in 10 ul 10 mM Tris, pH 8.0, at a concentration of 50 ng/ul.

The linker fragment is dissolved in 50 ul of 10 mM Tris, pH 8.1, at a concentration of 1 pmole/ul. The blunt-ended 6.65 kb fragment is ligated to the linker fragment in a total volume of 20 ul containing 2 pmoles of the linker fragment, 0.5 ug of the 6.65 kb fragment, 9 units of T4 DNA-ligase (Boehringer Mannheim), 30 ul 2X blunt-end ligation buffer, plus 100 mM ATP. The ligation is performed at 15°C for one hour and at 4°C overnight.

This ligation mixture is used to transform the bacterial strain, HB101, which is made competent as described in Materials and Procedures. Cells from the transformation mixture were spread on LB-ampicillin agar plates. Bacterial colonies able to grow on ampicillin plates are picked and grown up in 2 ml cultures and subjected to plasmid preparations as described above.

From 12 bacterial clones, four (OK 3, OK 4, OK 11, OK 12) were identified as having a plasmid showing the expected size after digestion with XhoI alone or together with BamHI and EcoRI. The plasmid DNA from OK 3 and OK 11 were propagated in HB101 for large scale plasmid preparations.

B. Isolation of a DNA Fragment Containing the Metallothionein Promoter and the pML2 Vector Backbone.

A 100 ug quantitiy of each of OK 3 and OK 11 was digested with the restriction endonucleases BamHI and XhoI which yield two fragments, one (5.8 kb) with the MMT-promoter located close to the XhoI end and the pML2-part at the BamHI end, and the other (0.85 kb) containing the sv-PAS-t sequence. The fragments were separated by electrophoresis in 0.8% agarose. The 5.8 kb fragment was purified by electro-elution, followed by two cycles of phenol/chloroform extraction, two cycles of chloroform extraction and two cycles of ethanol precipitation. The pelleted DNA was resuspended in 50 ul 10 mM Tris, pH 8.0.

C. Isolation of a DNA Fragment Containing the Rat Growth Hormone Gene.

Plasmid pBR322.rGH, containing a genomic BamHI insert encoding the rat growth hormone gene (in a counterclockwise orientation), was digested with BamHI and XhoI. The genomic BamHI-XhoI fragment containing the structural gene is isolated from a 0.8% preparative agarose gel as above.

D. Ligation of the Fragments Isolated as in B. and C. Above.

The fragments are ligated to each other such that the MMT-promoter will be linked, via the Xhol "sticky" end directly to the structural gene via its Xhol sticky end, in the correct orientation.

The ligation mixture is used to transform the bacterial strain, HB101, as described above. Transformants forming colonies on LB-ampicillin agar plates are picked and grown up in 2 ml cultures for small scale plasmid preparation. Plasmids yielding the two expected fragments upon digestion with BamHI and Xhol, designated pMMT-rGH, are then grown up in large scale for transfection into mammalian cells.

E. Introduction of the Recombinant DNA Vector Obtained in D. Above into Mammalian Cells and Establishment of Cell Lines Producing Rat Growth Hormone.

The vector, pMMT-rGH, is then transfected into eukaryotic host cells (mammalian) by standard co-transfection techniques to yield transfectants that synthesize the rat growth hormone polypeptide. Such transfectants are identified by the RIA technique using antibodies to rat growth hormone. Transfectants showing secretion of rat growth hormone are established as cell lines using conventional techniques.

Although the present invention has been described with reference to preferred embodiments, workers skilled in the art will recognize that changes may be made in form and detail without departing from the spirit and scope of the invention.

**Claims**

**Claims for the following Contracting States : BE, CH, DE, FR, GB, IT, LI, LU, NL, SE**

1. A recombinant DNA vector being free of non-HBV viral or oncogenic nucleic acid sequences comprising:

   (a) A DNA sequence comprising the hepatitis B $PreS_1$-$PreS_2$-S protein coding region; and
   (b) A promoter which is a metallothionin gene promoter and capable of controlling the expression of said coding region (a) in a mammalian host cell so as to allow the production of the $PreS_1$-$PreS_2$-S protein.

2. The vector of claim 1, wherein the promoter is incorporated into the vector comprising the DNA sequence in addition to or in place of a hepatitis B natural promoter, preferably immediately upstream of the $PreS_1$-$PreS_2$-S protein coding regions.

3. The vector according to claim 1 or 2 further including a selection marker, preferably a drug resistance marker, such as a neomycine resistance gene, and/or a transcription termination site, such as a DEF region of the mouse globin gene.

4. A recombinant DNA concatamer comprising the vector according to any one of claims 1 to 3 as a first vector and a second vector including a $PreS_2$-S protein coding region or an S protein coding region of a hepatitis B surface antigen.

5. The concatamer of claim 4, wherein the metallothionein gene promoter is incorporated into the additional vector and located preferably immediately upstream of the $PreS_2$-S protein coding region.

6. The concatamer of claim 4, wherein the first and/or additional vector includes a transcription termination site, such as the DEF region of the mouse globin gene, and/or a selection marker.

7. The concatamer of claim 4, wherein the DNA vectors contain no other viral DNA segments.

8. A eukaryotic cell transfected with the recombinant DNA vector of any of claims 1 to 3 or with the concatamer of any of claims 4 to 7.

9. A eukaryotic cell co-transfected with the recombinant DNA vector of any one of claims 1 to 3 being defined as the first recombinant DNA vector and with a second recombinant DNA vector including a $PreS_2$-S protein coding region or an S protein coding region and the promoter of a metallothionein gene.

10. A eukaryotic cell co-transfected with the recombinant vector of any one of claims 1 to 3 being defined as the first recombinant DNA vector and with a second recombinant DNA vector including a PreS$_2$-S protein coding region and with a third recombinant DNA vector including the promoter of a metallothionein gene and a selection marker, the metallothionein gene promoter in the third vector being located preferably immediately upstream of the selection marker.

11. The cell of claim 9 or 10, wherein the metallothionein gene promoter is incorporated into the second vector and is located preferably immediately upstream of the PreS$_2$-S protein coding region.

12. The cell of claim 9 or 10, wherein the second and/or third vector include a transcription termination site, such as the DEF region of the mouse globin gene, and/or wherein the second vector includes a selection marker.

13. The cell of claim 9 or 10, wherein the second and/or third DNA vector contain no other viral DNA segments.

14. The cell of claim 9 or 10, wherein the DNA vectors include different selection markers.

15. The cell of claim 10, wherein the first, the second and/or the third vector include a replicon.

16. The cell of any of claims 8 to 15, wherein the eukaryotic cell is a mammalian cell, such as a Chinese hamster ovary cell, a vero cell, an L-cell, a mouse fibroblastic cell or a rat fibroblastic cell.

17. A method of preparing the eukaryotic cell of claim 8, the method comprising:
transfecting a eukaryotic cell with the recombinant DNA vector according to any of claims 1 to 3 or with the concatamer according to any of claims 4 to 7.

18. The method of preparing the eukaryotic cell of claim 9, the method comprising:
co-transfecting a eukaryotic cell with the recombinant DNA vector of any of claims 1 to 3 and with a second recombinant DNA vector as defined in any of claims 9 or 11 to 14.

19. The method of preparing the eukaryotic cell of claim 11, the method comprising:
co-transfecting a eukaryotic cell with the recombinant DNA vector of any of claims 1 to 3 and with a second and third recombinant DNA vector as defined in any of claims 10 to 15.

20. The method of claim 19, wherein the DNA vectors are co-transfected in pairwise combinations, or are co-transfected together, or are transfected in a series of steps.

21. A method of preparing a particle comprising proteins coded for by the PreS$_1$-PreS$_2$-S coding region of hepatitis B surface antigen wherein at least one of such proteins corresponds to a polypeptide coded for by the entire PreS$_1$-PreS$_2$-S coding regions, the method comprising:

a. cultivating a transfected or co-transfected host cell according to any of claims 8 to 16 under culture medium conditions such that the host cell expresses the particle; and
b. isolating such particle.

22. The method of claim 21, wherein heavy metals, such as cadmium or zinc, or steroid hormones, such as dexamethasone, are added to the culture medium to induce the metallothionein gene promoter.

23. The method of claim 21, wherein the particles are isolated from a culture lysate of the host cell.

24. A method for preparing a vaccine for protecting a human against becoming infected with hepatitis B virus, comprising a method according to any one of claims 21 to 23 and combining the obtained particle with a usual carrier substance.

**Claims for the following Contracting State : AT**

1. A method for preparing a recombinant DNA vector being free of non-HBV viral or oncogenic nucleic acid sequences comprising combining

(a) A DNA sequence comprising the hepatitis B $PreS_1$-$PreS_2$-S protein coding region; and

(b) a promoter which is a metallothionin gene promoter and capable of controlling the expression of said coding region (a) in a mammalian host cell so as to allow the production of the $PreS_1$-$PreS_2$-S protein.

2. The method of claim 1, wherein the promoter is incorporated into the vector comprising the DNA sequence in addition to or in place of a hepatitis B natural promoter, preferably immediately upstream of the $PreS_1$-$PreS_2$-S protein coding regions.

3. The method according to claim 1 or 2 further including incorporating a selection marker into said vector, preferably a drug resistance marker, such as a neomycine resistance gene, and/or a transcription termination site, such as a DEF region of the mouse globin gene.

4. A method for preparing a recombinant DNA concatamer comprising combining the vector as defined in any one of claims 1 to 3 as a first vector and a second vector including a $PreS_2$-S protein coding region or an S protein coding region of a hepatitis B surface antigen.

5. The method of claim 4, wherein the metallothionein gene promoter is incorporated into the additional vector and located preferably immediately upstream of the $PreS_2$-S protein coding region.

6. The method of claim 4, wherein the first and/or additional vector includes a transcription termination site, such as the DEF region of the mouse globin gene, and/or a selection marker.

7. The method of claim 4, wherein the DNA vectors contain no other viral DNA segments.

8. A method of generating eukaryotic cell carrying the vector defined in any one of claims 1 to 3 or ten concatames of any one of claims 4 to 7 comprising transfecting a eukaryotic cell with the recombinant DNA vector defined in any one of claims 1 to 3 or with the concatamer defined in any one of claims 4 to 7.

9. A method of generating a recombinant eukaryotic cell comprising co-transfecting said cell with the recombinant DNA vector defined in any one of claims 1 to 3 being defined as the first recombinant DNA vector and with a second recombinant DNA vector including a $PreS_2$-S protein coding region or an S protein coding region and the promoter of a metallothionein gene.

10. A method of generating a recombinant eukaryotic cell comprising co-transfecting said cell with the recombinant vector defined in any one of claims 1 to 3 being defined as the first recombinant DNA vector and with a second recombinant DNA vector including a $PreS_2$-S protein coding region and with a third recombinant DNA vector including the promoter of a metallothionein gene and a selection marker, the metallothionein gene promoter in the third vector being located preferably immediately upstream of the selection marker.

11. The method of claim 9 or 10, wherein the metallothionein gene promoter is incorporated into the second vector and is located preferably immediately upstream of the $PreS_2$-S protein coding region.

12. The method of claim 9 or 10, wherein the second and/or third vector include a transcription termination site, such as the DEF region of the mouse globin gene, and/or wherein the second vector includes a selection marker.

13. The method of claim 9 or 10, wherein the second and/or third DNA vector contain no other viral DNA segments.

14. The method of claim 9 or 10, wherein the DNA vectors include different selection markers.

15. The method of claim 10, wherein the first, the second and/or the third vector include a replicon.

16. The method of any of claims 8 to 15, wherein the eukaryotic cell is a mammalian cell, such as a Chinese hamster ovary cell, a vero cell, an L-cell, a mouse fibroblastic cell or a rat fibroblastic cell.

17. A method of preparing the eukaryotic cell of claim 8, the method comprising:

transfecting a eukaryotic cell with the recombinant DNA vector according to any of claims 1 to 3 or with the concatamer according to any of claims 4 to 7.

**18.** The method of preparing the eukaryotic cell of claim 9, the method comprising:
co-transfecting a eukaryotic cell with the recombinant DNA vector of any of claims 1 to 3 and with a second recombinant DNA vector as defined in any of claims 9 or 11 to 14.

**19.** The method of preparing the eukaryotic cell of claim 11, the method comprising:
co-transfecting a eukaryotic cell with the recombinant DNA vector of any of claims 1 to 3 and with a second and third recombinant DNA vector as defined in any of claims 10 to 15.

**20.** The method of claim 19, wherein the DNA vectors are co-transfected in pairwise combinations, or are co-transfected together, or are transfected in a series of steps.

**21.** A method of preparing a particle comprising proteins coded for by the $PreS_1$-$PreS_2$-S coding region of hepatitis B surface antigen wherein at least one of such proteins corresponds to a polypeptide coded for by the entire $PreS_1$-$PreS_2$-S coding regions, the method comprising:

a. cultivating a transfected or co-transfected host cell according to any of claims 8 to 16 under culture medium conditions such that the host cell expresses the particle; and
b. isolating such particle.

**22.** The method of claim 21, wherein heavy metals, such as cadmium or zinc, or steroid hormones, such as dexamethasone, are added to the culture medium to induce the metallothionein gene promoter.

**23.** The method of claim 21, wherein the particles are isolated from a culture lysate of the host cell.

**24.** A method for preparing a vaccine for protecting a human against becoming infected with hepatitis B virus, comprising a method according to any one of claims 21 to 23 and combining the obtained particle with a usual carrier substance.

**Patentansprüche**

**Patentansprüche für folgende Vertragsstaaten : BE, CH, DE, FR, GB, IT, LI, LU, NL, SE**

**1.** Rekombinanter DNA-Vektor, der frei ist von viralen oder onkogenen nicht-HBV Nucleinsäuresequenzen, umfassend:

(a) eine DNA-Sequenz, die den Hepatitis B-$PreS_1$-$PreS_2$-S-Protein-codierenden Bereich umfaßt; und
(b) einen Promotor, der ein Metallothionein-Gen-Promotor ist und die Expression des codierenden Bereichs
(a) in einer Säuger-Wirtszelle kontrollieren kann, so daß das $PreS_1$-$PreS_2$-S-Protein produziert werden kann.

**2.** Vektor nach Anspruch 1, wobei der Promotor in den Vektor, der die DNA-Sequenz umfaßt, zusätzlich zu einem natürlichen Hepatitis B-Promotor oder an dessen Stelle, vorzugsweise unmittelbar stromaufwärts der $PreS_1$-$PreS_2$-S-Protein-codierenden Bereiche, eingebaut ist.

**3.** Vektor nach Anspruch 1 oder 2, der ferner einen Selektionsmarker enthält, vorzugsweise einen Arzneistoffresistenzmarker, wie ein Neomycin-Resistenz-Gen, und/oder eine Transkriptions-Terminationsstelle, wie einen DEF-Bereich des Maus-Globin-Gens.

**4.** Rekombinantes DNA-Concatamer, umfassend den Vektor nach einem der Ansprüche 1 bis 3 als einen ersten Vektor und einen zweiten Vektor, umfassend einen $PreS_2$-S-Protein-codierenden Bereich oder einen S-Protein-codierenden Bereich eines Hepatitis B-Oberflächenantigens.

**5.** Concatamer nach Anspruch 4, wobei der Metallothionein-Gen-Promotor in den weiteren Vektor eingebaut ist und vorzugsweise unmittelbar stromaufwärts des $PreS_2$-S-Protein-codierenden Bereichs liegt.

**6.** Concatamer nach Anspruch 4, wobei der erste und/oder weitere Vektor eine Transkriptions-Terminationsstelle, wie den DEF-Bereich des Maus-Globin-Gens, und/oder einen Selektionsmarker umfaßt.

7. Concatamer nach Anspruch 4, wobei die DNA-Vektoren keine anderen viralen DNA-Segmente enthalten.

8. Eukaryotische Zelle, die mit dem rekombinanten DNA-Vektor nach einem der Ansprüche 1 bis 3 oder mit dem Concatamer nach einem der Ansprüche 4 bis 7 transfiziert ist.

9. Eukaryotische Zelle, die mit dem rekombinanten DNA-Vektor nach einem der Ansprüche 1 bis 3, der als der erste rekombinante DNA-Vektor definiert ist, und mit einem zweiten rekombinanten DNA-Vektor, umfassend einen PreS$_2$-S-Protein-codierenden Bereich oder einen S-Protein-codierenden Bereich und den Promotor eines Metallothionein-Gens, co-transfiziert ist.

10. Eukaryotische Zelle, die mit dem rekombinanten Vektor nach einem der Ansprüche 1 bis 3, der als der erste rekombinante DNA-Vektor definiert ist, und mit einem zweiten rekombinanten DNA-Vektor, umfassend einen PreS2-S-Protein-codierenden Bereich, und mit einem dritten rekombinanten DNA-Vektor, umfassend den Promotor eines Metallothionein-Gens und einen Selektionsmarker, co-transfiziert ist, wobei der Metallothionein-Gen-Promotor im dritten Vektor vorzugsweise unmittelbar stromaufwärts des Selektionsmarkers liegt.

11. Zelle nach Anspruch 9 oder 10, wobei der Metallothionein-Gen-Promotor in den zweiten Vektor eingebaut ist und vorzugsweise unmittelbar stromaufwärts des PreS$_2$-S-Protein-codierenden Bereichs liegt.

12. Zelle nach Anspruch 9 oder 10, wobei der zweite und/oder der dritte Vektor eine Transkriptions-Terminationsstelle, wie den DEF-Bereich des Maus-Globin-Gens, umfassen und/oder wobei der zweite Vektor einen Selektionsmarker umfaßt.

13. Zelle nach Anspruch 9 oder 10, wobei der zweite und/oder der dritte DNA-Vektor keine anderen viralen DNA-Segmente enthalten.

14. Zelle nach Anspruch 9 oder 10, wobei die DNA-Vektoren verschiedene Selektionsmarker umfassen.

15. Zelle nach Anspruch 10, wobei der erste, der zweite und/oder der dritte Vektor ein Replicon enthalten.

16. Zelle nach einem der Ansprüche 8 bis 15, wobei die eukaryotische Zelle eine Säugerzelle, wie eine Ovarzelle des Chinesischen Hamsters, eine Vero-Zelle, eine L-Zelle, eine Maus-Fibroblastenzelle oder eine Ratten-Fibroblastenzelle ist.

17. Verfahren zur Herstellung der eukaryotischen Zelle nach Anspruch 8, wobei das Verfahren folgendes umfaßt:
    Transfektion einer eukaryotischen Zelle mit dem rekombinanten DNA-Vektor nach einem der Ansprüche 1 bis 3 oder mit dem Concatamer nach einem der Ansprüche 4 bis 7.

18. Verfahren zur Herstellung der eukaryotischen Zelle nach Anspruch 9, wobei das Verfahren folgendes umfaßt:
    Co-Transfektion einer eukaryotischen Zelle mit dem rekombinanten DNA-Vektor nach einem der Ansprüche 1 bis 3 und mit einem zweiten rekombinanten DNA-Vektor, wie in einem der Ansprüche 9 oder 11 bis 14 definiert.

19. Verfahren zur Herstellung der eukaryotischen Zelle nach Anspruch 11, wobei das Verfahren folgendes umfaßt:
    Co-Transfektion einer eukaryotischen Zelle mit dem rekombinanten DNA-Vektor nach einem der Ansprüche 1 bis 3 und mit einem zweiten und dritten rekombinanten DNA-Vektor, wie in einem der Ansprüche 10 bis 15 definiert.

20. Verfahren nach Anspruch 19, wobei die DNA-Vektoren in paarweisen Kombinationen co-transfiziert werden oder zusammen co-transfiziert werden oder in einer Serie von Schritten transfiziert werden.

21. Verfahren zur Herstellung eines Proteine umfassenden Partikels, die durch den PreS$_1$-PreS$_2$-S-codierenden Bereich des Hepatitis B-Oberflächenantigens codiert werden, wobei mindestens eines dieser Proteine einem Polypeptid entspricht, das durch die das gesamte PreS$_1$-PreS$_2$-S codierenden Bereiche codiert wird, wobei das Verfahren folgendes umfaßt:

    a. Züchtung einer transfizierten oder co-transfizierten Wirtszelle nach einem der Ansprüche 8 bis 16 unter Kulturmedium-Bedingungen, so daß die Wirtszelle das Partikel exprimiert; und
    b. Isolierung des Partikels.

**22.** Verfahren nach Anspruch 21, wobei Schwermetalle, wie Cadmium oder Zink, oder Steroidhormone, wie Dexamethason, zur Induktion des Metallothionein-Gen-Promotors dem Kulturmedium zugegeben werden.

**23.** Verfahren nach Anspruch 21, wobei die Partikel aus einem Kulturlysat der Wirtszelle isoliert werden.

**24.** Verfahren zur Herstellung eines Impfstoffes zum Schutz eines Menschen vor einer Infektion mit Hepatitis B-Virus, umfassend ein Verfahren nach einem der Ansprüche 21 bis 23 und Kombination des erhaltenen Partikels mit einer herkömmlichen Trägersubstanz.

**Patentansprüche für folgenden Vertragsstaat : AT**

**1.** Verfahren zur Herstellung eines rekombinanten DNA-Vektors, der frei ist von viralen oder onkogenen nicht-HBV Nucleinsäuresequenzen, umfassend die Kombination:

(a) einer DNA-Sequenz, die den Hepatitis B-PreS$_1$-PreS$_2$-S-Protein-codierenden Bereich umfaßt; und
(b) eines Promotors, der ein Metallothionein-Gen-Promotor ist und die Expression des codierenden Bereichs (a) in einer Säuger-Wirtszelle kontrollieren kann, so daß das PreS$_1$-PreS$_2$-S-Protein produziert werden kann.

**2.** Verfahren nach Anspruch 1, wobei der Promotor in den Vektor, der die DNA-Sequenz umfaßt, zusätzlich zu einem natürlichen Hepatitis B-Promotor oder an dessen Stelle, vorzugsweise unmittelbar stromaufwärts der PreS$_1$-PreS$_2$-S-Protein-codierenden Bereiche, eingebaut ist.

**3.** Verfahren nach Anspruch 1 oder 2, das ferner den Einbau eines Selektionsmarkers, vorzugsweise eines Arzneistoffresistenzmarkers, wie eines Neomycin-Resistenz-Gens, und/oder einer Transkriptions-Terminations-stelle, wie eines DEF-Bereichs des Maus-Globin-Gens, in den Vektor umfaßt.

**4.** Verfahren zur Herstellung eines rekombinanten DNA-Concatamers, umfassend die Kombination des Vektors, wie in einem der Ansprüche 1 bis 3 definiert, als einen ersten Vektor und eines zweiten Vektors, umfassend einen PreS2-S-Protein-codierenden Bereich oder einen S-Protein-codierenden Bereich eines Hepatitis B-Oberflächenantigens.

**5.** Verfahren nach Anspruch 4, wobei der Metallothionein-Gen-Promotor in den weiteren Vektor eingebaut ist und vorzugsweise unmittelbar stromaufwärts des PreS$_2$-S-Protein-codierenden Bereichs liegt.

**6.** Verfahren nach Anspruch 4, wobei der erste und/oder weitere Vektor eine Transkriptions-Terminationsstelle, wie den DEF-Bereich des Maus-Globin-Gens, und/oder einen Selektionsmarker umfaßt.

**7.** Verfahren nach Anspruch 4, wobei die DNA-Vektoren keine anderen viralen DNA-Segmente enthalten.

**8.** Verfahren zur Erzeugung einer eukaryotischen Zelle, die den Vektor, definiert in einem der Ansprüche 1 bis 3, oder die Concatamere nach einem der Ansprüche 4 bis 7 enthält, umfassend die Transfektion einer eukaryotischen Zelle mit dem rekombinanten DNA-Vektor, definiert in einem der Ansprüche 1 bis 3, oder mit dem Concatamer, definiert in einem der Ansprüche 4 bis 7.

**9.** Verfahren zur Erzeugung einer rekombinanten eukaryotischen Zelle, umfassend die Co-Transfektion der Zelle mit dem rekombinanten DNA-Vektor, definiert in einem der Ansprüche 1 bis 3, der als der erste rekombinante DNA-Vektor definiert ist, und mit einem zweiten rekombinanten DNA-Vektor, umfassend einen PreS$_2$-S-Protein-codierenden Bereich oder einen S-Protein-codierenden Bereich und den Promotor eines Metallothionein-Gens.

**10.** Verfahren zur Erzeugung einer rekombinanten eukaryotischen Zelle, umfassend die Co-Transfektion der Zelle mit dem rekombinanten Vektor, definiert in einem der Ansprüche 1 bis 3, der als der erste rekombinante DNA-Vektor definiert ist, und mit einem zweiten rekombinanten DNA-Vektor, umfassend einen PreS$_2$-S-Protein-codierenden Bereich, und mit einem dritten rekombinanten DNA-Vektor, umfassend den Promotor eines Metallothionein-Gens und einen Selektionsmarker, wobei der Metallothionein-Gen-Promotor im dritten Vektor vorzugsweise unmittelbar stromaufwärts des Selektionsmarkers liegt.

**11.** Verfahren nach Anspruch 9 oder 10, wobei der Metallothionein-Gen-Promotor in den zweiten Vektor eingebaut ist und vorzugsweise unmittelbar stromaufwärts des PreS2-S-Protein-codierenden Bereichs liegt.

**12.** Verfahren nach Anspruch 9 oder 10, wobei der zweite und/oder der dritte Vektor eine Transkriptions-Terminationsstelle, wie z.B. den DEF-Bereich des Maus-Globin-Gens, umfassen und/oder wobei der zweite Vektor einen Selektionsmarker umfaßt.

**13.** Verfahren nach Anspruch 9 oder 10, wobei der zweite und/oder der dritte DNA-Vektor keine anderen viralen DNA-Segmente enthalten.

**14.** Verfahren nach Anspruch 9 oder 10, wobei die DNA-Vektoren verschiedene Selektionsmarker umfassen.

**15.** Verfahren nach Anspruch 10, wobei der erste, der zweite und/oder der dritte Vektor ein Replicon enthalten.

**16.** Verfahren nach einem der Ansprüche 8 bis 15, wobei die eukaryotische Zelle eine Säugerzelle, wie eine Ovarzelle des Chinesischen Hamsters, eine Vero-Zelle, eine L-Zelle, eine Maus-Fibroblastenzelle oder eine Ratten-Fibroblastenzelle ist.

**17.** Verfahren zur Herstellung der eukaryotischen Zelle nach Anspruch 8, wobei das Verfahren folgendes umfaßt:
Transfektion einer eukaryotischen Zelle mit dem rekombinanten DNA-Vektor nach einem der Ansprüche 1 bis 3 oder mit dem Concatamer nach einem der Ansprüche 4 bis 7.

**18.** Verfahren zur Herstellung der eukaryotischen Zelle nach Anspruch 9, wobei das Verfahren folgendes umfaßt:
Co-Transfektion einer eukaryotischen Zelle mit dem rekombinanten DNA-Vektor nach einem der Ansprüche 1 bis 3 und mit einem zweiten rekombinanten DNA-Vektor, wie in einem der Ansprüche 9 oder 11 bis 14 definiert.

**19.** Verfahren zur Herstellung der eukaryotischen Zelle nach Anspruch 11, wobei das Verfahren folgendes umfaßt:
Co-Transfektion einer eukaryotischen Zelle mit dem rekombinanten DNA-Vektor nach einem der Ansprüche 1 bis 3 und mit einem zweiten und einem dritten rekombinanten DNA-Vektor, wie in einem der Ansprüche 10 bis 15 definiert.

**20.** Verfahren nach Anspruch 19, wobei die DNA-Vektoren in paarweisen Kombinationen co-transfiziert werden oder zusammen co-transfiziert werden oder in einer Serie von Schritten transfiziert werden.

**21.** Verfahren zur Herstellung eines Proteine umfassenden Partikels, die durch den PreS$_1$-PreS$_2$-S-codierenden Bereich des Hepatitis B-Oberflächenantigens codiert werden, wobei mindestens eines dieser Proteine einem Polypeptid entspricht, das durch die das gesamte PreS$_1$-PreS$_2$-S codierenden Bereiche codiert wird, wobei das Verfahren folgendes umfaßt:

a. Züchtung einer transfizierten oder co-transfizierten Wirtszelle nach einem der Ansprüche 8 bis 16 unter Kulturmedium-Bedingungen, so daß die Wirtszelle das Partikel exprimiert; und
b. Isolierung des Partikels.

**22.** Verfahren nach Anspruch 21, wobei Schwermetalle, wie Cadmium oder Zink, oder Steroidhormone, wie Dexamethason, zur Induktion des Metallothionein-Gen-Promotors dem Kulturmedium zugegeben werden.

**23.** Verfahren nach Anspruch 21, wobei die Partikel aus einem Kulturlysat der Wirtszelle isoliert werden.

**24.** Verfahren zur Herstellung eines Impfstoffes zum Schutz eines Menschen vor einer Infektion mit Hepatitis B-Virus, umfassend ein Verfahren nach einem der Ansprüche 21 bis 23 und Kombination des erhaltenen Partikels mit einer herkömmlichen Trägersubstanz.

**Revendications**

**Revendications pour les Etats contractants suivants : BE, CH, DE, FR, GB, IT, LI, LU, NL, SE**

**1.** Vecteur d'ADN recombinant exempt de séquences acide nucleique oncogénique ou viral non-HBV, comportant :

a) une séquence ADN comportant la région de codage de la protéine PreS$_1$-PreS$_2$-S de l'hépatite B; et
b) un promoteur qui est un promoteur de gêne de métallothionéine et capable de contrôler l'expression de ladite région de codage a) dans une cellule hote de mammifère de façon à permettre la production de la protéine PreS$_1$-PreS$_2$-S.

2. Vecteur selon la revendication 1, dans lequel le promoteur est incorporé dans le vecteur comprenant la séquence ADN en addition ou à la place du promoteur naturel de l'hépatite B, de préférence immédiatement en amont des régions de codage de la protéine PreS$_1$-PreS$_2$-S.

3. Vecteur selon la revendication 1 ou 2 incluant en outre un marqueur de sélection, de préférence un marqueur résistant aux médicaments, tel qu'un gêne résistant à la néomycine, et/ou un site de terminaison de transcription, tel qu'une région DEF du gêne de globine de souris.

4. Concatamère d'ADN recombinant comportant le vecteur selon une quelconque des revendication 1 à 3 comme un premier vecteur et un second vecteur comportant une région de codage de la protéine PreS$_2$-S ou une région de codage de la protéine S d'un antigène de surface de l'hépatite B.

5. Concatamère selon la revendication 4, dans lequel le promoteur de gêne de métallothionéine est incorporé dans le vecteur additionnel et est situé de préférence immédiatement en amont de la région de codage de la protéine PreS$_2$-S.

6. Concatamère selon la revendication 4, dans lequel le premier et/ou le vecteur additionnel comporte un site de terminaison de transcription, tel que la région DEF du gêne de globine de souris et/ou un marqueur de sélection.

7. Concatamère selon la revendication 4, dans lequel les vecteurs ADN ne contiennent pas d'autres segments ADN viraux.

8. Cellule eucaryotique transfectée avec le vecteur ADN recombinant selon une quelconque des revendications 1 à 3 ou avec le concatamère selon une quelconque des revendications 4 à 7.

9. Cellule eucaryotique transfectée avec le vecteur ADN recombinant selon une quelconque des revendications 1 à 3, étant défini comme le premier vecteur ADN recombinant, et avec un second vecteur ADN recombinant incluant une région de codage de la protéine PreS$_2$-S ou une région de codage de la protéine S et le promoteur d'un gêne métallothionéine.

10. Cellule eucaryotique co-transfectée avec le vecteur recombinant selon une quelconque des revendication 1 à 3, étant défini comme le premier vecteur ADN recombinant, avec un second vecteur ADN recombinant incluant la région de codage de la protéine PreS$_2$-S et avec un troisième vecteur ADN recombinant incluant le promoteur d'un gêne métallothionéine et un marqueur de sélection, le promoteur d'un gêne métallothionéine dans le troisième vecteur étant situé de préférence immédiatement en amont du marqueur de sélection.

11. Cellule selon la revendication 9 ou 10, dans laquelle le promoteur d'un gêne métallothionéine est incorporé dans le second vecteur et est situé de préférence immédiatement en amont de la région de codage de la protéine PreS$_2$-S.

12. Cellule selon la revendication 9 ou 10, dans laquelle le second et/ou le troisième vecteur inclut un site de terminaison de transcription, tel qu'une région DEF du gêne globine de souris, et/ou dans lequel le second vecteur inclut un marqueur de sélection.

13. Cellule selon la revendication 9 ou 10, dans laquelle le second et/ou le troisième vecteur ADN ne contient pas d'autres segments ADN viraux.

14. Cellule selon la revendication 9 ou 10, dans laquelle les vecteurs ADN incluent différents marqueur de sélection.

15. Cellule selon la revendication 10, dans laquelle le premier, le second et/ou le troisième vecteur inclut un réplicon.

16. Cellule selon l'une quelconque des revendications 8 à 15, dans laquelle la cellule eucaryotique est une cellule de mammifère, telle qu'une cellule d'ovaire de hamster de Chine, une cellule véro, une cellule L, une cellule fibroblastique de souris, ou une cellule fibroblastique de rat.

17. Procédé de préparation de la cellule eucaryotique de la revendication 8, comportant l'étape suivante :
   Transfecter une cellule eucaryotique avec le vecteur ADN recombinant selon une quelconque des revendications 1 à 3 ou avec le concatamère selon une quelconque des revendication 4 à 7.

18. Procédé de préparation de la cellule eucaryotique selon la revendication 9, comportant l'étape suivante :
   Co-transfecter une cellule eucaryotique avec le vecteur ADN recombinant selon une quelconque des revendications 1 à 3, et avec un second vecteur ADN recombinant tel que défini dans une quelconque des revendications 9 ou 11 à 14.

19. Procédé de préparation de la cellule eucaryotique selon la revendication 11, comportant l'étape suivante :
   Co-transfecter une cellule eucaryotique avec le vecteur ADN recombinant selon une quelconque des revendications 1 à 3, et avec un second et troisième vecteur ADN recombinant tel que défini dans une quelconque des revendications 10 à 15.

20. Procédé selon la revendication 19 dans lequel les vecteur ADN sont co-transfectés en combinaisons par paires, ou sont co-transfectés ensemble, ou sont transfectés dans une série d'étapes.

21. Procédé de préparation d'une particule comportant des protéines codées par la région de codage $PreS_1$-$PreS_2$-S de l'antigène de surface de l'hépatite B, dans lequel au moins une desdites protéines correspond à un polypeptide codé par les régions de codage entières $PreS_1$-$PreS_2$-S, comportant les étapes suivantes :

   a) cultiver une cellule hote transfectée ou co-transfectée selon une quelconque des revendications 8 à 16 dans des conditions de milieu de culture de façon que la cellule hote exprime la particule et
   b) isoler ladite particule.

22. Procédé selon la revendication 21, dans lequel des métaux lourds, tels que le cadmium ou le zinc, ou des hormones stéroïdes, tels que le dexaméthasone, sont ajoutés au milieu de culture pour induire le promoteur du gêne métallothionéine.

23. Procédé selon la revendication 21, dans lequel les particules sont isolées d'une culture de lysate de la cellule hote.

24. Procédé pour préparer un vaccin pour protéger un humain contre l'infection du virus de l'hépatite B, comportant un procédé selon une quelconque des revendications 21 à 23, et combiner la particule obtenue avec une substance support usuelle.

**Revendications pour l'Etat contractant suivant : AT**

1. Procédé de préparation d'un vecteur d'ADN recombinant exempt de séquences acide nucleique oncogénique ou viral non-HBV, comportant la combinaison de :

   a) une séquence ADN comportant la région de codage de la protéine $PreS_1$$PreS_2$-S de l'hépatite B; et
   b) un promoteur qui est un promoteur de gêne de métallothionéine et capable de contrôler l'expression de ladite région de codage a) dans une cellule hote de mammifère de façon à permettre la production de la protéine $PreS_1$-$PreS_2$-S.

2. Procédé selon la revendication 1, dans lequel le promoteur est incorporé dans le vecteur comprenant la séquence ADN en addition ou à la place du promoteur naturel de l'hépatite B, de préférence immédiatement en amont des régions de codage de la protéine $PreS_1$-$PreS_2$-S.

3. Procédé selon la revendication 1 ou 2 incluant en outre l'incorporation d'un marqueur de sélection dans ledit vecteur, de préférence un marqueur résistant aux médicaments, tel qu'un gêne résistant à la néomycine, et/ou un site de terminaison de transcription, tel qu'une région DEF du gêne de globine de souris.

4. Procédé de préparation d'un concatamère d'ADN recombinant comportant la combinaison du vecteur tel que défini dans une quelconque des revendication 1 à 3 comme un premier vecteur et un second vecteur comportant une région de codage de la protéine PreS$_2$-S ou une région de codage de la protéine S d'un antigène de surface de l'hépatite B.

5. Procédé selon la revendication 4, dans lequel le promoteur de gêne de métallothionéine est incorporé dans le vecteur additionnel et est situé de préférence immédiatement en amont de la région de codage de la protéine PreS$_2$-S.

6. Procédé selon la revendication 4, dans lequel le premier et/ou le vecteur additionnel comporte un site de terminaison de transcription, tel que la région DEF du gêne de globine de souris et/ou un marqueur de sélection.

7. Procédé selon la revendication 4, dans lequel les vecteurs ADN ne contiennent pas d'autres segments ADN viraux.

8. Procédé pour engendrer une cellule eucaryotique comportant le vecteur défini dans une quelconque des revendications 1 à 3 ou le concatamère d'une quelconque des revendications 4 à 7 comportant l'étape de transfecter une cellule eucaryotique avec le vecteur ADN recombinant défini dans une quelconque des revendications 1 à 3 ou avec le concatamère défini dans une quelconque des revendications 4 à 7.

9. Procédé pour engendrer une cellule eucaryotique recombinante comportant la co-transfection de ladite cellule avec le vecteur ADN recombinant défini dans une quelconque des revendications 1 à 3, étant défini comme le premier vecteur ADN recombinant, et avec un second vecteur ADN recombinant incluant une région de codage de la protéine PreS$_2$-S ou une région de codage de la protéine S et le promoteur d'un gêne métallothionéine.

10. Procédé pour engendrer une cellule eucaryotique recombinante, comportant l'étape de co-transfecter ladite cellule avec le vecteur recombinant défini dans une quelconque des revendication 1 à 3, étant défini comme le premier vecteur ADN recombinant, avec un second vecteur ADN recombinant incluant la région de codage de la protéine PreS$_2$-S et avec un troisième vecteur ADN recombinant incluant le promoteur d'un gêne métallothionéine et un marqueur de sélection, le promoteur d'un gêne métallothionéine dans le troisième vecteur étant situé de préférence immédiatement en amont du marqueur de sélection.

11. Procédé selon la revendication 9 ou 10, dans lequel le promoteur d'un gêne métallothionéine est incorporé dans le second vecteur et est situé de préférence immédiatement en amont de la région de codage de la protéine PreS$_2$-S.

12. Procédé selon la revendication 9 ou 10, dans lequel le second et/ou le troisième vecteur inclut un site de terminaison de transcription, tel qu'une région DEF du gêne globine de souris, et/ou dans lequel le second vecteur inclut un marqueur de sélection.

13. Procédé selon la revendication 9 ou 10, dans lequel le second et/ou le troisième vecteur ADN ne contient pas d'autres segments ADN viraux.

14. Procédé selon la revendication 9 ou 10, dans lequel les vecteurs ADN incluent différents marqueur de sélection.

15. Procédé selon la revendication 10, dans lequel le premier, le second et/ou le troisième vecteur inclut un réplicon.

16. Procédé selon l'une quelconque des revendications 8 à 15, dans lequel la cellule eucaryotique est une cellule de mammifère, telle qu'une cellule d'ovaire de hamster de Chine, une cellule véro, une cellule L, une cellule fibroblastique de souris, ou une cellule fibroblastique de rat.

17. Procédé de préparation de la cellule eucaryotique de la revendication 8, comportant l'étape suivante :
Transfecter une cellule eucaryotique avec le vecteur ADN recombinant selon une quelconque des revendications 1 à 3 ou avec le concatamère selon une quelconque des revendication 4 à 7.

18. Procédé de préparation de la cellule eucaryotique selon la revendication 9, comportant l'étape suivante :
Co-transfecter une cellule eucaryotique avec le vecteur ADN recombinant selon une quelconque des revendications 1 à 3, et avec un second vecteur ADN recombinant tel que défini dans une quelconque des revencations 9 ou 11 à 14.

19. Procédé de préparation de la cellule eucaryotique selon la revendication 11, comportant l'étape suivante :

Co-transfecter une cellule eucaryotique avec le vecteur ADN recombinant selon une quelconque des revendications 1 à 3, et avec un second et troisième vecteur ADN recombinant tel que défini dans une quelconque des revendications 10 à 15.

20. Procédé selon la revendication 19 dans lequel les vecteur ADN sont co-transfectés en combinaisons par paires, ou sont co-transfectés ensemble, ou sont transfectés dans une série d'étapes.

21. Procédé de préparation d'une particule comportant des protéines codées par la région de codage $PreS_1$-$PreS_2$-S de l'antigène de surface de l'hépatite B, dans lequel au moins une desdites protéines correspond à un polypeptide codé par les régions de codage entières $PreS_1$-$PreS_2$-S, comportant les étapes suivantes :

a) cultiver une cellule hote transfectée ou co-transfectée selon une quelconque des revendications 8 à 16 dans des conditions de milieu de culture de façon que la cellule hote exprime la particule et
b) isoler ladite particule.

22. Procédé selon la revendication 21, dans lequel des métaux lourds, tels que le cadmium ou le zinc, ou des hormones stéroïdes, tels que le dexaméthasone, sont ajoutés au milieu de culture pour induire le promoteur du gêne métallothionéine.

23. Procédé selon la revendication 21, dans lequel les particules sont isolées d'une culture de lysate de la cellule hote.

24. Procédé pour préparer un vaccin pour protéger un humain contre l'infection du virus de l'hépatite B, comportant un procédé selon une quelconque des revendications 21 à 23, et combiner la particule obtenue avec une substance support usuelle.

*Fig. 1*

*Fig.2*

$Fig. 3$

Fig.4A

Fig.4 B

EP 0 198 474 B1

*Fig.5*

# Fig.6

## Column Chromatography (A5m) Profile

● ng/ml by NML RIA assay

Fig.7

Isopycnic Centrifugation Profile

○ absorbance
● ng/ml by NML RIA assay

FRACTION NUMBER

ABSORBANCE (280 nm)

(ng/ml)

Fig. 8A

SERVOCONVERSION BY LOT

LOT NO. 1 (Vero)
LOT NO. 2 (Vero)
LOT NO. 3 (Vero)
LOT NO. 4 (Vero)

SERVOCONVERSION BY LOT    _Fig.8B_

LOT NO.5 (Vero)

LOT NO. 7 (L)

LOT NO.6 (L)

LOT NO. 8(L)

*Fig 8C*

SERVOCONVERSION BY LOT

LOT NO. 9(L)

LOT NO. 10(L)

Fig.9A

Fig.9 B

Fig.10

Fig.11